# EUROPEAN PATENT APPLICATION

(11) **EP 2 980 099 A1**
(43) Date of publication of application: **03.02.2016**
(21) Application number: 14179359.6
(22) Date of filing: 31.07.2014
(51) Int. Cl.: C07K 14/18, G01N 33/53, G01N 33/68

(54) **Compositions and methods for detecting flaviviridae infections**

(71) Applicant: Bernhard-Nocht-Institut für Tropenmedizin, 20359 Hamburg (DE)
(72) Inventor: Schreiber, Michael, 20359 Hamburg (DE); Franzke, Kati, 22335 Hamburg (DE)
(74) Representative: Jones Day

(57) **Abstract**

The present invention relates to compounds and methods for detecting flavivirus infections, in particular dengue virus infections, that allow early detection of flavivirus infections with satisfying sensitivity. Specifically, the invention relates to polypeptides comprising flavivirus envelope protein domain III that are denatured. Further embodiments of the invention relate to compositions where these denatured polypeptides are attached to solid substrates. The invention provides methods for detecting flavivirus infections in samples from subjects suspected to have a flavivirus infection as well as kits for performing said methods.

SEQUENCE LISTING

<110>  Bernhard-Nocht-Institut für Tropenmedizin

<120>  COMPOSITIONS AND METHODS FOR DETECTING FLAVIVIRIDAE INFECTIONS

<130>  103405EP

<160>  31

<170>  PatentIn version 3.5

<210>  1
<211>  142
<212>  PRT
<213>  Dengue virus type 1

<400>  1

Val Met Cys Thr Gly Ser Phe Lys Leu Glu Lys Glu Val Ala Glu Thr
1               5                   10                  15

Gln His Gly Thr Val Leu Val Gln Val Lys Tyr Glu Gly Thr Asp Ala
            20                  25                  30

Pro Cys Lys Ile Pro Phe Ser Ser Gln Asp Glu Lys Gly Val Thr Gln
        35                  40                  45

Asn Gly Arg Leu Ile Thr Ala Asn Pro Ile Val Thr Asp Lys Glu Lys
    50                  55                  60

Pro Val Asn Ile Glu Ala Glu Pro Pro Phe Gly Glu Ser Tyr Ile Val
65                  70                  75                  80

Val Gly Ala Gly Glu Lys Ala Leu Lys Leu Ser Trp Phe Lys Lys Gly
                85                  90                  95

Ser Ser Ile Gly Lys Met Phe Glu Ala Thr Ala Arg Gly Ala Arg Arg
            100                 105                 110

Met Ala Ile Leu Gly Asp Thr Ala Trp Asp Phe Gly Ser Ile Gly Gly
        115                 120                 125

Val Phe Thr Ser Val Gly Lys Leu Ile His Gln Ile Phe Gly
    130                 135                 140

<210>  2
<211>  117
<212>  PRT
<213>  Dengue virus type 1

<400>  2

Val Met Cys Thr Gly Ser Phe Lys Leu Glu Lys Glu Val Ala Glu Thr

1               5                   10                  15

Gln His Gly Thr Val Leu Val Gln Val Lys Tyr Glu Gly Thr Asp Ala
            20                  25                  30

Pro Cys Lys Ile Pro Phe Ser Ser Gln Asp Glu Lys Gly Val Thr Gln
        35                  40                  45

Asn Gly Arg Leu Ile Thr Ala Asn Pro Ile Val Thr Asp Lys Glu Lys
    50                  55                  60

Pro Val Asn Ile Glu Ala Glu Pro Pro Phe Gly Glu Ser Tyr Ile Val
65                  70                  75                  80

Val Gly Ala Gly Glu Lys Ala Leu Lys Leu Ser Trp Phe Lys Lys Gly
                85                  90                  95

Ser Ser Ile Gly Lys Met Phe Glu Ala Thr Ala Arg Gly Ala Arg Arg
            100                 105                 110

Met Ala Ile Leu Gly
        115

<210>  3
<211>  97
<212>  PRT
<213>  Dengue virus type 1

<400>  3

Val Met Cys Thr Gly Ser Phe Lys Leu Glu Lys Glu Val Ala Glu Thr
1               5                   10                  15

Gln His Gly Thr Val Leu Val Gln Val Lys Tyr Glu Gly Thr Asp Ala
            20                  25                  30

Pro Cys Lys Ile Pro Phe Ser Ser Gln Asp Glu Lys Gly Val Thr Gln
        35                  40                  45

Asn Gly Arg Leu Ile Thr Ala Asn Pro Ile Val Thr Asp Lys Glu Lys
    50                  55                  60

Pro Val Asn Ile Glu Ala Glu Pro Pro Phe Gly Glu Ser Tyr Ile Val
65                  70                  75                  80

Val Gly Ala Gly Glu Lys Ala Leu Lys Leu Ser Trp Phe Lys Lys Gly
                85                  90                  95

Ser

<210>  4
<211>  100
<212>  PRT
<213>  artificial sequence

<220>
<223>  Dengue virus serotype 1, ED3 domain, consensus polypeptide
       sequence

<400>  4

Met Ser Tyr Val Met Cys Thr Gly Ser Phe Lys Leu Glu Lys Glu Val
1               5                   10                  15

Ala Glu Thr Gln His Gly Thr Val Leu Val Gln Val Lys Tyr Glu Gly
            20                  25                  30

Thr Asp Ala Pro Cys Lys Ile Pro Phe Ser Thr Gln Asp Glu Lys Gly
        35                  40                  45

Val Thr Gln Asn Gly Arg Leu Ile Thr Ala Asn Pro Ile Val Thr Asp
    50                  55                  60

Lys Glu Lys Pro Val Asn Ile Glu Ala Glu Pro Pro Phe Gly Glu Ser
65                  70                  75                  80

Tyr Ile Val Ile Gly Ala Gly Glu Lys Ala Leu Lys Leu Ser Trp Phe
                85                  90                  95

Lys Lys Gly Ser
            100


<210>  5
<211>  144
<212>  PRT
<213>  Dengue virus type 2

<400>  5

Ser Tyr Ser Met Cys Thr Gly Lys Phe Lys Val Val Lys Glu Ile Ala
1               5                   10                  15

Glu Thr Gln His Gly Thr Ile Val Ile Arg Val Gln Tyr Glu Gly Asp
            20                  25                  30

Gly Ser Pro Cys Lys Ile Pro Phe Glu Ile Met Asp Leu Glu Lys Arg
        35                  40                  45

His Val Leu Gly Arg Leu Ile Thr Val Asn Pro Ile Val Thr Glu Lys
    50                  55                  60

Asp Ser Pro Val Asn Ile Glu Ala Glu Pro Pro Phe Gly Asp Ser Tyr
65                  70                  75                  80

Ile Ile Ile Gly Val Glu Pro Gly Gln Leu Lys Leu Asn Trp Phe Lys
                85                  90                  95

Lys Gly Ser Ser Ile Gly Gln Met Phe Glu Thr Thr Met Arg Gly Ala
            100                 105                 110

Lys Arg Met Ala Ile Leu Gly Asp Thr Ala Trp Asp Phe Gly Ser Leu
        115                 120                 125

Gly Gly Val Phe Thr Ser Ile Gly Lys Ala Leu His Gln Val Phe Gly
    130                 135                 140

<210>  6
<211>  119
<212>  PRT
<213>  Dengue virus type 2

<400>  6

Ser Tyr Ser Met Cys Thr Gly Lys Phe Lys Val Val Lys Glu Ile Ala
1               5                   10                  15

Glu Thr Gln His Gly Thr Ile Val Ile Arg Val Gln Tyr Glu Gly Asp
            20                  25                  30

Gly Ser Pro Cys Lys Ile Pro Phe Glu Ile Met Asp Leu Glu Lys Arg
        35                  40                  45

His Val Leu Gly Arg Leu Ile Thr Val Asn Pro Ile Val Thr Glu Lys
    50                  55                  60

Asp Ser Pro Val Asn Ile Glu Ala Glu Pro Pro Phe Gly Asp Ser Tyr
65                  70                  75                  80

Ile Ile Ile Gly Val Glu Pro Gly Gln Leu Lys Leu Asn Trp Phe Lys
                85                  90                  95

Lys Gly Ser Ser Ile Gly Gln Met Phe Glu Thr Thr Met Arg Gly Ala
            100                 105                 110

Lys Arg Met Ala Ile Leu Gly
        115

<210>  7
<211>  101

<212>  PRT
<213>  Dengue virus type 2

<400>  7

Ser Tyr Ser Met Cys Thr Gly Lys Phe Lys Val Val Lys Glu Ile Ala
1               5                   10                  15

Glu Thr Gln His Gly Thr Ile Val Ile Arg Val Gln Tyr Glu Gly Asp
            20                  25                  30

Gly Ser Pro Cys Lys Ile Pro Phe Glu Ile Met Asp Leu Glu Lys Arg
        35                  40                  45

His Val Leu Gly Arg Leu Ile Thr Val Asn Pro Ile Val Thr Glu Lys
    50                  55                  60

Asp Ser Pro Val Asn Ile Glu Ala Glu Pro Pro Phe Gly Asp Ser Tyr
65                  70                  75                  80

Ile Ile Ile Gly Val Glu Pro Gly Gln Leu Lys Leu Asn Trp Phe Lys
                85                  90                  95

Lys Gly Ser Ser Ile
            100


<210>  8
<211>  100
<212>  PRT
<213>  Artificial sequence

<220>
<223>  Dengue virus serotype 2, ED3 domain, consensus polypeptide
       sequence

<400>  8

Met Ser Tyr Ser Met Cys Thr Gly Lys Phe Lys Val Val Lys Glu Ile
1               5                   10                  15

Ala Glu Thr Gln His Gly Thr Ile Val Ile Arg Val Gln Tyr Glu Gly
            20                  25                  30

Asp Gly Ser Pro Cys Lys Ile Pro Phe Glu Ile Met Asp Leu Glu Lys
        35                  40                  45

Arg His Val Leu Gly Arg Leu Ile Thr Val Asn Pro Ile Val Thr Glu
    50                  55                  60

Lys Asp Ser Pro Val Asn Ile Glu Ala Glu Pro Pro Phe Gly Asp Ser
65                  70                  75                  80

Tyr Ile Ile Ile Gly Val Glu Pro Gly Gln Leu Lys Leu Asn Trp Phe
                85                  90                  95

Lys Lys Gly Ser
            100

<210> 9
<211> 145
<212> PRT
<213> Dengue virus type 3

<400> 9

Met Ser Tyr Ala Met Cys Leu Asn Thr Phe Val Leu Lys Lys Glu Val
1               5                   10                  15

Ser Glu Thr Gln His Gly Thr Ile Leu Ile Lys Val Glu Tyr Lys Gly
            20                  25                  30

Glu Asp Ala Pro Cys Lys Ile Pro Phe Ser Thr Glu Asp Gly Gln Gly
        35                  40                  45

Lys Ala His Asn Gly Arg Leu Ile Thr Ala Asn Pro Val Val Thr Lys
    50                  55                  60

Lys Glu Glu Pro Val Asn Ile Glu Ala Glu Pro Pro Phe Gly Glu Ser
65                  70                  75                  80

Asn Ile Val Ile Gly Ile Gly Asp Lys Ala Leu Lys Ile Asn Trp Tyr
                85                  90                  95

Arg Lys Gly Ser Ser Ile Gly Lys Met Phe Glu Ala Thr Ala Arg Gly
            100                 105                 110

Ala Arg Arg Met Ala Ile Leu Gly Asp Thr Ala Trp Asp Phe Gly Ser
        115                 120                 125

Val Gly Gly Val Leu Asn Ser Leu Gly Lys Met Val His Gln Ile Phe
    130                 135                 140

Gly
145

<210> 10
<211> 120
<212> PRT
<213> Dengue virus type 3

<400> 10

Met Ser Tyr Ala Met Cys Leu Asn Thr Phe Val Leu Lys Lys Glu Val
1               5                   10                  15

Ser Glu Thr Gln His Gly Thr Ile Leu Ile Lys Val Glu Tyr Lys Gly
            20                  25                  30

Glu Asp Ala Pro Cys Lys Ile Pro Phe Ser Thr Glu Asp Gly Gln Gly
        35                  40                  45

Lys Ala His Asn Gly Arg Leu Ile Thr Ala Asn Pro Val Val Thr Lys
    50                  55                  60

Lys Glu Glu Pro Val Asn Ile Glu Ala Glu Pro Pro Phe Gly Glu Ser
65                  70                  75                  80

Asn Ile Val Ile Gly Ile Gly Asp Lys Ala Leu Lys Ile Asn Trp Tyr
                85                  90                  95

Arg Lys Gly Ser Ser Ile Gly Lys Met Phe Glu Ala Thr Ala Arg Gly
            100                 105                 110

Ala Arg Arg Met Ala Ile Leu Gly
        115                 120

<210>  11
<211>  103
<212>  PRT
<213>  Dengue virus type 3

<400>  11

Met Ser Tyr Ala Met Cys Leu Asn Thr Phe Val Leu Lys Lys Glu Val
1               5                   10                  15

Ser Glu Thr Gln His Gly Thr Ile Leu Ile Lys Val Glu Tyr Lys Gly
            20                  25                  30

Glu Asp Ala Pro Cys Lys Ile Pro Phe Ser Thr Glu Asp Gly Gln Gly
        35                  40                  45

Lys Ala His Asn Gly Arg Leu Ile Thr Ala Asn Pro Val Val Thr Lys
    50                  55                  60

Lys Glu Glu Pro Val Asn Ile Glu Ala Glu Pro Pro Phe Gly Glu Ser
65                  70                  75                  80

Asn Ile Val Ile Gly Ile Gly Asp Lys Ala Leu Lys Ile Asn Trp Tyr
                85                  90                  95

Arg Lys Gly Ser Ser Ile Gly
            100

<210>  12
<211>  100
<212>  PRT
<213>  Artificial sequence

<220>
<223>  Dengue virus serotype 3, ED3 domain, consensus polypeptide
       sequence

<400>  12

Met Ser Tyr Ala Met Cys Leu Asn Thr Phe Val Leu Lys Lys Glu Val
1               5                   10                  15

Ser Glu Thr Gln His Gly Thr Ile Leu Ile Lys Val Glu Tyr Lys Gly
            20                  25                  30

Glu Asp Ala Pro Cys Lys Ile Pro Phe Ser Thr Glu Asp Gly Gln Gly
        35                  40                  45

Lys Ala His Asn Gly Arg Leu Ile Thr Ala Asn Pro Val Val Thr Lys
    50                  55                  60

Lys Glu Glu Pro Val Asn Ile Glu Ala Glu Pro Pro Phe Gly Glu Ser
65                  70                  75                  80

Asn Ile Val Ile Gly Ile Gly Asp Lys Ala Leu Lys Ile Asn Trp Tyr
                85                  90                  95

Lys Lys Gly Ser
            100

<210>  13
<211>  142
<212>  PRT
<213>  Dengue virus type 4

<400>  13

Thr Met Cys Ser Gly Lys Phe Ser Ile Asp Lys Glu Met Ala Glu Thr
1               5                   10                  15

Gln His Gly Thr Thr Val Val Lys Val Lys Tyr Glu Gly Ala Gly Ala
            20                  25                  30

Pro Cys Lys Val Pro Ile Glu Ile Arg Asp Val Asn Lys Glu Lys Val
        35                  40                  45

Val Gly Arg Ile Ile Ser Ser Thr Pro Phe Ala Glu Tyr Thr Asn Ser

50                  55                  60

Val Thr Asn Ile Glu Leu Glu Pro Pro Phe Gly Asp Ser Tyr Ile Val
65                  70                  75                  80

Ile Gly Val Gly Asp Ser Ala Leu Thr Leu His Trp Phe Arg Lys Gly
                85                  90                  95

Ser Ser Ile Gly Lys Met Leu Glu Ser Thr Tyr Arg Gly Ala Lys Arg
            100                 105                 110

Met Ala Ile Leu Gly Glu Thr Ala Trp Asp Phe Gly Ser Val Gly Gly
        115                 120                 125

Leu Leu Thr Ser Leu Gly Lys Ala Val His Gln Val Phe Gly
    130                 135                 140

<210>  14
<211>  117
<212>  PRT
<213>  Dengue virus type 4

<400>  14

Thr Met Cys Ser Gly Lys Phe Ser Ile Asp Lys Glu Met Ala Glu Thr
1               5                   10                  15

Gln His Gly Thr Thr Val Val Lys Val Lys Tyr Glu Gly Ala Gly Ala
            20                  25                  30

Pro Cys Lys Val Pro Ile Glu Ile Arg Asp Val Asn Lys Glu Lys Val
        35                  40                  45

Val Gly Arg Ile Ile Ser Ser Thr Pro Phe Ala Glu Tyr Thr Asn Ser
    50                  55                  60

Val Thr Asn Ile Glu Leu Glu Pro Pro Phe Gly Asp Ser Tyr Ile Val
65                  70                  75                  80

Ile Gly Val Gly Asp Ser Ala Leu Thr Leu His Trp Phe Arg Lys Gly
                85                  90                  95

Ser Ser Ile Gly Lys Met Leu Glu Ser Thr Tyr Arg Gly Ala Lys Arg
            100                 105                 110

Met Ala Ile Leu Gly
        115

<210>  15

<211>  96
<212>  PRT
<213>  Dengue virus type 4

<400>  15

Thr Met Cys Ser Gly Lys Phe Ser Ile Asp Lys Glu Met Ala Glu Thr
1               5                   10                  15

Gln His Gly Thr Thr Val Val Lys Val Lys Tyr Glu Gly Ala Gly Ala
            20                  25                  30

Pro Cys Lys Val Pro Ile Glu Ile Arg Asp Val Asn Lys Glu Lys Val
        35                  40                  45

Val Gly Arg Ile Ile Ser Ser Thr Pro Phe Ala Glu Tyr Thr Asn Ser
    50                  55                  60

Val Thr Asn Ile Glu Leu Glu Pro Pro Phe Gly Asp Ser Tyr Ile Val
65                  70                  75                  80

Ile Gly Val Gly Asp Ser Ala Leu Thr Leu His Trp Phe Arg Lys Gly
                85                  90                  95

<210>  16
<211>  100
<212>  PRT
<213>  Artificial Sequence

<220>
<223>  Dengue virus serotype 4, ED3 domain, consensus polypeptide
       sequence

<400>  16

Met Ser Tyr Thr Met Cys Ser Gly Lys Phe Ser Ile Asp Lys Glu Met
1               5                   10                  15

Ala Glu Thr Gln His Gly Thr Thr Val Val Lys Val Lys Tyr Glu Gly
            20                  25                  30

Ala Gly Ala Pro Cys Lys Val Pro Ile Glu Ile Arg Asp Val Asn Lys
        35                  40                  45

Glu Lys Val Val Gly Arg Ile Ile Ser Ser Thr Pro Phe Ala Glu Asn
    50                  55                  60

Thr Asn Ser Val Thr Asn Ile Glu Leu Glu Pro Pro Phe Gly Asp Ser
65                  70                  75                  80

Tyr Ile Val Ile Gly Val Gly Asp Ser Ala Leu Thr Leu His Trp Phe
                85                  90                  95

Arg Lys Gly Ser
            100

<210>  17
<211>  101
<212>  PRT
<213>  West Nile virus

<400>  17

Thr Thr Tyr Gly Val Cys Ser Lys Ala Phe Lys Phe Ala Arg Thr Pro
1               5                   10                  15

Ala Asp Thr Gly His Gly Thr Val Val Leu Glu Leu Gln Tyr Thr Gly
            20                  25                  30

Thr Asp Gly Pro Cys Lys Val Pro Ile Ser Ser Val Ala Ser Leu Asn
        35                  40                  45

Asp Leu Thr Pro Val Gly Arg Leu Val Thr Val Asn Pro Phe Val Ser
    50                  55                  60

Val Ala Thr Ala Asn Ser Lys Val Leu Ile Glu Leu Glu Pro Pro Phe
65                  70                  75                  80

Gly Asp Ser Tyr Ile Val Val Gly Arg Gly Glu Gln Gln Ile Asn His
                85                  90                  95

His Trp His Lys Ser
            100

<210>  18
<211>  103
<212>  PRT
<213>  Artificial Sequence

<220>
<223>  West Nile virus, ED3 domain, consensus polypeptide sequence

<400>  18

Thr Thr Tyr Gly Val Cys Ser Lys Ala Phe Lys Phe Leu Gly Thr Pro
1               5                   10                  15

Ala Asp Thr Gly His Gly Thr Val Val Leu Glu Leu Gln Tyr Thr Gly
            20                  25                  30

Thr Asp Gly Pro Cys Lys Val Pro Ile Ser Ser Val Ala Ser Leu Asn
        35                  40                  45

Asp Leu Thr Pro Val Gly Arg Leu Val Thr Val Asn Pro Phe Val Ser
    50                  55                  60

Val Ala Thr Ala Asn Ala Lys Val Leu Ile Glu Leu Glu Pro Pro Phe
65                  70                  75                  80

Gly Asp Ser Tyr Ile Val Val Gly Arg Gly Glu Gln Gln Ile Asn His
                85                  90                  95

His Trp His Lys Ser Gly Ser
            100

<210>  19
<211>  99
<212>  PRT
<213>  Japanese encephalitis virus

<400>  19

Met Cys Thr Glu Lys Phe Ser Phe Ser Lys Asn Pro Ala Asp Thr Gly
1               5                   10                  15

His Gly Thr Val Val Ile Glu Leu Gln Tyr Thr Gly Thr Asp Gly Pro
            20                  25                  30

Cys Lys Ile Pro Ile Ser Ser Val Ala Ser Leu Asn Asp Leu Thr Pro
        35                  40                  45

Val Gly Arg Leu Val Thr Val Asn Pro Phe Val Ala Thr Ser Thr Ala
    50                  55                  60

Asn Ser Lys Val Leu Val Glu Leu Glu Pro Pro Phe Gly Asp Ser Phe
65                  70                  75                  80

Ile Val Val Gly Arg Gly Asp Lys Gln Ile Asn His His Trp His Lys
                85                  90                  95

Ala Gly Ser

<210>  20
<211>  103
<212>  PRT
<213>  Artificial Sequence

<220>
<223>  Japanese encephalitis virus, ED3 domain, consensus polypeptide
       sequence

<400>  20

Thr Thr Tyr Gly Met Cys Thr Glu Lys Phe Ser Phe Ala Lys Asn Pro

1               5                   10                  15

Ala Asp Thr Gly His Gly Thr Val Val Ile Glu Leu Thr Tyr Ser Gly
            20                  25                  30

Ser Asp Gly Pro Cys Lys Ile Pro Ile Val Ser Val Ala Ser Leu Asn
        35                  40                  45

Asp Met Thr Pro Val Gly Arg Leu Val Thr Val Asn Pro Phe Val Ala
    50                  55                  60

Thr Ser Ser Ser Asn Ser Lys Val Leu Val Glu Met Glu Pro Pro Phe
65                  70                  75                  80

Gly Asp Ser Tyr Ile Val Val Gly Arg Gly Asp Lys Gln Ile Asn His
                85                  90                  95

His Trp His Lys Ala Gly Ser
            100

<210>  21
<211>  94
<212>  PRT
<213>  Tick-borne encephalitis virus

<400>  21

Leu Thr Tyr Thr Met Cys Asp Lys Thr Lys Phe Thr Trp Lys Arg Ile
1               5                   10                  15

Pro Thr Asp Ser Gly His Asp Thr Val Val Met Glu Val Ala Phe Ser
            20                  25                  30

Gly Thr Lys Pro Cys Arg Ile Pro Val Arg Ala Val Ala His Gly Ser
        35                  40                  45

Pro Asp Val Asn Val Ala Met Leu Ile Thr Pro Asn Pro Thr Ile Glu
    50                  55                  60

Thr Asn Gly Gly Gly Phe Ile Glu Met Gln Leu Pro Pro Gly Asp Asn
65                  70                  75                  80

Ile Ile Tyr Val Gly Glu Leu Ser His Gln Trp Phe Gln Lys
                85                  90

<210>  22
<211>  96
<212>  PRT
<213>  Artificial Sequence

<220>
<223>  Tick-borne encephalitis virus, ED3 domain, consensus polypeptide
      sequence

<400>  22

Leu Thr Tyr Thr Met Cys Asp Lys Thr Lys Phe Thr Trp Lys Arg Ala
1               5                   10                  15

Pro Thr Asp Ser Gly His Asp Thr Val Val Met Glu Val Thr Phe Ser
            20                  25                  30

Gly Thr Lys Pro Cys Arg Ile Pro Val Arg Ala Val Ala His Gly Ser
        35                  40                  45

Pro Asp Val Asn Val Ala Met Leu Ile Thr Pro Asn Pro Thr Ile Glu
    50                  55                  60

Asn Asn Gly Gly Gly Phe Ile Glu Met Gln Leu Pro Pro Gly Asp Asn
65                  70                  75                  80

Ile Ile Tyr Val Gly Glu Leu Ser His Gln Trp Phe Gln Lys Gly Ser
                85                  90                  95

<210>  23
<211>  100
<212>  PRT
<213>  Artificial Sequence

<220>
<223>  Yellow fever virus, ED3 domain, consensus polypeptide sequence

<400>  23

Thr Ser Tyr Lys Met Cys Thr Asp Lys Met Ser Phe Val Lys Asn Pro
1               5                   10                  15

Thr Asp Thr Gly His Gly Thr Val Val Met Gln Val Lys Val Pro Lys
            20                  25                  30

Gly Ala Pro Cys Arg Ile Pro Val Ile Val Ala Asp Asp Leu Thr Ala
        35                  40                  45

Ala Ile Asn Lys Gly Ile Leu Val Thr Val Asn Pro Ile Ala Ser Thr
    50                  55                  60

Asn Asp Asp Glu Val Leu Ile Glu Val Asn Pro Pro Phe Gly Asp Ser
65                  70                  75                  80

Tyr Ile Ile Val Gly Thr Gly Asp Ser Arg Leu Thr Tyr Gln Trp His
                85                  90                  95

Lys Glu Gly Ser
            100

<210>  24
<211>  3392
<212>  PRT
<213>  Dengue virus type 1

<400>  24

Met Asn Asn Gln Arg Lys Lys Thr Gly Arg Pro Ser Phe Asn Met Leu
1               5                   10                  15

Lys Arg Ala Arg Asn Arg Val Ser Thr Val Ser Gln Leu Ala Lys Arg
            20                  25                  30

Phe Ser Lys Gly Leu Leu Ser Gly Gln Gly Pro Met Lys Leu Val Met
        35                  40                  45

Ala Phe Ile Ala Phe Leu Arg Phe Leu Ala Ile Pro Pro Thr Ala Gly
    50                  55                  60

Ile Leu Ala Arg Trp Gly Ser Phe Lys Lys Asn Gly Ala Ile Lys Val
65                  70                  75                  80

Leu Arg Gly Phe Lys Lys Glu Ile Ser Asn Met Leu Asn Ile Met Asn
                85                  90                  95

Arg Arg Lys Arg Ser Val Thr Met Leu Leu Met Leu Leu Pro Thr Ala
            100                 105                 110

Leu Ala Phe His Leu Thr Thr Arg Gly Gly Glu Pro His Met Ile Val
        115                 120                 125

Ser Lys Gln Glu Arg Gly Lys Ser Leu Leu Phe Lys Thr Ser Ala Gly
    130                 135                 140

Val Asn Met Cys Thr Leu Ile Ala Met Asp Leu Gly Glu Leu Cys Glu
145                 150                 155                 160

Asp Thr Met Thr Tyr Lys Cys Pro Arg Ile Thr Glu Thr Glu Pro Asp
                165                 170                 175

Asp Val Asp Cys Trp Cys Asn Ala Thr Glu Thr Trp Val Thr Tyr Gly
            180                 185                 190

Thr Cys Ser Gln Thr Gly Glu His Arg Arg Asp Lys Arg Ser Val Ala
        195                 200                 205

Leu Ala Pro His Val Gly Leu Gly Leu Glu Thr Arg Thr Glu Thr Trp
    210                 215                 220

Met Ser Ser Glu Gly Ala Trp Lys Gln Ile Gln Lys Val Glu Thr Trp
225                 230                 235                 240

Ala Leu Arg His Pro Gly Phe Thr Val Ile Ala Leu Phe Leu Ala His
                245                 250                 255

Ala Ile Gly Thr Ser Ile Thr Gln Lys Gly Ile Ile Phe Ile Leu Leu
            260                 265                 270

Met Leu Val Thr Pro Ser Met Ala Met Arg Cys Val Gly Ile Gly Asn
        275                 280                 285

Arg Asp Phe Val Glu Gly Leu Ser Gly Ala Thr Trp Val Asp Val Val
    290                 295                 300

Leu Glu His Gly Ser Cys Val Thr Thr Met Ala Lys Asp Lys Pro Thr
305                 310                 315                 320

Leu Asp Ile Glu Leu Leu Lys Thr Glu Val Thr Asn Pro Ala Val Leu
                325                 330                 335

Arg Lys Leu Cys Ile Glu Ala Lys Ile Ser Asn Thr Thr Thr Asp Ser
            340                 345                 350

Arg Cys Pro Thr Gln Gly Glu Ala Thr Leu Val Glu Glu Gln Asp Thr
        355                 360                 365

Asn Phe Val Cys Arg Arg Thr Phe Val Asp Arg Gly Trp Gly Asn Gly
    370                 375                 380

Cys Gly Leu Phe Gly Lys Gly Ser Leu Ile Thr Cys Ala Lys Phe Lys
385                 390                 395                 400

Cys Val Thr Lys Leu Glu Gly Lys Ile Val Gln Tyr Glu Asn Leu Lys
                405                 410                 415

Tyr Ser Val Ile Val Thr Val His Thr Gly Asp Gln His Gln Val Gly
            420                 425                 430

Asn Glu Thr Thr Glu His Gly Thr Thr Ala Thr Ile Thr Pro Gln Ala
        435                 440                 445

Pro Thr Ser Glu Ile Gln Leu Thr Asp Tyr Gly Ala Leu Thr Leu Asp
    450                 455                 460

Cys Ser Pro Arg Thr Gly Leu Asp Phe Asn Glu Met Val Leu Leu Thr
465                 470                 475                 480

Met Glu Lys Lys Ser Trp Leu Val His Lys Gln Trp Phe Leu Asp Leu
                485                 490                 495

Pro Leu Pro Trp Thr Ser Gly Ala Ser Thr Ser Gln Glu Thr Trp Asn
            500                 505                 510

Arg Gln Asp Leu Leu Val Thr Phe Lys Thr Ala His Ala Lys Lys Gln
        515                 520                 525

Glu Val Val Val Leu Gly Ser Gln Glu Gly Ala Met His Thr Ala Leu
    530                 535                 540

Thr Gly Ala Thr Glu Ile Gln Thr Ser Gly Thr Thr Thr Ile Phe Ala
545                 550                 555                 560

Gly His Leu Lys Cys Arg Leu Lys Met Asp Lys Leu Thr Leu Lys Gly
                565                 570                 575

Met Ser Tyr Val Met Cys Thr Gly Ser Phe Lys Leu Glu Lys Glu Val
            580                 585                 590

Ala Glu Thr Gln His Gly Thr Val Leu Val Gln Val Lys Tyr Glu Gly
        595                 600                 605

Thr Asp Ala Pro Cys Lys Ile Pro Phe Ser Ser Gln Asp Glu Lys Gly
    610                 615                 620

Val Thr Gln Asn Gly Arg Leu Ile Thr Ala Asn Pro Ile Val Thr Asp
625                 630                 635                 640

Lys Glu Lys Pro Val Asn Ile Glu Ala Glu Pro Pro Phe Gly Glu Ser
                645                 650                 655

Tyr Ile Val Val Gly Ala Gly Glu Lys Ala Leu Lys Leu Ser Trp Phe
            660                 665                 670

Lys Lys Gly Ser Ser Ile Gly Lys Met Phe Glu Ala Thr Ala Arg Gly
        675                 680                 685

Ala Arg Arg Met Ala Ile Leu Gly Asp Thr Ala Trp Asp Phe Gly Ser
    690                 695                 700

Ile Gly Gly Val Phe Thr Ser Val Gly Lys Leu Ile His Gln Ile Phe

705                 710                 715                 720

Gly Thr Ala Tyr Gly Val Leu Phe Ser Gly Val Ser Trp Thr Met Lys
                725                 730                 735

Ile Gly Ile Gly Ile Leu Leu Thr Trp Leu Gly Leu Asn Ser Arg Ser
            740                 745                 750

Thr Ser Leu Ser Met Thr Cys Ile Ala Val Gly Met Val Thr Leu Tyr
        755                 760                 765

Leu Gly Val Met Val Gln Ala Asp Ser Gly Cys Val Ile Asn Trp Lys
    770                 775                 780

Gly Arg Glu Leu Lys Cys Gly Ser Gly Ile Phe Val Thr Asn Glu Val
785                 790                 795                 800

His Thr Trp Thr Glu Gln Tyr Lys Phe Gln Ala Asp Ser Pro Lys Arg
                805                 810                 815

Leu Ser Ala Ala Ile Gly Lys Ala Trp Glu Glu Gly Val Cys Gly Ile
            820                 825                 830

Arg Ser Ala Thr Arg Leu Glu Asn Ile Met Trp Lys Gln Ile Ser Asn
        835                 840                 845

Glu Leu Asn His Ile Leu Leu Glu Asn Asp Met Lys Phe Thr Val Val
    850                 855                 860

Val Gly Asp Val Ser Gly Ile Leu Ala Gln Gly Lys Lys Met Ile Arg
865                 870                 875                 880

Pro Gln Pro Met Glu His Lys Tyr Ser Trp Lys Ser Trp Gly Lys Ala
                885                 890                 895

Lys Ile Ile Gly Ala Asp Val Gln Asn Thr Thr Phe Ile Ile Asp Gly
            900                 905                 910

Pro Asn Thr Pro Glu Cys Pro Asp Asn Gln Arg Ala Trp Asn Ile Trp
        915                 920                 925

Glu Val Glu Asp Tyr Gly Phe Gly Ile Phe Thr Thr Asn Ile Trp Leu
    930                 935                 940

Lys Leu Arg Asp Ser Tyr Thr Gln Val Cys Asp His Arg Leu Met Ser
945                 950                 955                 960

Ala Ala Ile Lys Asp Ser Lys Ala Val His Ala Asp Met Gly Tyr Trp
                965                 970                 975

Ile Glu Ser Glu Lys Asn Glu Thr Trp Lys Leu Ala Arg Ala Ser Phe
            980                 985                 990

Ile Glu Val Lys Thr Cys Ile Trp Pro Lys Ser His Thr Leu Trp Ser
        995                 1000                1005

Asn Gly Val Leu Glu Ser Glu Met Ile Ile Pro Lys Ile Tyr Gly
    1010                1015                1020

Gly Pro Ile Ser Gln His Asn Tyr Arg Pro Gly Tyr Phe Thr Gln
    1025                1030                1035

Thr Ala Gly Pro Trp His Leu Gly Lys Leu Glu Leu Asp Phe Asp
    1040                1045                1050

Leu Cys Glu Gly Thr Thr Val Val Val Asp Glu His Cys Gly Asn
    1055                1060                1065

Arg Gly Pro Ser Leu Arg Thr Thr Thr Val Thr Gly Lys Thr Ile
    1070                1075                1080

His Glu Trp Cys Cys Arg Ser Cys Thr Leu Pro Pro Leu Arg Phe
    1085                1090                1095

Lys Gly Glu Asp Gly Cys Trp Tyr Gly Met Glu Ile Arg Pro Val
    1100                1105                1110

Lys Glu Lys Glu Glu Asn Leu Val Lys Ser Met Val Ser Ala Gly
    1115                1120                1125

Ser Gly Glu Val Asp Ser Phe Ser Leu Gly Leu Leu Cys Ile Ser
    1130                1135                1140

Ile Met Ile Glu Glu Val Met Arg Ser Arg Trp Ser Arg Lys Met
    1145                1150                1155

Leu Met Thr Gly Thr Leu Ala Val Phe Leu Leu Leu Thr Met Gly
    1160                1165                1170

Gln Leu Thr Trp Asn Asp Leu Ile Arg Leu Cys Ile Met Val Gly
    1175                1180                1185

Ala Asn Ala Ser Asp Lys Met Gly Met Gly Thr Thr Tyr Leu Ala
    1190                1195                1200

Leu Met Ala Thr Phe Arg Met Arg Pro Met Phe Ala Val Gly Leu
    1205                1210                1215

Leu Phe Arg Arg Leu Thr Ser Arg Glu Val Leu Leu Leu Thr Val
    1220                1225                1230

Gly Leu Ser Leu Val Ala Ser Val Glu Leu Pro Asn Ser Leu Glu
    1235                1240                1245

Glu Leu Gly Asp Gly Leu Ala Met Gly Ile Met Met Leu Lys Leu
    1250                1255                1260

Leu Thr Asp Phe Gln Ser His Gln Leu Trp Ala Thr Leu Leu Ser
    1265                1270                1275

Leu Thr Phe Val Lys Thr Thr Phe Ser Leu His Tyr Ala Trp Lys
    1280                1285                1290

Thr Met Ala Met Ile Leu Ser Ile Val Ser Leu Phe Pro Leu Cys
    1295                1300                1305

Leu Ser Thr Thr Ser Gln Lys Thr Thr Trp Leu Pro Val Leu Leu
    1310                1315                1320

Gly Ser Leu Gly Cys Lys Pro Leu Thr Met Phe Leu Ile Thr Glu
    1325                1330                1335

Asn Lys Ile Trp Gly Arg Lys Ser Trp Pro Leu Asn Glu Gly Ile
    1340                1345                1350

Met Ala Val Gly Ile Val Ser Ile Leu Leu Ser Ser Leu Leu Lys
    1355                1360                1365

Asn Asp Val Pro Leu Ala Gly Pro Leu Ile Ala Gly Gly Met Leu
    1370                1375                1380

Ile Ala Cys Tyr Val Ile Ser Gly Ser Ser Ala Asp Leu Ser Leu
    1385                1390                1395

Glu Lys Ala Ala Glu Val Ser Trp Glu Glu Glu Ala Glu His Ser
    1400                1405                1410

Gly Ala Ser His Asn Ile Leu Val Glu Val Gln Asp Asp Gly Thr
    1415                1420                1425

Met Lys Ile Lys Asp Glu Glu Arg Asp Asp Thr Leu Thr Ile Leu
    1430                1435                1440

Leu Lys Ala Thr Leu Leu Ala Ile Ser Gly Val Tyr Pro Met Ser
    1445                1450                1455

Ile Pro Ala Thr Leu Phe Val Trp Tyr Phe Trp Gln Lys Lys Lys
    1460                1465                1470

Gln Arg Ser Gly Val Leu Trp Asp Thr Pro Ser Pro Pro Glu Val
    1475                1480                1485

Glu Arg Ala Val Leu Asp Asp Gly Ile Tyr Arg Ile Leu Gln Arg
    1490                1495                1500

Gly Leu Leu Gly Arg Ser Gln Val Gly Val Gly Val Phe Gln Glu
    1505                1510                1515

Gly Val Phe His Thr Met Trp His Val Thr Arg Gly Ala Val Leu
    1520                1525                1530

Met Tyr Gln Gly Lys Arg Leu Glu Pro Ser Trp Ala Ser Val Lys
    1535                1540                1545

Lys Asp Leu Ile Ser Tyr Gly Gly Gly Trp Arg Phe Gln Gly Ser
    1550                1555                1560

Trp Asn Ala Gly Glu Glu Val Gln Val Ile Ala Val Glu Pro Gly
    1565                1570                1575

Lys Asn Pro Lys Asn Val Gln Thr Ala Pro Gly Thr Phe Lys Thr
    1580                1585                1590

Pro Glu Gly Glu Val Gly Ala Ile Ala Leu Asp Phe Lys Pro Gly
    1595                1600                1605

Thr Ser Gly Ser Pro Ile Val Asn Arg Glu Gly Lys Ile Val Gly
    1610                1615                1620

Leu Tyr Gly Asn Gly Val Val Thr Thr Ser Gly Thr Tyr Val Ser
    1625                1630                1635

Ala Ile Ala Gln Ala Lys Ala Ser Gln Glu Gly Pro Leu Pro Glu
    1640                1645                1650

Ile Glu Asp Glu Val Phe Arg Lys Arg Asn Leu Thr Ile Met Asp
    1655                1660                1665

Leu His Pro Gly Ser Gly Lys Thr Arg Arg Tyr Leu Pro Ala Ile

1670                1675                1680

Val Arg Glu Ala Ile Arg Arg Asn Val Arg Thr Leu Val Leu Ala
    1685                1690                1695

Pro Thr Arg Val Val Ala Ser Glu Met Ala Glu Ala Leu Lys Gly
    1700                1705                1710

Met Pro Ile Arg Tyr Gln Thr Thr Ala Val Lys Ser Glu His Thr
    1715                1720                1725

Gly Lys Glu Ile Val Asp Leu Met Cys His Ala Thr Phe Thr Met
    1730                1735                1740

Arg Leu Leu Ser Pro Val Arg Val Pro Asn Tyr Asn Met Ile Ile
    1745                1750                1755

Met Asp Glu Ala His Phe Thr Asp Pro Ala Ser Ile Ala Ala Arg
    1760                1765                1770

Gly Tyr Ile Ser Thr Arg Val Gly Met Gly Glu Ala Ala Ala Ile
    1775                1780                1785

Phe Met Thr Ala Thr Pro Pro Gly Ser Val Glu Ala Phe Pro Gln
    1790                1795                1800

Ser Asn Ala Val Ile Gln Asp Glu Glu Arg Asp Ile Pro Glu Arg
    1805                1810                1815

Ser Trp Asn Ser Gly Tyr Asp Trp Ile Thr Asp Phe Pro Gly Lys
    1820                1825                1830

Thr Val Trp Phe Val Pro Ser Ile Lys Ser Gly Asn Asp Ile Ala
    1835                1840                1845

Asn Cys Leu Arg Lys Asn Gly Lys Arg Val Val Gln Leu Ser Arg
    1850                1855                1860

Lys Thr Phe Asp Thr Glu Tyr Gln Lys Thr Lys Asn Asn Asp Trp
    1865                1870                1875

Asp Tyr Val Val Thr Thr Asp Ile Ser Glu Met Gly Ala Asn Phe
    1880                1885                1890

Arg Ala Asp Arg Val Ile Asp Pro Arg Arg Cys Leu Lys Pro Val
    1895                1900                1905

Ile Leu Lys Asp Gly Pro Glu Arg Val Ile Leu Ala Gly Pro Met
    1910                1915                1920

Pro Val Thr Val Ala Ser Ala Ala Gln Arg Arg Gly Arg Ile Gly
    1925                1930                1935

Arg Asn Gln Asn Lys Glu Gly Asp Gln Tyr Ile Tyr Met Gly Gln
    1940                1945                1950

Pro Leu Asn Asn Asp Glu Asp His Ala His Trp Thr Glu Ala Lys
    1955                1960                1965

Met Leu Leu Asp Asn Ile Asn Thr Pro Glu Gly Ile Ile Pro Ala
    1970                1975                1980

Leu Phe Glu Pro Glu Arg Glu Lys Ser Ala Ala Ile Asp Gly Glu
    1985                1990                1995

Tyr Arg Leu Arg Gly Glu Ala Arg Lys Thr Phe Val Glu Leu Met
    2000                2005                2010

Arg Arg Gly Asp Leu Pro Val Trp Leu Ser Tyr Lys Val Ala Ser
    2015                2020                2025

Glu Gly Phe Gln Tyr Ser Asp Arg Arg Trp Cys Phe Asp Gly Glu
    2030                2035                2040

Arg Asn Asn Gln Val Leu Glu Glu Asn Met Asp Val Glu Ile Trp
    2045                2050                2055

Thr Lys Glu Gly Glu Arg Lys Lys Leu Arg Pro Arg Trp Leu Asp
    2060                2065                2070

Ala Arg Thr Tyr Ser Asp Pro Leu Ala Leu Arg Glu Phe Lys Glu
    2075                2080                2085

Phe Ala Ala Gly Arg Arg Ser Val Ser Gly Asp Leu Ile Leu Glu
    2090                2095                2100

Ile Gly Lys Leu Pro Gln His Leu Thr Gln Arg Ala Gln Asn Ala
    2105                2110                2115

Leu Asp Asn Leu Val Met Leu His Asn Ser Glu Gln Gly Gly Lys
    2120                2125                2130

Ala Tyr Arg His Ala Met Glu Glu Leu Pro Asp Thr Ile Glu Thr
    2135                2140                2145

Leu Met Leu Leu Ala Leu Ile Ala Val Leu Thr Gly Gly Val Thr
    2150                2155                2160

Leu Phe Phe Leu Ser Gly Arg Gly Leu Gly Lys Thr Ser Ile Gly
    2165                2170                2175

Leu Leu Cys Val Ile Ala Ser Ser Ala Leu Leu Trp Met Ala Ser
    2180                2185                2190

Val Glu Pro His Trp Ile Ala Ala Ser Ile Ile Leu Glu Phe Phe
    2195                2200                2205

Leu Met Val Leu Leu Ile Pro Glu Pro Asp Arg Gln Arg Thr Pro
    2210                2215                2220

Gln Asp Asn Gln Leu Ala Tyr Val Val Ile Gly Leu Leu Phe Met
    2225                2230                2235

Ile Leu Thr Ala Ala Ala Asn Glu Met Gly Leu Leu Glu Thr Thr
    2240                2245                2250

Lys Lys Asp Leu Gly Ile Gly His Ala Ala Ala Glu Asn His His
    2255                2260                2265

His Ala Ala Met Leu Asp Val Asp Leu His Pro Ala Ser Ala Trp
    2270                2275                2280

Thr Leu Tyr Ala Val Ala Thr Thr Ile Ile Thr Pro Met Met Arg
    2285                2290                2295

His Thr Ile Glu Asn Thr Thr Ala Asn Ile Ser Leu Thr Ala Ile
    2300                2305                2310

Ala Asn Gln Ala Ala Ile Leu Met Gly Leu Asp Lys Gly Trp Pro
    2315                2320                2325

Ile Ser Lys Met Asp Ile Gly Val Pro Leu Leu Ala Leu Gly Cys
    2330                2335                2340

Tyr Ser Gln Val Asn Pro Leu Thr Leu Thr Ala Ala Val Phe Met
    2345                2350                2355

Leu Val Ala His Tyr Ala Ile Ile Gly Pro Gly Leu Gln Ala Lys
    2360                2365                2370

Ala Thr Arg Glu Ala Gln Lys Arg Thr Ala Ala Gly Ile Met Lys
    2375                2380                2385

Asn Pro Thr Val Asp Gly Ile Val Ala Ile Asp Leu Asp Pro Val
    2390                2395                2400

Val Tyr Asp Ala Lys Phe Glu Lys Gln Leu Gly Gln Ile Met Leu
    2405                2410                2415

Leu Ile Leu Cys Thr Ser Gln Ile Leu Leu Met Arg Thr Thr Trp
    2420                2425                2430

Ala Leu Cys Glu Ser Ile Thr Leu Ala Thr Gly Pro Leu Thr Thr
    2435                2440                2445

Leu Trp Glu Gly Ser Pro Gly Lys Phe Trp Asn Thr Thr Ile Ala
    2450                2455                2460

Val Ser Met Ala Asn Ile Phe Arg Gly Ser Tyr Leu Ala Gly Ala
    2465                2470                2475

Gly Leu Ala Phe Ser Leu Met Lys Ser Leu Gly Gly Gly Arg Arg
    2480                2485                2490

Gly Thr Gly Ala Gln Gly Glu Thr Leu Gly Glu Lys Trp Lys Arg
    2495                2500                2505

Gln Leu Asn Gln Leu Ser Lys Ser Glu Phe Asn Thr Tyr Lys Arg
    2510                2515                2520

Ser Gly Ile Ile Glu Val Asp Arg Ser Glu Ala Lys Glu Gly Leu
    2525                2530                2535

Lys Arg Gly Glu Pro Thr Lys His Ala Val Ser Arg Gly Thr Ala
    2540                2545                2550

Lys Leu Arg Trp Phe Val Glu Arg Asn Leu Val Lys Pro Glu Gly
    2555                2560                2565

Lys Val Ile Asp Leu Gly Cys Gly Arg Gly Gly Trp Ser Tyr Tyr
    2570                2575                2580

Cys Ala Gly Leu Lys Lys Val Thr Glu Val Lys Gly Tyr Thr Lys
    2585                2590                2595

Gly Gly Pro Gly His Glu Glu Pro Ile Pro Met Ala Thr Tyr Gly
    2600                2605                2610

Trp Asn Leu Val Lys Leu Tyr Ser Gly Lys Asp Val Phe Phe Thr

2615                2620                2625

Pro Pro Glu Lys Cys Asp Thr Leu Leu Cys Asp Ile Gly Glu Ser
    2630                2635                2640

Ser Pro Asn Pro Thr Ile Glu Glu Gly Arg Thr Leu Arg Val Leu
    2645                2650                2655

Lys Met Val Glu Pro Trp Leu Arg Gly Asn Gln Phe Cys Ile Lys
    2660                2665                2670

Ile Leu Asn Pro Tyr Met Pro Ser Val Val Glu Thr Leu Glu Gln
    2675                2680                2685

Met Gln Arg Lys His Gly Gly Met Leu Val Arg Asn Pro Leu Ser
    2690                2695                2700

Arg Asn Ser Thr His Glu Met Tyr Trp Val Ser Cys Gly Thr Gly
    2705                2710                2715

Asn Ile Val Ser Ala Val Asn Met Thr Ser Arg Met Leu Leu Asn
    2720                2725                2730

Arg Phe Thr Met Ala His Arg Lys Pro Thr Tyr Glu Arg Asp Val
    2735                2740                2745

Asp Leu Gly Ala Gly Thr Arg His Val Ala Val Glu Pro Glu Val
    2750                2755                2760

Ala Asn Leu Asp Ile Ile Gly Gln Arg Ile Glu Asn Ile Lys Asn
    2765                2770                2775

Gly His Lys Ser Thr Trp His Tyr Asp Glu Asp Asn Pro Tyr Lys
    2780                2785                2790

Thr Trp Ala Tyr His Gly Ser Tyr Glu Val Lys Pro Ser Gly Ser
    2795                2800                2805

Ala Ser Ser Met Val Asn Gly Val Val Arg Leu Leu Thr Lys Pro
    2810                2815                2820

Trp Asp Val Ile Pro Met Val Thr Gln Ile Ala Met Thr Asp Thr
    2825                2830                2835

Thr Pro Phe Gly Gln Gln Arg Val Phe Lys Glu Lys Val Asp Thr
    2840                2845                2850

Arg Thr Pro Lys Ala Lys Arg Gly Thr Ala Gln Ile Met Glu Val
    2855                2860                2865

Thr Ala Arg Trp Leu Trp Gly Phe Leu Ser Arg Asn Lys Lys Pro
    2870                2875                2880

Arg Ile Cys Thr Arg Glu Glu Phe Thr Arg Lys Val Arg Ser Asn
    2885                2890                2895

Ala Ala Ile Gly Ala Val Phe Val Asp Glu Asn Gln Trp Asn Ser
    2900                2905                2910

Ala Lys Glu Ala Val Glu Asp Glu Arg Phe Trp Asp Leu Val His
    2915                2920                2925

Arg Glu Arg Glu Leu His Lys Gln Gly Lys Cys Ala Thr Cys Val
    2930                2935                2940

Tyr Asn Met Met Gly Lys Arg Glu Lys Lys Leu Gly Glu Phe Gly
    2945                2950                2955

Lys Ala Lys Gly Ser Arg Ala Ile Trp Tyr Met Trp Leu Gly Ala
    2960                2965                2970

Arg Phe Leu Glu Phe Glu Ala Leu Gly Phe Met Asn Glu Asp His
    2975                2980                2985

Trp Phe Ser Arg Glu Asn Ser Leu Ser Gly Val Glu Gly Glu Gly
    2990                2995                3000

Leu His Lys Leu Gly Tyr Ile Leu Arg Asp Ile Ser Lys Ile Pro
    3005                3010                3015

Gly Gly Asn Met Tyr Ala Asp Asp Thr Ala Gly Trp Asp Thr Arg
    3020                3025                3030

Ile Thr Glu Asp Asp Leu Gln Asn Glu Ala Lys Ile Thr Asp Ile
    3035                3040                3045

Met Glu Pro Glu His Ala Leu Leu Ala Thr Ser Ile Phe Lys Leu
    3050                3055                3060

Thr Tyr Gln Asn Lys Val Val Arg Val Gln Arg Pro Ala Lys Asn
    3065                3070                3075

Gly Thr Val Met Asp Val Ile Ser Arg Arg Asp Gln Arg Gly Ser
    3080                3085                3090

Gly Gln Val Gly Thr Tyr Gly Leu Asn Thr Phe Thr Asn Met Glu
    3095                3100                3105

Ala Gln Leu Ile Arg Gln Met Glu Ser Glu Gly Ile Phe Ser Pro
    3110                3115                3120

Ser Glu Leu Glu Thr Pro Asn Leu Ala Glu Arg Val Leu Asp Trp
    3125                3130                3135

Leu Lys Lys His Gly Thr Glu Arg Leu Lys Arg Met Ala Ile Ser
    3140                3145                3150

Gly Asp Asp Cys Val Val Lys Pro Ile Asp Asp Arg Phe Ala Thr
    3155                3160                3165

Ala Leu Thr Ala Leu Asn Asp Met Gly Lys Val Arg Lys Asp Ile
    3170                3175                3180

Pro Gln Trp Glu Pro Ser Lys Gly Trp Asn Asp Trp Gln Gln Val
    3185                3190                3195

Pro Phe Cys Ser His His Phe His Gln Leu Ile Met Lys Asp Gly
    3200                3205                3210

Arg Glu Ile Val Val Pro Cys Arg Asn Gln Asp Glu Leu Val Gly
    3215                3220                3225

Arg Ala Arg Val Ser Gln Gly Ala Gly Trp Ser Leu Arg Glu Thr
    3230                3235                3240

Ala Cys Leu Gly Lys Ser Tyr Ala Gln Met Trp Gln Leu Met Tyr
    3245                3250                3255

Phe His Arg Arg Asp Leu Arg Leu Ala Ala Asn Ala Ile Cys Ser
    3260                3265                3270

Ala Val Pro Val Asp Trp Val Pro Thr Ser Arg Thr Thr Trp Ser
    3275                3280                3285

Ile His Ala His His Gln Trp Met Thr Thr Glu Asp Met Leu Ser
    3290                3295                3300

Val Trp Asn Arg Val Trp Ile Glu Glu Asn Pro Trp Met Glu Asp
    3305                3310                3315

Lys Thr His Val Ser Ser Trp Glu Asp Val Pro Tyr Leu Gly Lys
    3320                3325                3330

Arg Glu Asp Arg Trp Cys Gly Ser Leu Ile Gly Leu Thr Ala Arg
    3335                3340                3345

Ala Thr Trp Ala Thr Asn Ile Gln Val Ala Ile Asn Gln Val Arg
    3350                3355                3360

Arg Leu Ile Gly Asn Glu Asn Tyr Leu Asp Phe Met Thr Ser Met
    3365                3370                3375

Lys Arg Phe Lys Asn Glu Ser Asp Pro Glu Gly Ala Leu Trp
    3380                3385                3390

<210> 25
<211> 3391
<212> PRT
<213> Dengue virus type 2

<400> 25

Met Asn Asn Gln Arg Lys Lys Ala Lys Asn Thr Pro Phe Asn Met Leu
1               5                   10                  15

Lys Arg Glu Arg Asn Arg Val Ser Thr Val Gln Gln Leu Thr Lys Arg
            20                  25                  30

Phe Ser Leu Gly Met Leu Gln Gly Arg Gly Pro Leu Lys Leu Phe Met
        35                  40                  45

Ala Leu Val Ala Phe Leu Arg Phe Leu Thr Ile Pro Pro Thr Ala Gly
    50                  55                  60

Ile Leu Lys Arg Trp Gly Thr Ile Lys Lys Ser Lys Ala Ile Asn Val
65                  70                  75                  80

Leu Arg Gly Phe Arg Lys Glu Ile Gly Arg Met Leu Asn Ile Leu Asn
                85                  90                  95

Arg Arg Arg Arg Ser Ala Gly Met Ile Ile Met Leu Ile Pro Thr Val
            100                 105                 110

Met Ala Phe His Leu Thr Thr Arg Asn Gly Glu Pro His Met Ile Val
        115                 120                 125

Ser Arg Gln Glu Lys Gly Lys Ser Leu Leu Phe Lys Thr Glu Asp Gly
    130                 135                 140

Val Asn Met Cys Thr Leu Met Ala Met Asp Leu Gly Glu Leu Cys Glu
145                 150                 155                 160

Asp Thr Ile Thr Tyr Lys Cys Pro Leu Leu Arg Gln Asn Glu Pro Glu
                165                 170                 175

Asp Ile Asp Cys Trp Cys Asn Ser Thr Ser Thr Trp Val Thr Tyr Gly
            180                 185                 190

Thr Cys Thr Thr Met Gly Glu His Arg Arg Glu Lys Arg Ser Val Ala
        195                 200                 205

Leu Val Pro His Val Gly Met Gly Leu Glu Thr Arg Thr Glu Thr Trp
    210                 215                 220

Met Ser Ser Glu Gly Ala Trp Lys His Val Gln Arg Ile Glu Thr Trp
225                 230                 235                 240

Ile Leu Arg His Pro Gly Phe Thr Met Met Ala Ala Ile Leu Ala Tyr
                245                 250                 255

Thr Ile Gly Thr Thr His Phe Gln Arg Ala Leu Ile Phe Ile Leu Leu
            260                 265                 270

Thr Ala Val Thr Pro Ser Met Thr Met Arg Cys Ile Gly Met Ser Asn
        275                 280                 285

Arg Asp Phe Val Glu Gly Val Ser Gly Gly Ser Trp Val Asp Ile Val
    290                 295                 300

Leu Glu His Gly Ser Cys Val Thr Thr Met Ala Lys Asn Lys Pro Thr
305                 310                 315                 320

Leu Asp Phe Glu Leu Ile Lys Thr Glu Ala Lys Gln Pro Ala Thr Leu
                325                 330                 335

Arg Lys Tyr Cys Ile Glu Ala Lys Leu Thr Asn Thr Thr Thr Glu Ser
            340                 345                 350

Arg Cys Pro Thr Gln Gly Glu Pro Ser Leu Asn Glu Glu Gln Asp Lys
        355                 360                 365

Arg Phe Val Cys Lys His Ser Met Val Asp Arg Gly Trp Gly Asn Gly
    370                 375                 380

Cys Gly Leu Phe Gly Lys Gly Gly Ile Val Thr Cys Ala Met Phe Arg
385                 390                 395                 400

Cys Lys Lys Asn Met Glu Gly Lys Val Val Gln Pro Glu Asn Leu Glu

                405                 410                 415

Tyr Thr Ile Val Ile Thr Pro His Ser Gly Glu Glu His Ala Val Gly
            420                 425                 430

Asn Asp Thr Gly Lys His Gly Lys Glu Ile Lys Ile Thr Pro Gln Ser
        435                 440                 445

Ser Ile Thr Glu Ala Glu Leu Thr Gly Tyr Gly Thr Val Thr Met Glu
    450                 455                 460

Cys Ser Pro Arg Thr Gly Leu Asp Phe Asn Glu Met Val Leu Leu Gln
465                 470                 475                 480

Met Glu Asn Lys Ala Trp Leu Val His Arg Gln Trp Phe Leu Asp Leu
                485                 490                 495

Pro Leu Pro Trp Leu Pro Gly Ala Asp Thr Gln Gly Ser Asn Trp Ile
            500                 505                 510

Gln Lys Glu Thr Leu Val Thr Phe Lys Asn Pro His Ala Lys Lys Gln
        515                 520                 525

Asp Val Val Val Leu Gly Ser Gln Glu Gly Ala Met His Thr Ala Leu
    530                 535                 540

Thr Gly Ala Thr Glu Ile Gln Met Ser Ser Gly Asn Leu Leu Phe Thr
545                 550                 555                 560

Gly His Leu Lys Cys Arg Leu Arg Met Asp Lys Leu Gln Leu Lys Gly
                565                 570                 575

Met Ser Tyr Ser Met Cys Thr Gly Lys Phe Lys Val Val Lys Glu Ile
            580                 585                 590

Ala Glu Thr Gln His Gly Thr Ile Val Ile Arg Val Gln Tyr Glu Gly
        595                 600                 605

Asp Gly Ser Pro Cys Lys Ile Pro Phe Glu Ile Met Asp Leu Glu Lys
    610                 615                 620

Arg His Val Leu Gly Arg Leu Ile Thr Val Asn Pro Ile Val Thr Glu
625                 630                 635                 640

Lys Asp Ser Pro Val Asn Ile Glu Ala Glu Pro Pro Phe Gly Asp Ser
                645                 650                 655

Tyr Ile Ile Ile Gly Val Glu Pro Gly Gln Leu Lys Leu Asn Trp Phe
        660                 665                 670

Lys Lys Gly Ser Ser Ile Gly Gln Met Phe Glu Thr Thr Met Arg Gly
        675                 680                 685

Ala Lys Arg Met Ala Ile Leu Gly Asp Thr Ala Trp Asp Phe Gly Ser
    690                 695                 700

Leu Gly Gly Val Phe Thr Ser Ile Gly Lys Ala Leu His Gln Val Phe
705                 710                 715                 720

Gly Ala Ile Tyr Gly Ala Ala Phe Ser Gly Val Ser Trp Thr Met Lys
                725                 730                 735

Ile Leu Ile Gly Val Ile Ile Thr Trp Ile Gly Met Asn Ser Arg Ser
            740                 745                 750

Thr Ser Leu Ser Val Thr Leu Val Leu Val Gly Ile Val Thr Leu Tyr
        755                 760                 765

Leu Gly Val Met Val Gln Ala Asp Ser Gly Cys Val Val Ser Trp Lys
    770                 775                 780

Asn Lys Glu Leu Lys Cys Gly Ser Gly Ile Phe Ile Thr Asp Asn Val
785                 790                 795                 800

His Thr Trp Thr Glu Gln Tyr Lys Phe Gln Pro Glu Ser Pro Ser Lys
                805                 810                 815

Leu Ala Ser Ala Ile Gln Lys Ala His Glu Glu Gly Ile Cys Gly Ile
            820                 825                 830

Arg Ser Val Thr Arg Leu Glu Asn Leu Met Trp Lys Gln Ile Thr Pro
        835                 840                 845

Glu Leu Asn His Ile Leu Ser Glu Asn Glu Val Lys Leu Thr Ile Met
    850                 855                 860

Thr Gly Asp Ile Lys Gly Ile Met Gln Ala Gly Lys Arg Ser Leu Arg
865                 870                 875                 880

Pro Gln Pro Thr Glu Leu Lys Tyr Ser Trp Lys Thr Trp Gly Lys Ala
                885                 890                 895

Lys Met Leu Ser Thr Glu Ser His Asn Gln Thr Phe Leu Ile Asp Gly
            900                 905                 910

Pro Glu Thr Ala Glu Cys Pro Asn Thr Asn Arg Ala Trp Asn Ser Leu
        915                 920                 925

Glu Val Glu Asp Tyr Gly Phe Gly Val Phe Thr Thr Asn Ile Trp Leu
    930                 935                 940

Lys Leu Lys Glu Lys Gln Asp Val Phe Cys Asp Ser Lys Leu Met Ser
945                 950                 955                 960

Ala Ala Ile Lys Asp Asn Arg Ala Val His Ala Asp Met Gly Tyr Trp
                965                 970                 975

Ile Glu Ser Ala Leu Asn Asp Thr Trp Lys Ile Glu Lys Ala Ser Phe
            980                 985                 990

Ile Glu Val Lys Asn Cys His Trp Pro Lys Ser His Thr Leu Trp Ser
        995                 1000                1005

Asn Gly Val Leu Glu Ser Glu Met Ile Ile Pro Lys Asn Leu Ala
    1010                1015                1020

Gly Pro Val Ser Gln His Asn Tyr Arg Pro Gly Tyr His Thr Gln
    1025                1030                1035

Ile Thr Gly Pro Trp His Leu Gly Lys Leu Glu Met Asp Phe Asp
    1040                1045                1050

Phe Cys Asp Gly Thr Thr Val Val Val Thr Glu Asp Cys Gly Asn
    1055                1060                1065

Arg Gly Pro Ser Leu Arg Thr Thr Thr Ala Ser Gly Lys Leu Ile
    1070                1075                1080

Thr Glu Trp Cys Cys Arg Ser Cys Thr Leu Pro Pro Leu Arg Tyr
    1085                1090                1095

Arg Gly Glu Asp Gly Cys Trp Tyr Gly Met Glu Ile Arg Pro Leu
    1100                1105                1110

Lys Glu Lys Glu Glu Asn Leu Val Asn Ser Leu Val Thr Ala Gly
    1115                1120                1125

His Gly Gln Val Asp Asn Phe Ser Leu Gly Val Leu Gly Met Ala
    1130                1135                1140

Leu Phe Leu Glu Glu Met Leu Arg Thr Arg Val Gly Thr Lys His
    1145                1150                1155

Ala Ile Leu Leu Val Ala Val Ser Phe Val Thr Leu Ile Thr Gly
    1160                1165                1170

Asn Met Ser Phe Arg Asp Leu Gly Arg Val Met Val Met Val Gly
    1175                1180                1185

Ala Thr Met Thr Asp Asp Ile Gly Met Gly Val Thr Tyr Leu Ala
    1190                1195                1200

Leu Leu Ala Ala Phe Lys Val Arg Pro Thr Phe Ala Ala Gly Leu
    1205                1210                1215

Leu Leu Arg Lys Leu Thr Ser Lys Glu Leu Met Met Thr Thr Ile
    1220                1225                1230

Gly Ile Val Leu Leu Ser Gln Ser Thr Ile Pro Glu Thr Ile Leu
    1235                1240                1245

Glu Leu Thr Asp Ala Leu Ala Leu Gly Met Met Val Leu Lys Met
    1250                1255                1260

Val Arg Asn Met Glu Lys Tyr Gln Leu Ala Val Thr Ile Met Ala
    1265                1270                1275

Ile Leu Cys Val Pro Asn Ala Val Ile Leu Gln Asn Ala Trp Lys
    1280                1285                1290

Val Ser Cys Thr Ile Leu Ala Val Val Ser Val Ser Pro Leu Leu
    1295                1300                1305

Leu Thr Ser Ser Gln Gln Lys Thr Asp Trp Ile Pro Leu Ala Leu
    1310                1315                1320

Thr Ile Lys Gly Leu Asn Pro Thr Ala Ile Phe Leu Thr Thr Leu
    1325                1330                1335

Ser Arg Thr Ser Lys Lys Arg Ser Trp Pro Leu Asn Glu Ala Ile
    1340                1345                1350

Met Ala Val Gly Met Val Ser Ile Leu Ala Ser Ser Leu Leu Lys
    1355                1360                1365

Asn Asp Ile Pro Met Thr Gly Pro Leu Val Ala Gly Gly Leu Leu
    1370                1375                1380

Thr Val Cys Tyr Val Leu Thr Gly Arg Ser Ala Asp Leu Glu Leu

1385                1390                1395

Glu Arg Ala Ala Asp Val Lys Trp Glu Asp Gln Ala Glu Ile Ser
    1400                1405                1410

Gly Ser Ser Pro Ile Leu Ser Ile Thr Ile Ser Glu Asp Gly Ser
    1415                1420                1425

Met Ser Ile Lys Asn Glu Glu Glu Glu Gln Thr Leu Thr Ile Leu
    1430                1435                1440

Ile Arg Thr Gly Leu Leu Val Ile Ser Gly Leu Phe Pro Val Ser
    1445                1450                1455

Ile Pro Ile Thr Ala Ala Ala Trp Tyr Leu Trp Glu Val Lys Lys
    1460                1465                1470

Gln Arg Ala Gly Val Leu Trp Asp Val Pro Ser Pro Pro Pro Met
    1475                1480                1485

Gly Lys Ala Glu Leu Glu Asp Gly Ala Tyr Arg Ile Lys Gln Lys
    1490                1495                1500

Gly Ile Leu Gly Tyr Ser Gln Ile Gly Ala Gly Val Tyr Lys Glu
    1505                1510                1515

Gly Thr Phe His Thr Met Trp His Val Thr Arg Gly Ala Val Leu
    1520                1525                1530

Met His Lys Gly Lys Arg Ile Glu Pro Ser Trp Ala Asp Val Lys
    1535                1540                1545

Lys Asp Leu Ile Ser Tyr Gly Gly Gly Trp Lys Leu Glu Gly Glu
    1550                1555                1560

Trp Lys Glu Gly Glu Glu Val Gln Val Leu Ala Leu Glu Pro Gly
    1565                1570                1575

Lys Asn Pro Arg Ala Val Gln Thr Lys Pro Gly Leu Phe Lys Thr
    1580                1585                1590

Asn Ala Gly Thr Ile Gly Ala Val Ser Leu Asp Phe Ser Pro Gly
    1595                1600                1605

Thr Ser Gly Ser Pro Ile Ile Asp Lys Lys Gly Lys Val Val Gly
    1610                1615                1620

Leu Tyr Gly Asn Gly Val Val Thr Arg Ser Gly Ala Tyr Val Ser
    1625                1630                1635

Ala Ile Ala Gln Thr Glu Lys Ser Ile Glu Asp Asn Pro Glu Ile
    1640                1645                1650

Glu Asp Asp Ile Phe Arg Lys Arg Arg Leu Thr Ile Met Asp Leu
    1655                1660                1665

His Pro Gly Ala Gly Lys Thr Lys Arg Tyr Leu Pro Ala Ile Val
    1670                1675                1680

Arg Glu Ala Ile Lys Arg Gly Leu Arg Thr Leu Ile Leu Ala Pro
    1685                1690                1695

Thr Arg Val Val Ala Ala Glu Met Glu Glu Ala Leu Arg Gly Leu
    1700                1705                1710

Pro Ile Arg Tyr Gln Thr Pro Ala Ile Arg Ala Glu His Thr Gly
    1715                1720                1725

Arg Glu Ile Val Asp Leu Met Cys His Ala Thr Phe Thr Met Arg
    1730                1735                1740

Leu Leu Ser Pro Val Arg Val Pro Asn Tyr Asn Leu Ile Ile Met
    1745                1750                1755

Asp Glu Ala His Phe Thr Asp Pro Ala Ser Ile Ala Ala Arg Gly
    1760                1765                1770

Tyr Ile Ser Thr Arg Val Glu Met Gly Glu Ala Ala Gly Ile Phe
    1775                1780                1785

Met Thr Ala Thr Pro Pro Gly Ser Arg Asp Pro Phe Pro Gln Ser
    1790                1795                1800

Asn Ala Pro Ile Ile Asp Glu Glu Arg Glu Ile Pro Glu Arg Ser
    1805                1810                1815

Trp Asn Ser Gly His Glu Trp Val Thr Asp Phe Lys Gly Lys Thr
    1820                1825                1830

Val Trp Phe Val Pro Ser Ile Lys Ala Gly Asn Asp Ile Ala Ala
    1835                1840                1845

Cys Leu Arg Lys Asn Gly Lys Lys Val Ile Gln Leu Ser Arg Lys
    1850                1855                1860

Thr Phe Asp Ser Glu Tyr Val Lys Thr Arg Thr Asn Asp Trp Asp
    1865                1870                1875

Phe Val Val Thr Thr Asp Ile Ser Glu Met Gly Ala Asn Phe Lys
    1880                1885                1890

Ala Glu Arg Val Ile Asp Pro Arg Arg Cys Met Lys Pro Val Ile
    1895                1900                1905

Leu Thr Asp Gly Glu Glu Arg Val Ile Leu Ala Gly Pro Met Pro
    1910                1915                1920

Val Thr His Ser Ser Ala Ala Gln Arg Arg Gly Arg Ile Gly Arg
    1925                1930                1935

Asn Pro Lys Asn Glu Asn Asp Gln Tyr Ile Tyr Met Gly Glu Pro
    1940                1945                1950

Leu Glu Asn Asp Glu Asp Cys Ala His Trp Lys Glu Ala Lys Met
    1955                1960                1965

Leu Leu Asp Asn Ile Asn Thr Pro Glu Gly Ile Ile Pro Ser Met
    1970                1975                1980

Phe Glu Pro Glu Arg Glu Lys Val Asp Ala Ile Asp Gly Glu Tyr
    1985                1990                1995

Arg Leu Arg Gly Glu Ala Arg Lys Thr Phe Val Asp Leu Met Arg
    2000                2005                2010

Arg Gly Asp Leu Pro Val Trp Leu Ala Tyr Arg Val Ala Ala Glu
    2015                2020                2025

Gly Ile Asn Tyr Ala Asp Arg Arg Trp Cys Phe Asp Gly Val Lys
    2030                2035                2040

Asn Asn Gln Ile Leu Glu Glu Asn Val Glu Val Glu Ile Trp Thr
    2045                2050                2055

Lys Glu Gly Glu Arg Lys Lys Leu Lys Pro Arg Trp Leu Asp Ala
    2060                2065                2070

Arg Ile Tyr Ser Asp Pro Leu Ala Leu Lys Glu Phe Lys Glu Phe
    2075                2080                2085

Ala Ala Gly Arg Lys Ser Leu Thr Leu Asn Leu Ile Thr Glu Met
    2090                2095                2100

Gly Arg Leu Pro Thr Phe Met Thr Gln Lys Ala Arg Asp Ala Leu
    2105                2110                2115

Asp Asn Leu Ala Val Leu His Thr Ala Glu Ala Gly Gly Arg Ala
    2120                2125                2130

Tyr Asn His Ala Leu Ser Glu Leu Pro Glu Thr Leu Glu Thr Leu
    2135                2140                2145

Leu Leu Leu Thr Leu Leu Ala Thr Val Thr Gly Gly Ile Phe Leu
    2150                2155                2160

Phe Leu Met Ser Gly Arg Gly Ile Gly Lys Met Thr Leu Gly Met
    2165                2170                2175

Cys Cys Ile Ile Thr Ala Ser Ile Leu Leu Trp Tyr Ala Gln Ile
    2180                2185                2190

Gln Pro His Trp Ile Ala Ala Ser Ile Ile Leu Glu Phe Phe Leu
    2195                2200                2205

Ile Val Leu Leu Ile Pro Glu Pro Glu Lys Gln Arg Thr Pro Gln
    2210                2215                2220

Asp Asn Gln Leu Thr Tyr Val Val Ile Ala Ile Leu Thr Val Val
    2225                2230                2235

Ala Ala Thr Met Ala Asn Glu Met Gly Phe Leu Glu Lys Thr Lys
    2240                2245                2250

Lys Asp Leu Gly Leu Gly Ser Ile Ala Thr Gln Gln Pro Glu Ser
    2255                2260                2265

Asn Ile Leu Asp Ile Asp Leu Arg Pro Ala Ser Ala Trp Thr Leu
    2270                2275                2280

Tyr Ala Val Ala Thr Thr Phe Val Thr Pro Met Leu Arg His Ser
    2285                2290                2295

Ile Glu Asn Ser Ser Val Asn Val Ser Leu Thr Ala Ile Ala Asn
    2300                2305                2310

Gln Ala Thr Val Leu Met Gly Leu Gly Lys Gly Trp Pro Leu Ser
    2315                2320                2325

Lys Met Asp Ile Gly Val Pro Leu Leu Ala Ile Gly Cys Tyr Ser

2330                2335                2340

Gln Val Asn Pro Ile Thr Leu Thr Ala Ala Leu Phe Leu Leu Val
    2345                2350                2355

Ala His Tyr Ala Ile Ile Gly Pro Gly Leu Gln Ala Lys Ala Thr
    2360                2365                2370

Arg Glu Ala Gln Lys Arg Ala Ala Ala Gly Ile Met Lys Asn Pro
    2375                2380                2385

Thr Val Asp Gly Ile Thr Val Ile Asp Leu Asp Pro Ile Pro Tyr
    2390                2395                2400

Asp Pro Lys Phe Glu Lys Gln Leu Gly Gln Val Met Leu Leu Val
    2405                2410                2415

Leu Cys Val Thr Gln Val Leu Met Met Arg Thr Thr Trp Ala Leu
    2420                2425                2430

Cys Glu Ala Leu Thr Leu Ala Thr Gly Pro Ile Ser Thr Leu Trp
    2435                2440                2445

Glu Gly Asn Pro Gly Arg Phe Trp Asn Thr Thr Ile Ala Val Ser
    2450                2455                2460

Met Ala Asn Ile Phe Arg Gly Ser Tyr Leu Ala Gly Ala Gly Leu
    2465                2470                2475

Leu Phe Ser Ile Met Lys Asn Thr Thr Asn Thr Arg Arg Gly Thr
    2480                2485                2490

Gly Asn Ile Gly Glu Thr Leu Gly Glu Lys Trp Lys Ser Arg Leu
    2495                2500                2505

Asn Ala Leu Gly Lys Ser Glu Phe Gln Ile Tyr Lys Lys Ser Gly
    2510                2515                2520

Ile Gln Glu Val Asp Arg Thr Leu Ala Lys Glu Gly Ile Lys Arg
    2525                2530                2535

Gly Glu Thr Asp His His Ala Val Ser Arg Gly Ser Ala Lys Leu
    2540                2545                2550

Arg Trp Phe Val Glu Arg Asn Met Val Thr Pro Glu Gly Lys Val
    2555                2560                2565

Val Asp Leu Gly Cys Gly Arg Gly Gly Trp Ser Tyr Tyr Cys Gly
    2570                2575                2580

Gly Leu Lys Asn Val Arg Glu Val Lys Gly Leu Thr Lys Gly Gly
    2585                2590                2595

Pro Gly His Glu Glu Pro Ile Pro Met Ser Thr Tyr Gly Trp Asn
    2600                2605                2610

Leu Val Arg Leu Gln Ser Gly Val Asp Val Phe Phe Ile Pro Pro
    2615                2620                2625

Glu Lys Cys Asp Thr Leu Leu Cys Asp Ile Gly Glu Ser Ser Pro
    2630                2635                2640

Asn Pro Thr Val Glu Ala Gly Arg Thr Leu Arg Val Leu Asn Leu
    2645                2650                2655

Val Glu Asn Trp Leu Asn Asn Asn Thr Gln Phe Cys Ile Lys Val
    2660                2665                2670

Leu Asn Pro Tyr Met Pro Ser Val Ile Glu Lys Met Glu Ala Leu
    2675                2680                2685

Gln Arg Lys Tyr Gly Gly Ala Leu Val Arg Asn Pro Leu Ser Arg
    2690                2695                2700

Asn Ser Thr His Glu Met Tyr Trp Val Ser Asn Ala Ser Gly Asn
    2705                2710                2715

Ile Val Ser Ser Val Asn Met Ile Ser Arg Met Leu Ile Asn Arg
    2720                2725                2730

Phe Thr Met Arg Tyr Lys Lys Ala Thr Tyr Glu Pro Asp Val Asp
    2735                2740                2745

Leu Gly Ser Gly Thr Arg Asn Ile Gly Ile Glu Ser Glu Ile Pro
    2750                2755                2760

Asn Leu Asp Ile Ile Gly Lys Arg Ile Glu Lys Ile Lys Gln Glu
    2765                2770                2775

His Glu Thr Ser Trp His Tyr Asp Gln Asp His Pro Tyr Lys Thr
    2780                2785                2790

Trp Ala Tyr His Gly Ser Tyr Glu Thr Lys Gln Thr Gly Ser Ala
    2795                2800                2805

Ser Ser Met Val Asn Gly Val Val Arg Leu Leu Thr Lys Pro Trp
    2810                2815                2820

Asp Val Val Pro Met Val Thr Gln Met Ala Met Thr Asp Thr Thr
    2825                2830                2835

Pro Phe Gly Gln Gln Arg Val Phe Lys Glu Lys Val Asp Thr Arg
    2840                2845                2850

Thr Gln Glu Pro Lys Glu Gly Thr Lys Lys Leu Met Lys Ile Thr
    2855                2860                2865

Ala Glu Trp Leu Trp Lys Glu Leu Gly Lys Lys Lys Thr Pro Arg
    2870                2875                2880

Met Cys Thr Arg Glu Glu Phe Thr Arg Lys Val Arg Ser Asn Ala
    2885                2890                2895

Ala Leu Gly Ala Ile Phe Ala Asp Glu Asn Lys Trp Lys Ser Ala
    2900                2905                2910

Arg Glu Ala Val Glu Asp Ser Arg Phe Trp Glu Leu Val Asp Lys
    2915                2920                2925

Glu Arg Asn Leu His Leu Glu Gly Lys Cys Glu Thr Cys Val Tyr
    2930                2935                2940

Asn Met Met Gly Lys Arg Glu Lys Lys Leu Gly Glu Phe Gly Lys
    2945                2950                2955

Ala Lys Gly Ser Arg Ala Ile Trp Tyr Met Trp Leu Gly Ala Arg
    2960                2965                2970

Phe Leu Glu Phe Glu Ala Leu Gly Phe Leu Asn Glu Asp His Trp
    2975                2980                2985

Phe Ser Arg Glu Asn Ser Leu Ser Gly Val Glu Gly Glu Gly Leu
    2990                2995                3000

His Lys Leu Gly Tyr Ile Leu Arg Asp Val Ser Lys Lys Glu Gly
    3005                3010                3015

Gly Ala Met Tyr Ala Asp Asp Thr Ala Gly Trp Asp Thr Arg Ile
    3020                3025                3030

Thr Leu Glu Asp Leu Lys Asn Glu Glu Met Val Thr Asn His Met
    3035                3040                3045

Glu Gly Glu His Lys Lys Leu Ala Glu Ala Ile Phe Lys Leu Thr
    3050                3055                3060

Tyr Gln Asn Lys Val Val Arg Val Gln Arg Pro Thr Pro Arg Gly
    3065                3070                3075

Thr Val Met Asp Ile Ile Ser Arg Arg Asp Gln Arg Gly Ser Gly
    3080                3085                3090

Gln Val Gly Thr Tyr Gly Leu Asn Thr Phe Thr Asn Met Glu Ala
    3095                3100                3105

Gln Leu Ile Arg Gln Met Glu Gly Glu Gly Val Phe Lys Ser Ile
    3110                3115                3120

Gln His Leu Thr Ile Thr Glu Glu Ile Ala Val Gln Asn Trp Leu
    3125                3130                3135

Ala Arg Val Gly Arg Glu Arg Leu Ser Arg Met Ala Ile Ser Gly
    3140                3145                3150

Asp Asp Cys Val Val Lys Pro Leu Asp Asp Arg Phe Ala Ser Ala
    3155                3160                3165

Leu Thr Ala Leu Asn Asp Met Gly Lys Ile Arg Lys Asp Ile Gln
    3170                3175                3180

Gln Trp Glu Pro Ser Arg Gly Trp Asn Asp Trp Thr Gln Val Pro
    3185                3190                3195

Phe Cys Ser His His Phe His Glu Leu Ile Met Lys Asp Gly Arg
    3200                3205                3210

Val Leu Val Val Pro Cys Arg Asn Gln Asp Glu Leu Ile Gly Arg
    3215                3220                3225

Ala Arg Ile Ser Gln Gly Ala Gly Trp Ser Leu Arg Glu Thr Ala
    3230                3235                3240

Cys Leu Gly Lys Ser Tyr Ala Gln Met Trp Ser Leu Met Tyr Phe
    3245                3250                3255

His Arg Arg Asp Leu Arg Leu Ala Ala Asn Ala Ile Cys Ser Ala
    3260                3265                3270

Val Pro Ser His Trp Val Pro Thr Ser Arg Thr Thr Trp Ser Ile

3275                    3280                    3285

His Ala  Lys His Glu Trp Met  Thr Thr Glu Asp Met  Leu Thr Val
    3290                    3295                    3300

Trp Asn  Arg Val Trp Ile Gln  Glu Asn Pro Trp Met  Glu Asp Lys
    3305                    3310                    3315

Thr Pro  Val Glu Ser Trp Glu  Glu Ile Pro Tyr Leu  Gly Lys Arg
    3320                    3325                    3330

Glu Asp  Gln Trp Cys Gly Ser  Leu Ile Gly Leu Thr  Ser Arg Ala
    3335                    3340                    3345

Thr Trp  Ala Lys Asn Ile Gln  Ala Ala Ile Asn Gln  Val Arg Ser
    3350                    3355                    3360

Leu Ile  Gly Asn Glu Glu Tyr  Thr Asp Tyr Met Pro  Ser Met Lys
    3365                    3370                    3375

Arg Phe  Arg Arg Glu Glu Glu  Glu Ala Gly Val Leu  Trp
    3380                    3385                    3390

<210>  26
<211>  3390
<212>  PRT
<213>  Dengue virus type 3

<400>  26

Met Asn Asn Gln Arg Lys Lys Thr Gly Lys Pro Ser Ile Asn Met Leu
1               5                   10                  15

Lys Arg Val Arg Asn Arg Val Ser Thr Gly Ser Gln Leu Ala Lys Arg
            20                  25                  30

Phe Ser Arg Gly Leu Leu Asn Gly Gln Gly Pro Met Lys Leu Val Met
        35                  40                  45

Ala Phe Ile Ala Phe Leu Arg Phe Leu Ala Ile Pro Pro Thr Ala Gly
    50                  55                  60

Val Leu Ala Arg Trp Gly Thr Phe Lys Lys Ser Gly Ala Ile Lys Val
65                  70                  75                  80

Leu Lys Gly Phe Lys Lys Glu Ile Ser Asn Met Leu Ser Ile Ile Asn
                85                  90                  95

Lys Arg Lys Lys Thr Ser Leu Cys Leu Met Met Met Leu Pro Ala Thr

            100                 105                 110

Leu Ala Phe His Leu Thr Ser Arg Asp Gly Glu Pro Arg Met Ile Val
        115                 120                 125

Gly Lys Asn Glu Arg Gly Lys Ser Leu Leu Phe Lys Thr Ala Ser Gly
    130                 135                 140

Ile Asn Met Cys Thr Leu Ile Ala Met Asp Leu Gly Glu Met Cys Asp
145                 150                 155                 160

Asp Thr Val Thr Tyr Lys Cys Pro His Ile Thr Glu Val Glu Pro Glu
                165                 170                 175

Asp Ile Asp Cys Trp Cys Asn Leu Thr Ser Thr Trp Val Thr Tyr Gly
            180                 185                 190

Thr Cys Asn Gln Ala Gly Glu His Arg Arg Asp Lys Arg Ser Val Ala
        195                 200                 205

Leu Ala Pro His Val Gly Met Gly Leu Asp Thr Arg Thr Gln Thr Trp
    210                 215                 220

Met Ser Ala Glu Gly Ala Trp Arg Gln Val Glu Lys Val Glu Thr Trp
225                 230                 235                 240

Ala Leu Arg His Pro Gly Phe Thr Ile Leu Ala Leu Phe Leu Ala His
                245                 250                 255

Tyr Ile Gly Thr Ser Leu Thr Gln Lys Val Val Ile Phe Ile Leu Leu
            260                 265                 270

Met Leu Val Thr Pro Ser Met Thr Met Arg Cys Val Gly Val Gly Asn
        275                 280                 285

Arg Asp Phe Val Glu Gly Leu Ser Gly Ala Thr Trp Val Asp Val Val
    290                 295                 300

Leu Glu His Gly Gly Cys Val Thr Thr Met Ala Lys Asn Lys Pro Thr
305                 310                 315                 320

Leu Asp Ile Glu Leu Gln Lys Thr Glu Ala Thr Gln Leu Ala Thr Leu
                325                 330                 335

Arg Lys Leu Cys Ile Glu Gly Lys Ile Thr Asn Ile Thr Thr Asp Ser
            340                 345                 350

Arg Cys Pro Thr Gln Gly Glu Ala Ile Leu Pro Glu Glu Gln Asp Gln
        355                 360                 365

Asn Tyr Val Cys Lys His Thr Tyr Val Asp Arg Gly Trp Gly Asn Gly
    370                 375                 380

Cys Gly Leu Phe Gly Lys Gly Ser Leu Val Thr Cys Ala Lys Phe Gln
385                 390                 395                 400

Cys Leu Glu Ser Ile Glu Gly Lys Val Val Gln His Glu Asn Leu Lys
                405                 410                 415

Tyr Thr Val Ile Ile Thr Val His Thr Gly Asp Gln His Gln Val Gly
            420                 425                 430

Asn Glu Thr Gln Gly Val Thr Ala Glu Ile Thr Ser Gln Ala Ser Thr
        435                 440                 445

Ala Glu Ala Ile Leu Pro Glu Tyr Gly Thr Leu Gly Leu Glu Cys Ser
    450                 455                 460

Pro Arg Thr Gly Leu Asp Phe Asn Glu Met Ile Leu Leu Thr Met Lys
465                 470                 475                 480

Asn Lys Ala Trp Met Val His Arg Gln Trp Phe Phe Asp Leu Pro Leu
                485                 490                 495

Pro Trp Thr Ser Gly Ala Thr Thr Lys Thr Pro Thr Trp Asn Arg Lys
            500                 505                 510

Glu Leu Leu Val Thr Phe Lys Asn Ala His Ala Lys Lys Gln Glu Val
        515                 520                 525

Val Val Leu Gly Ser Gln Glu Gly Ala Met His Thr Ala Leu Thr Gly
    530                 535                 540

Ala Thr Glu Ile Gln Thr Ser Gly Gly Thr Ser Ile Phe Ala Gly His
545                 550                 555                 560

Leu Lys Cys Arg Leu Lys Met Asp Lys Leu Lys Leu Lys Gly Met Ser
                565                 570                 575

Tyr Ala Met Cys Leu Asn Thr Phe Val Leu Lys Lys Glu Val Ser Glu
            580                 585                 590

Thr Gln His Gly Thr Ile Leu Ile Lys Val Glu Tyr Lys Gly Glu Asp
        595                 600                 605

Ala Pro Cys Lys Ile Pro Phe Ser Thr Glu Asp Gly Gln Gly Lys Ala
    610                 615                 620

His Asn Gly Arg Leu Ile Thr Ala Asn Pro Val Val Thr Lys Lys Glu
625                 630                 635                 640

Glu Pro Val Asn Ile Glu Ala Glu Pro Pro Phe Gly Glu Ser Asn Ile
                645                 650                 655

Val Ile Gly Ile Gly Asp Lys Ala Leu Lys Ile Asn Trp Tyr Arg Lys
            660                 665                 670

Gly Ser Ser Ile Gly Lys Met Phe Glu Ala Thr Ala Arg Gly Ala Arg
        675                 680                 685

Arg Met Ala Ile Leu Gly Asp Thr Ala Trp Asp Phe Gly Ser Val Gly
    690                 695                 700

Gly Val Leu Asn Ser Leu Gly Lys Met Val His Gln Ile Phe Gly Ser
705                 710                 715                 720

Ala Tyr Thr Ala Leu Phe Ser Gly Val Ser Trp Ile Met Lys Ile Gly
                725                 730                 735

Ile Gly Val Leu Leu Thr Trp Ile Gly Leu Asn Ser Lys Asn Thr Ser
            740                 745                 750

Met Ser Phe Ser Cys Ile Ala Ile Gly Ile Ile Thr Leu Tyr Leu Gly
        755                 760                 765

Val Val Val Gln Ala Asp Met Gly Cys Val Ile Asn Trp Lys Gly Lys
    770                 775                 780

Glu Leu Lys Cys Gly Ser Gly Ile Phe Val Thr Asn Glu Val His Thr
785                 790                 795                 800

Trp Thr Glu Gln Tyr Lys Phe Gln Ala Asp Ser Pro Lys Arg Val Ala
                805                 810                 815

Thr Ala Ile Ala Gly Ala Trp Glu Asn Gly Val Cys Gly Ile Arg Ser
            820                 825                 830

Thr Thr Arg Met Glu Asn Leu Leu Trp Lys Gln Ile Ala Asn Glu Leu
        835                 840                 845

Asn Tyr Ile Leu Trp Glu Asn Asp Ile Lys Leu Thr Val Val Val Gly
    850                 855                 860

Asp Ile Thr Gly Val Leu Glu Gln Gly Lys Arg Thr Leu Thr Pro Gln
865                 870                 875                 880

Pro Met Glu Leu Lys Tyr Ser Trp Lys Thr Trp Gly Leu Ala Lys Ile
                885                 890                 895

Val Thr Ala Glu Thr Gln Asn Ser Ser Phe Ile Ile Asp Gly Pro Ser
            900                 905                 910

Thr Pro Glu Cys Pro Ser Ala Ser Arg Ala Trp Asn Val Trp Glu Val
        915                 920                 925

Glu Asp Tyr Gly Phe Gly Val Phe Thr Thr Asn Ile Trp Leu Lys Leu
    930                 935                 940

Arg Glu Val Tyr Thr Gln Leu Cys Asp His Arg Leu Met Ser Ala Ala
945                 950                 955                 960

Val Lys Asp Glu Arg Ala Val His Ala Asp Met Gly Tyr Trp Ile Glu
                965                 970                 975

Ser Gln Lys Asn Gly Ser Trp Lys Leu Glu Lys Ala Ser Leu Ile Glu
            980                 985                 990

Val Lys Thr Cys Thr Trp Pro Lys  Ser His Thr Leu Trp  Ser Asn Gly
        995                 1000                1005

Val Leu  Glu Ser Asp Met Ile  Ile Pro Lys Ser Leu  Ala Gly Pro
    1010                1015                1020

Ile Ser  Gln His Asn His Arg  Pro Gly Tyr His Thr  Gln Thr Ala
    1025                1030                1035

Gly Pro  Trp His Leu Gly Lys  Leu Glu Leu Asp Phe  Asn Tyr Cys
    1040                1045                1050

Glu Gly  Thr Thr Val Val Ile  Ser Glu Asn Cys Gly  Thr Arg Gly
    1055                1060                1065

Pro Ser  Leu Arg Thr Thr Thr  Val Ser Gly Lys Leu  Ile His Glu
    1070                1075                1080

Trp Cys  Cys Arg Ser Cys Thr  Leu Pro Pro Leu Arg  Tyr Met Gly
    1085                1090                1095

Glu Asp  Gly Cys Trp Tyr Gly  Met Glu Ile Arg Pro  Ile Asn Glu

1100                1105                1110

Lys Glu Glu Asn Met Val Lys Ser Leu Ala Ser Ala Gly Ser Gly
    1115                1120                1125

Lys Val Asp Asn Phe Thr Met Gly Val Leu Cys Leu Ala Ile Leu
    1130                1135                1140

Phe Glu Glu Val Met Arg Gly Lys Phe Gly Lys Lys His Met Ile
    1145                1150                1155

Ala Gly Val Leu Phe Thr Phe Val Leu Leu Leu Ser Gly Gln Ile
    1160                1165                1170

Thr Trp Arg Gly Met Ala His Thr Leu Ile Met Ile Gly Ser Asn
    1175                1180                1185

Ala Ser Asp Arg Met Gly Met Gly Val Thr Tyr Leu Ala Leu Ile
    1190                1195                1200

Ala Thr Phe Lys Ile Gln Pro Phe Leu Ala Leu Gly Phe Phe Leu
    1205                1210                1215

Arg Lys Leu Thr Ser Arg Glu Asn Leu Leu Leu Gly Val Gly Leu
    1220                1225                1230

Ala Met Ala Ala Thr Leu Arg Leu Pro Glu Asp Ile Glu Gln Met
    1235                1240                1245

Ala Asn Gly Ile Ala Leu Gly Leu Met Ala Leu Lys Leu Ile Thr
    1250                1255                1260

Gln Phe Glu Thr Tyr Gln Leu Trp Thr Ala Leu Val Ser Leu Thr
    1265                1270                1275

Cys Ser Asn Thr Ile Phe Thr Leu Thr Val Ala Trp Arg Thr Ala
    1280                1285                1290

Thr Leu Ile Leu Ala Gly Ile Ser Leu Leu Pro Val Cys Gln Ser
    1295                1300                1305

Ser Ser Met Arg Lys Thr Asp Trp Leu Pro Met Thr Val Ala Ala
    1310                1315                1320

Met Gly Val Pro Pro Leu Pro Leu Phe Ile Phe Ser Leu Lys Asp
    1325                1330                1335

Thr Leu Lys Arg Arg Ser Trp Pro Leu Asn Glu Gly Val Met Ala
    1340                1345                1350

Val Gly Leu Val Ser Ile Leu Ala Ser Ser Leu Leu Arg Asn Asp
    1355                1360                1365

Val Pro Met Ala Gly Pro Leu Val Ala Gly Gly Leu Leu Ile Ala
    1370                1375                1380

Cys Tyr Val Ile Thr Gly Thr Ser Ala Asp Leu Thr Val Glu Lys
    1385                1390                1395

Ala Ala Asp Val Thr Trp Glu Glu Glu Ala Glu Gln Thr Gly Val
    1400                1405                1410

Ser His Asn Leu Met Ile Thr Val Asp Asp Asp Gly Thr Met Arg
    1415                1420                1425

Ile Lys Asp Asp Glu Thr Glu Asn Ile Leu Thr Val Leu Leu Lys
    1430                1435                1440

Thr Ala Leu Leu Ile Val Ser Gly Ile Phe Pro Tyr Ser Ile Pro
    1445                1450                1455

Ala Thr Met Leu Val Trp His Thr Trp Gln Lys Gln Thr Gln Arg
    1460                1465                1470

Ser Gly Val Leu Trp Asp Val Pro Ser Pro Pro Glu Thr Gln Lys
    1475                1480                1485

Ala Glu Leu Glu Glu Gly Val Tyr Arg Ile Lys Gln Gln Gly Ile
    1490                1495                1500

Phe Gly Lys Thr Gln Val Gly Val Gly Val Gln Lys Glu Gly Val
    1505                1510                1515

Phe His Thr Met Trp His Val Thr Arg Gly Ala Val Leu Thr His
    1520                1525                1530

Asn Gly Lys Arg Leu Glu Pro Asn Trp Ala Ser Val Lys Lys Asp
    1535                1540                1545

Leu Ile Ser Tyr Gly Gly Gly Trp Arg Leu Ser Ala Gln Trp Gln
    1550                1555                1560

Lys Gly Glu Glu Val Gln Val Ile Ala Val Glu Pro Gly Lys Asn
    1565                1570                1575

Pro Lys Asn Phe Gln Thr Met Pro Gly Ile Phe Gln Thr Thr Thr
    1580                1585                1590

Gly Glu Ile Gly Ala Ile Ala Leu Asp Phe Lys Pro Gly Thr Ser
    1595                1600                1605

Gly Ser Pro Ile Ile Asn Arg Glu Gly Lys Val Val Gly Leu Tyr
    1610                1615                1620

Gly Asn Gly Val Val Thr Lys Asn Gly Gly Tyr Val Ser Gly Ile
    1625                1630                1635

Ala Gln Thr Asn Ala Glu Pro Asp Gly Pro Thr Pro Glu Leu Glu
    1640                1645                1650

Glu Glu Met Phe Lys Lys Arg Asn Leu Thr Ile Met Asp Leu His
    1655                1660                1665

Pro Gly Ser Gly Lys Thr Arg Lys Tyr Leu Pro Ala Ile Val Arg
    1670                1675                1680

Glu Ala Ile Lys Arg Arg Leu Arg Thr Leu Ile Leu Ala Pro Thr
    1685                1690                1695

Arg Val Val Ala Ala Glu Met Glu Glu Ala Met Lys Gly Leu Pro
    1700                1705                1710

Ile Arg Tyr Gln Thr Thr Ala Thr Lys Ser Glu His Thr Gly Arg
    1715                1720                1725

Glu Ile Val Asp Leu Met Cys His Ala Thr Phe Thr Met Arg Leu
    1730                1735                1740

Leu Ser Pro Val Arg Val Pro Asn Tyr Asn Leu Ile Ile Met Asp
    1745                1750                1755

Glu Ala His Phe Thr Asp Pro Ala Ser Ile Ala Ala Arg Gly Tyr
    1760                1765                1770

Ile Ser Thr Arg Val Gly Met Gly Glu Ala Ala Ala Ile Phe Met
    1775                1780                1785

Thr Ala Thr Pro Pro Gly Thr Ala Asp Ala Phe Pro Gln Ser Asn
    1790                1795                1800

Ala Pro Ile Gln Asp Glu Glu Arg Asp Ile Pro Glu Arg Ser Trp
    1805                1810                1815

Asn Ser Gly Asn Glu Trp Ile Thr Asp Phe Val Gly Lys Thr Val
    1820                1825                1830

Trp Phe Val Pro Ser Ile Lys Ala Gly Asn Val Ile Ala Asn Cys
    1835                1840                1845

Leu Arg Lys Asn Gly Lys Lys Val Ile Gln Leu Ser Arg Lys Thr
    1850                1855                1860

Phe Asp Thr Glu Tyr Gln Lys Thr Lys Leu Asn Asp Trp Asp Phe
    1865                1870                1875

Val Val Thr Thr Asp Ile Ser Glu Met Gly Ala Asn Phe Ile Ala
    1880                1885                1890

Asp Arg Val Ile Asp Pro Arg Arg Cys Leu Lys Pro Val Ile Leu
    1895                1900                1905

Thr Asp Gly Pro Glu Arg Val Ile Leu Ala Gly Pro Met Pro Val
    1910                1915                1920

Thr Val Ala Ser Ala Ala Gln Arg Arg Gly Arg Val Gly Arg Asn
    1925                1930                1935

Pro Gln Lys Glu Asn Asp Gln Tyr Ile Phe Met Gly Gln Pro Leu
    1940                1945                1950

Asn Lys Asp Glu Asp His Ala His Trp Thr Glu Ala Lys Met Leu
    1955                1960                1965

Leu Asp Asn Ile Asn Thr Pro Glu Gly Ile Ile Pro Ala Leu Phe
    1970                1975                1980

Glu Pro Glu Arg Glu Lys Ser Ala Ala Ile Asp Gly Glu Tyr Arg
    1985                1990                1995

Leu Lys Gly Glu Ser Arg Lys Thr Phe Val Glu Leu Met Arg Arg
    2000                2005                2010

Gly Asp Leu Pro Val Trp Leu Ala His Lys Val Ala Ser Glu Gly
    2015                2020                2025

Ile Lys Tyr Thr Asp Arg Lys Trp Cys Phe Asp Gly Glu Arg Asn
    2030                2035                2040

Asn Gln Ile Leu Glu Glu Asn Met Asp Val Glu Ile Trp Thr Lys

2045                2050                2055

Glu Gly Glu Lys Lys Lys Leu Arg Pro Arg Trp Leu Asp Ala Arg
    2060                2065                2070

Thr Tyr Ser Asp Pro Leu Ala Leu Lys Glu Phe Lys Asp Phe Ala
    2075                2080                2085

Ala Gly Arg Lys Ser Ile Ala Leu Asp Leu Val Thr Glu Ile Gly
    2090                2095                2100

Arg Val Pro Ser His Leu Ala His Arg Thr Arg Asn Ala Leu Asp
    2105                2110                2115

Asn Leu Val Met Leu His Thr Ser Glu His Gly Gly Arg Ala Tyr
    2120                2125                2130

Arg His Ala Val Glu Glu Leu Pro Glu Thr Met Glu Thr Leu Leu
    2135                2140                2145

Leu Leu Gly Leu Met Ile Leu Leu Thr Gly Gly Ala Met Leu Phe
    2150                2155                2160

Leu Ile Ser Gly Lys Gly Ile Gly Lys Thr Ser Ile Gly Leu Ile
    2165                2170                2175

Cys Val Ile Ala Ser Ser Gly Met Leu Trp Met Ala Asp Val Pro
    2180                2185                2190

Leu Gln Trp Ile Ala Ser Ala Ile Val Leu Glu Phe Phe Met Met
    2195                2200                2205

Val Leu Leu Ile Pro Glu Pro Glu Lys Gln Arg Thr Pro Gln Asp
    2210                2215                2220

Asn Gln Leu Ala Tyr Val Val Ile Gly Ile Leu Thr Leu Ala Ala
    2225                2230                2235

Ile Val Ala Ala Asn Glu Met Gly Leu Leu Glu Thr Thr Lys Arg
    2240                2245                2250

Asp Leu Gly Met Ser Lys Glu Pro Gly Val Val Ser Pro Thr Ser
    2255                2260                2265

Tyr Leu Asp Val Asp Leu His Pro Ala Ser Ala Trp Thr Leu Tyr
    2270                2275                2280

Ala Val Ala Thr Thr Val Ile Thr Pro Met Leu Arg His Thr Ile
    2285                2290                2295

Glu Asn Ser Thr Ala Asn Val Ser Leu Ala Ala Ile Ala Asn Gln
    2300                2305                2310

Ala Val Val Leu Met Gly Leu Asp Lys Gly Trp Pro Ile Ser Lys
    2315                2320                2325

Met Asp Leu Gly Val Pro Leu Leu Ala Leu Gly Cys Tyr Ser Gln
    2330                2335                2340

Val Asn Pro Leu Thr Leu Ile Ala Ala Val Leu Leu Leu Val Thr
    2345                2350                2355

His Tyr Ala Ile Ile Gly Pro Gly Leu Gln Ala Lys Ala Thr Arg
    2360                2365                2370

Glu Ala Gln Lys Arg Thr Ala Ala Gly Ile Met Lys Asn Pro Thr
    2375                2380                2385

Val Asp Gly Ile Met Thr Ile Asp Leu Asp Pro Val Ile Tyr Asp
    2390                2395                2400

Ser Lys Phe Glu Lys Gln Leu Gly Gln Val Met Leu Leu Val Leu
    2405                2410                2415

Cys Ala Val Gln Leu Leu Leu Met Arg Thr Ser Trp Ala Leu Cys
    2420                2425                2430

Glu Val Leu Thr Leu Ala Thr Gly Pro Ile Thr Thr Leu Trp Glu
    2435                2440                2445

Gly Ser Pro Gly Lys Phe Trp Asn Thr Thr Ile Ala Val Ser Met
    2450                2455                2460

Ala Asn Ile Phe Arg Gly Ser Tyr Leu Ala Gly Ala Gly Leu Ala
    2465                2470                2475

Leu Ser Ile Met Lys Ser Val Gly Thr Gly Lys Arg Gly Thr Gly
    2480                2485                2490

Ser Gln Gly Glu Thr Leu Gly Glu Lys Trp Lys Lys Lys Leu Asn
    2495                2500                2505

Gln Leu Ser Arg Lys Glu Phe Asp Leu Tyr Lys Lys Ser Gly Ile
    2510                2515                2520

Thr Glu Val Asp Arg Thr Glu Ala Lys Glu Gly Leu Lys Arg Gly
    2525                2530                2535

Glu Ile Thr His His Ala Val Ser Arg Gly Ser Ala Lys Leu Gln
    2540                2545                2550

Trp Phe Val Glu Arg Asn Met Val Ile Pro Glu Gly Arg Val Ile
    2555                2560                2565

Asp Leu Gly Cys Gly Arg Gly Gly Trp Ser Tyr Tyr Cys Ala Gly
    2570                2575                2580

Leu Lys Lys Val Thr Glu Val Arg Gly Tyr Thr Lys Gly Gly Pro
    2585                2590                2595

Gly His Glu Glu Pro Val Pro Met Ser Thr Tyr Gly Trp Asn Ile
    2600                2605                2610

Val Lys Leu Met Ser Gly Lys Asp Val Phe Tyr Leu Pro Pro Glu
    2615                2620                2625

Lys Cys Asp Thr Leu Leu Cys Asp Ile Gly Glu Ser Ser Pro Ser
    2630                2635                2640

Pro Thr Val Glu Glu Ser Arg Thr Ile Arg Val Leu Lys Met Val
    2645                2650                2655

Glu Pro Trp Leu Lys Asn Asn Gln Phe Cys Ile Lys Val Leu Asn
    2660                2665                2670

Pro Tyr Met Pro Thr Val Ile Glu His Leu Glu Arg Leu Gln Arg
    2675                2680                2685

Lys His Gly Gly Met Leu Val Arg Asn Pro Leu Ser Arg Asn Ser
    2690                2695                2700

Thr His Glu Met Tyr Trp Ile Ser Asn Gly Thr Gly Asn Ile Val
    2705                2710                2715

Ser Ser Val Asn Met Val Ser Arg Leu Leu Leu Asn Arg Phe Thr
    2720                2725                2730

Met Thr His Arg Arg Pro Thr Ile Glu Lys Asp Val Asp Leu Gly
    2735                2740                2745

Ala Gly Thr Arg His Val Asn Ala Glu Pro Glu Thr Pro Asn Met
    2750                2755                2760

Asp Val Ile Gly Glu Arg Ile Lys Arg Ile Lys Glu Glu His Ser
    2765                2770                2775

Ser Thr Trp His Tyr Asp Asp Glu Asn Pro Tyr Lys Thr Trp Ala
    2780                2785                2790

Tyr His Gly Ser Tyr Glu Val Lys Ala Thr Gly Ser Ala Ser Ser
    2795                2800                2805

Met Ile Asn Gly Val Val Lys Leu Leu Thr Lys Pro Trp Asp Val
    2810                2815                2820

Val Pro Met Val Thr Gln Met Ala Met Thr Asp Thr Thr Pro Phe
    2825                2830                2835

Gly Gln Gln Arg Val Phe Lys Glu Lys Val Asp Thr Arg Thr Pro
    2840                2845                2850

Arg Pro Met Pro Gly Thr Arg Lys Val Met Glu Ile Thr Ala Glu
    2855                2860                2865

Trp Leu Trp Arg Thr Leu Gly Arg Asn Lys Arg Pro Arg Leu Cys
    2870                2875                2880

Thr Arg Glu Glu Phe Thr Lys Lys Val Arg Thr Asn Ala Ala Met
    2885                2890                2895

Gly Ala Val Phe Thr Glu Glu Asn Gln Trp Asp Ser Ala Arg Ala
    2900                2905                2910

Ala Val Glu Asp Glu Glu Phe Trp Lys Leu Val Asp Arg Glu Arg
    2915                2920                2925

Glu Leu His Lys Leu Gly Lys Cys Gly Ser Cys Val Tyr Asn Met
    2930                2935                2940

Met Gly Lys Arg Glu Lys Lys Leu Gly Glu Phe Gly Lys Ala Lys
    2945                2950                2955

Gly Ser Arg Ala Ile Trp Tyr Met Trp Leu Gly Ala Arg Tyr Leu
    2960                2965                2970

Glu Phe Glu Ala Leu Gly Phe Leu Asn Glu Asp His Trp Phe Ser
    2975                2980                2985

Arg Glu Asn Ser Tyr Ser Gly Val Glu Gly Glu Gly Leu His Lys

2990                2995                3000

Leu Gly Tyr Ile Leu Arg Asp Ile Ser Lys Ile Pro Gly Gly Ala
    3005                3010                3015

Met Tyr Ala Asp Asp Thr Ala Gly Trp Asp Thr Arg Ile Thr Glu
    3020                3025                3030

Asp Asp Leu His Asn Glu Glu Lys Ile Thr Gln Gln Met Asp Pro
    3035                3040                3045

Glu His Arg Gln Leu Ala Asn Ala Ile Phe Lys Leu Thr Tyr Gln
    3050                3055                3060

Asn Lys Val Val Lys Val Gln Arg Pro Thr Pro Lys Gly Thr Val
    3065                3070                3075

Met Asp Ile Ile Ser Arg Lys Asp Gln Arg Gly Ser Gly Gln Val
    3080                3085                3090

Gly Thr Tyr Gly Leu Asn Thr Phe Thr Asn Met Glu Ala Gln Leu
    3095                3100                3105

Ile Arg Gln Met Glu Gly Glu Gly Val Leu Ser Lys Ala Asp Leu
    3110                3115                3120

Glu Asn Pro His Pro Leu Glu Lys Lys Ile Thr Gln Trp Leu Glu
    3125                3130                3135

Thr Lys Gly Val Glu Arg Leu Lys Arg Met Ala Ile Ser Gly Asp
    3140                3145                3150

Asp Cys Val Val Lys Pro Ile Asp Asp Arg Phe Ala Asn Ala Leu
    3155                3160                3165

Leu Ala Leu Asn Asp Met Gly Lys Val Arg Lys Asp Ile Pro Gln
    3170                3175                3180

Trp Gln Pro Ser Lys Gly Trp His Asp Trp Gln Gln Val Pro Phe
    3185                3190                3195

Cys Ser His His Phe His Glu Leu Ile Met Lys Asp Gly Arg Lys
    3200                3205                3210

Leu Val Val Pro Cys Arg Pro Gln Asp Glu Leu Ile Gly Arg Ala
    3215                3220                3225

Arg Ile Ser Gln Gly Ala Gly Trp Ser Leu Arg Glu Thr Ala Cys
    3230                3235                3240

Leu Gly Lys Ala Tyr Ala Gln Met Trp Thr Leu Met Tyr Phe His
    3245                3250                3255

Arg Arg Asp Leu Arg Leu Ala Ser Asn Ala Ile Cys Ser Ala Val
    3260                3265                3270

Pro Val His Trp Val Pro Thr Ser Arg Thr Thr Trp Ser Ile His
    3275                3280                3285

Ala His His Gln Trp Met Thr Thr Glu Asp Met Leu Thr Val Trp
    3290                3295                3300

Asn Arg Val Trp Ile Glu Asp Asn Pro Trp Met Glu Asp Lys Thr
    3305                3310                3315

Pro Val Thr Thr Trp Glu Asp Val Pro Tyr Leu Gly Lys Arg Glu
    3320                3325                3330

Asp Gln Trp Cys Gly Ser Leu Ile Gly Leu Thr Ser Arg Ala Thr
    3335                3340                3345

Trp Ala Gln Asn Ile Leu Thr Ala Ile Gln Gln Val Arg Ser Leu
    3350                3355                3360

Ile Gly Asn Glu Glu Phe Leu Asp Tyr Met Pro Ser Met Lys Arg
    3365                3370                3375

Phe Arg Lys Glu Glu Glu Ser Glu Gly Ala Ile Trp
    3380                3385                3390

<210> 27
<211> 3387
<212> PRT
<213> Dengue virus type 4

<400> 27

Met Asn Gln Arg Lys Lys Val Val Arg Pro Pro Phe Asn Met Leu Lys
1               5                   10                  15

Arg Glu Arg Asn Arg Val Ser Thr Pro Gln Gly Leu Val Lys Arg Phe
            20                  25                  30

Ser Thr Gly Leu Phe Ser Gly Lys Gly Pro Leu Arg Met Val Leu Ala
        35                  40                  45

Phe Ile Thr Phe Leu Arg Val Leu Ser Ile Pro Pro Thr Ala Gly Ile
    50                  55                  60

Leu Lys Arg Trp Gly Gln Leu Lys Lys Asn Lys Ala Ile Lys Ile Leu
65                  70                  75                  80

Thr Gly Phe Arg Lys Glu Ile Gly Arg Met Leu Asn Ile Leu Asn Gly
                85                  90                  95

Arg Lys Arg Ser Thr Met Thr Leu Leu Cys Leu Ile Pro Thr Val Met
            100                 105                 110

Ala Phe His Leu Ser Thr Arg Asp Gly Glu Pro Leu Met Ile Val Ala
        115                 120                 125

Lys His Glu Arg Gly Arg Pro Leu Leu Phe Lys Thr Thr Glu Gly Ile
    130                 135                 140

Asn Lys Cys Thr Leu Ile Ala Met Asp Leu Gly Glu Met Cys Glu Asp
145                 150                 155                 160

Thr Val Thr Tyr Glu Cys Pro Leu Leu Val Asn Thr Glu Pro Glu Asp
                165                 170                 175

Ile Asp Cys Trp Cys Asn Leu Thr Ser Ala Trp Val Met Tyr Gly Thr
            180                 185                 190

Cys Thr Gln Ser Gly Glu Arg Arg Arg Glu Lys Arg Ser Val Ala Leu
        195                 200                 205

Thr Pro His Ser Gly Met Gly Leu Glu Thr Arg Ala Glu Thr Trp Met
    210                 215                 220

Ser Ser Glu Gly Ala Trp Lys His Ala Gln Arg Val Glu Ser Trp Ile
225                 230                 235                 240

Leu Arg Asn Pro Gly Phe Ala Leu Leu Ala Gly Phe Met Ala Tyr Met
                245                 250                 255

Ile Gly Gln Thr Gly Ile Gln Arg Thr Val Phe Phe Val Leu Met Met
            260                 265                 270

Leu Val Ala Pro Ser Tyr Gly Met Arg Cys Val Gly Val Gly Asn Arg
        275                 280                 285

Asp Phe Val Glu Gly Val Ser Gly Gly Ala Trp Val Asp Leu Val Leu
    290                 295                 300

Glu His Gly Gly Cys Val Thr Thr Met Ala Gln Gly Lys Pro Thr Leu
305                 310                 315                 320

Asp Phe Glu Leu Ile Lys Thr Thr Ala Lys Glu Val Ala Leu Leu Arg
                325                 330                 335

Thr Tyr Cys Ile Glu Ala Ser Ile Ser Asn Ile Thr Thr Ala Thr Arg
            340                 345                 350

Cys Pro Thr Gln Gly Glu Pro Tyr Leu Lys Glu Glu Gln Asp Gln Gln
        355                 360                 365

Tyr Ile Cys Arg Arg Asp Val Val Asp Arg Gly Trp Gly Asn Gly Cys
    370                 375                 380

Gly Leu Phe Gly Lys Gly Gly Val Val Thr Cys Ala Lys Phe Ser Cys
385                 390                 395                 400

Ser Gly Lys Ile Thr Gly Asn Leu Val Gln Ile Glu Asn Leu Glu Tyr
                405                 410                 415

Thr Val Val Val Thr Val His Asn Gly Asp Thr His Ala Val Gly Asn
            420                 425                 430

Asp Ile Pro Asn His Gly Val Thr Ala Thr Ile Thr Pro Arg Ser Pro
        435                 440                 445

Ser Val Glu Val Lys Leu Pro Asp Tyr Gly Glu Leu Thr Leu Asp Cys
    450                 455                 460

Glu Pro Arg Ser Gly Ile Asp Phe Asn Glu Met Ile Leu Met Lys Met
465                 470                 475                 480

Lys Lys Lys Thr Trp Leu Val His Lys Gln Trp Phe Leu Asp Leu Pro
                485                 490                 495

Leu Pro Trp Ala Ala Gly Ala Asp Thr Ser Glu Val His Trp Asn Tyr
            500                 505                 510

Lys Glu Arg Met Val Thr Phe Lys Val Pro His Ala Lys Arg Gln Asp
        515                 520                 525

Val Ile Val Leu Gly Ser Gln Glu Gly Ala Met His Ser Ala Leu Thr
    530                 535                 540

Gly Ala Thr Glu Val Asp Ser Gly Asp Gly Asn His Met Phe Ala Gly
545                 550                 555                 560

His Leu Lys Cys Lys Val Arg Met Glu Lys Leu Arg Ile Lys Gly Met
                565                 570                 575

Ser Tyr Thr Met Cys Ser Gly Lys Phe Ser Ile Asp Lys Glu Met Ala
            580                 585                 590

Glu Thr Gln His Gly Thr Thr Val Val Lys Val Lys Tyr Glu Gly Ala
        595                 600                 605

Gly Ala Pro Cys Lys Val Pro Ile Glu Ile Arg Asp Val Asn Lys Glu
    610                 615                 620

Lys Val Val Gly Arg Ile Ile Ser Ser Thr Pro Phe Ala Glu Tyr Thr
625                 630                 635                 640

Asn Ser Val Thr Asn Ile Glu Leu Glu Pro Pro Phe Gly Asp Ser Tyr
                645                 650                 655

Ile Val Ile Gly Val Gly Asp Ser Ala Leu Thr Leu His Trp Phe Arg
            660                 665                 670

Lys Gly Ser Ser Ile Gly Lys Met Leu Glu Ser Thr Tyr Arg Gly Ala
        675                 680                 685

Lys Arg Met Ala Ile Leu Gly Glu Thr Ala Trp Asp Phe Gly Ser Val
    690                 695                 700

Gly Gly Leu Leu Thr Ser Leu Gly Lys Ala Val His Gln Val Phe Gly
705                 710                 715                 720

Ser Val Tyr Thr Thr Met Phe Gly Gly Val Ser Trp Met Val Arg Ile
                725                 730                 735

Leu Ile Gly Phe Leu Val Leu Trp Ile Gly Thr Asn Ser Arg Asn Thr
            740                 745                 750

Ser Met Ala Met Thr Cys Ile Ala Val Gly Gly Ile Thr Leu Phe Leu
        755                 760                 765

Gly Phe Thr Val His Ala Asp Thr Gly Cys Ala Val Ser Trp Ser Gly
    770                 775                 780

Lys Glu Leu Lys Cys Gly Ser Gly Ile Phe Val Ile Asp Asn Val His
785                 790                 795                 800

Thr Trp Thr Glu Gln Tyr Lys Phe Gln Pro Glu Ser Pro Ala Arg Leu

                805                 810                 815

Ala Ser Ala Ile Leu Asn Ala His Lys Asp Gly Val Cys Gly Ile Arg
            820                 825                 830

Ser Thr Thr Arg Leu Glu Asn Ile Met Trp Lys Gln Ile Thr Asn Glu
        835                 840                 845

Leu Asn Tyr Val Leu Trp Glu Gly Gly His Asp Leu Thr Val Val Ala
    850                 855                 860

Gly Asp Val Lys Gly Val Leu Ser Lys Gly Lys Arg Ala Leu Ala Pro
865                 870                 875                 880

Pro Val Asn Asp Leu Lys Tyr Ser Trp Lys Thr Trp Gly Lys Ala Lys
                885                 890                 895

Ile Phe Thr Pro Glu Ala Lys Asn Ser Thr Phe Leu Ile Asp Gly Pro
            900                 905                 910

Asp Thr Ser Glu Cys Pro Asn Glu Arg Arg Ala Trp Asn Phe Leu Glu
        915                 920                 925

Val Glu Asp Tyr Gly Phe Gly Met Phe Thr Thr Asn Ile Trp Met Lys
    930                 935                 940

Phe Arg Glu Gly Ser Ser Glu Val Cys Asp His Arg Leu Met Ser Ala
945                 950                 955                 960

Ala Ile Lys Asp Gln Lys Ala Val His Ala Asp Met Gly Tyr Trp Ile
                965                 970                 975

Glu Ser Ser Lys Asn Gln Thr Trp Gln Ile Glu Lys Ala Ser Leu Ile
            980                 985                 990

Glu Val Lys Thr Cys Leu Trp Pro Lys Thr His Thr Leu Trp Ser Asn
        995                 1000                1005

Gly Val Leu Glu Ser Gln Met Leu Ile Pro Lys Ala Tyr Ala Gly
    1010                1015                1020

Pro Phe Ser Gln His Asn Tyr Arg Gln Gly Tyr Ala Thr Gln Thr
    1025                1030                1035

Val Gly Pro Trp His Leu Gly Lys Leu Glu Ile Asp Phe Gly Glu
    1040                1045                1050

Cys Pro Gly Thr Thr Val Thr Ile Gln Glu Asp Cys Asp His Arg
    1055                1060                1065

Gly Pro Ser Leu Arg Thr Thr Thr Ala Ser Gly Lys Leu Val Thr
    1070                1075                1080

Gln Trp Cys Cys Arg Ser Cys Thr Met Pro Pro Leu Arg Phe Leu
    1085                1090                1095

Gly Glu Asp Gly Cys Trp Tyr Gly Met Glu Ile Arg Pro Leu Ser
    1100                1105                1110

Glu Lys Glu Glu Asn Met Val Lys Ser Gln Val Ser Ala Gly Gln
    1115                1120                1125

Gly Thr Ser Glu Thr Phe Ser Met Gly Leu Leu Cys Leu Thr Leu
    1130                1135                1140

Phe Val Glu Glu Cys Leu Arg Arg Arg Val Thr Arg Lys His Met
    1145                1150                1155

Ile Leu Val Val Val Thr Thr Leu Cys Ala Ile Ile Leu Gly Gly
    1160                1165                1170

Leu Thr Trp Met Asp Leu Leu Arg Ala Leu Ile Met Leu Gly Asp
    1175                1180                1185

Thr Met Ser Gly Arg Met Gly Gly Gln Ile His Leu Ala Ile Met
    1190                1195                1200

Ala Val Phe Lys Met Ser Pro Gly Tyr Val Leu Gly Ile Phe Leu
    1205                1210                1215

Arg Lys Leu Thr Ser Arg Glu Thr Ala Leu Met Val Ile Gly Met
    1220                1225                1230

Ala Met Thr Thr Val Leu Ser Ile Pro His Asp Leu Met Glu Phe
    1235                1240                1245

Ile Asp Gly Ile Ser Leu Gly Leu Ile Leu Leu Lys Met Val Thr
    1250                1255                1260

His Phe Asp Asn Thr Gln Val Gly Thr Leu Ala Leu Ser Leu Thr
    1265                1270                1275

Phe Ile Arg Ser Thr Met Pro Leu Val Met Ala Trp Arg Thr Ile
    1280                1285                1290

Met Ala Val Leu Phe Val Val Thr Leu Ile Pro Leu Cys Arg Thr
    1295                1300                1305

Ser Cys Leu Gln Lys Gln Ser His Trp Val Glu Ile Thr Ala Leu
    1310                1315                1320

Ile Leu Gly Ala Gln Ala Leu Pro Val Tyr Leu Met Thr Leu Met
    1325                1330                1335

Lys Gly Ala Ser Lys Arg Ser Trp Pro Leu Asn Glu Gly Ile Met
    1340                1345                1350

Ala Val Gly Leu Val Ser Leu Leu Gly Ser Ala Leu Leu Lys Asn
    1355                1360                1365

Asp Val Pro Leu Ala Gly Pro Met Val Ala Gly Gly Leu Leu Leu
    1370                1375                1380

Ala Ala Tyr Val Met Ser Gly Ser Ser Ala Asp Leu Ser Leu Glu
    1385                1390                1395

Lys Ala Ala Asn Val Gln Trp Asp Glu Met Ala Asp Ile Thr Gly
    1400                1405                1410

Ser Ser Pro Ile Ile Glu Val Lys Gln Asp Glu Asp Gly Ser Phe
    1415                1420                1425

Ser Ile Arg Asp Ile Glu Glu Thr Asn Met Ile Thr Leu Leu Val
    1430                1435                1440

Lys Leu Ala Leu Ile Thr Val Ser Gly Leu Tyr Pro Leu Ala Ile
    1445                1450                1455

Pro Val Thr Met Thr Leu Trp Tyr Met Trp Gln Val Lys Thr Gln
    1460                1465                1470

Arg Ser Gly Ala Leu Trp Asp Val Pro Ser Pro Ala Ala Ala Gln
    1475                1480                1485

Lys Ala Thr Leu Thr Glu Gly Val Tyr Arg Ile Met Gln Arg Gly
    1490                1495                1500

Leu Phe Gly Lys Thr Gln Val Gly Val Gly Ile His Met Glu Gly
    1505                1510                1515

Val Phe His Thr Met Trp His Val Thr Arg Gly Ser Val Ile Cys
    1520                1525                1530

His Glu Thr Gly Arg Leu Glu Pro Ser Trp Ala Asp Val Arg Asn
    1535                1540                1545

Asp Met Ile Ser Tyr Gly Gly Gly Trp Arg Leu Gly Asp Lys Trp
    1550                1555                1560

Asp Lys Glu Glu Asp Val Gln Val Leu Ala Ile Glu Pro Gly Lys
    1565                1570                1575

Asn Pro Lys His Val Gln Thr Lys Pro Gly Leu Phe Lys Thr Leu
    1580                1585                1590

Thr Gly Glu Ile Gly Ala Val Thr Leu Asp Phe Lys Pro Gly Thr
    1595                1600                1605

Ser Gly Ser Pro Ile Ile Asn Arg Lys Gly Lys Val Ile Gly Leu
    1610                1615                1620

Tyr Gly Asn Gly Val Val Thr Lys Ser Gly Asp Tyr Val Ser Ala
    1625                1630                1635

Ile Thr Gln Ala Glu Arg Thr Gly Glu Pro Asp Tyr Glu Val Asp
    1640                1645                1650

Glu Asp Ile Phe Arg Lys Lys Arg Leu Thr Ile Met Asp Leu His
    1655                1660                1665

Pro Gly Ala Gly Lys Thr Lys Arg Ile Leu Pro Ser Ile Val Arg
    1670                1675                1680

Glu Ala Leu Lys Arg Arg Leu Arg Thr Leu Ile Leu Ala Pro Thr
    1685                1690                1695

Arg Val Val Ala Ala Glu Met Glu Glu Ala Leu Arg Gly Leu Pro
    1700                1705                1710

Ile Arg Tyr Gln Thr Pro Ala Val Lys Ser Glu His Thr Gly Arg
    1715                1720                1725

Glu Ile Val Asp Leu Met Cys His Ala Thr Phe Thr Thr Arg Leu
    1730                1735                1740

Leu Ser Ser Thr Arg Val Pro Asn Tyr Asn Leu Ile Val Met Asp
    1745                1750                1755

Glu Ala His Phe Thr Asp Pro Ser Ser Val Ala Ala Arg Gly Tyr

1760                1765                1770

Ile Ser Thr Arg Val Glu Met Gly Glu Ala Ala Ala Ile Phe Met
    1775                1780                1785

Thr Ala Thr Pro Pro Gly Ala Thr Asp Pro Phe Pro Gln Ser Asn
    1790                1795                1800

Ser Pro Ile Glu Asp Ile Glu Arg Glu Ile Pro Glu Arg Ser Trp
    1805                1810                1815

Asn Thr Gly Phe Asp Trp Ile Thr Asp Tyr Gln Gly Lys Thr Val
    1820                1825                1830

Trp Phe Val Pro Ser Ile Lys Ala Gly Asn Asp Ile Ala Asn Cys
    1835                1840                1845

Leu Arg Lys Ser Gly Lys Lys Val Ile Gln Leu Ser Arg Lys Thr
    1850                1855                1860

Phe Asp Thr Glu Tyr Pro Lys Thr Lys Leu Thr Asp Trp Asp Phe
    1865                1870                1875

Val Val Thr Thr Asp Ile Ser Glu Met Gly Ala Asn Phe Arg Ala
    1880                1885                1890

Gly Arg Val Ile Asp Pro Arg Arg Cys Leu Lys Pro Val Ile Ser
    1895                1900                1905

Thr Asp Gly Pro Glu Arg Val Ile Leu Ala Gly Pro Ile Pro Val
    1910                1915                1920

Thr Pro Ala Ser Ala Ala Gln Arg Arg Gly Arg Ile Gly Arg Asn
    1925                1930                1935

Pro Ala Gln Glu Asp Asp Gln Tyr Val Phe Ser Gly Asp Pro Leu
    1940                1945                1950

Lys Asn Asp Glu Asp His Ala His Trp Thr Glu Ala Lys Met Leu
    1955                1960                1965

Leu Asp Asn Ile Tyr Thr Pro Glu Gly Ile Ile Pro Thr Leu Phe
    1970                1975                1980

Gly Pro Glu Arg Glu Lys Asn Gln Ala Ile Asp Gly Glu Phe Arg
    1985                1990                1995

Leu Arg Gly Glu Gln Arg Lys Thr Phe Val Glu Leu Met Arg Arg
    2000                2005                2010

Gly Asp Leu Pro Val Trp Leu Ser Tyr Lys Val Ala Ser Ala Gly
    2015                2020                2025

Ile Ser Tyr Lys Asp Arg Glu Trp Cys Phe Thr Gly Glu Arg Asn
    2030                2035                2040

Asn Gln Ile Leu Glu Glu Asn Met Glu Val Glu Ile Trp Thr Arg
    2045                2050                2055

Glu Gly Glu Lys Lys Lys Leu Arg Pro Lys Trp Leu Asp Ala Arg
    2060                2065                2070

Val Tyr Ala Asp Pro Met Ala Leu Lys Asp Phe Lys Glu Phe Ala
    2075                2080                2085

Ser Gly Arg Lys Ser Ile Thr Leu Asp Ile Leu Thr Glu Ile Ala
    2090                2095                2100

Ser Leu Pro Thr Tyr Leu Ser Ser Arg Ala Lys Leu Ala Leu Asp
    2105                2110                2115

Asn Ile Val Met Leu His Thr Thr Glu Arg Gly Gly Lys Ala Tyr
    2120                2125                2130

Gln His Ala Leu Asn Glu Leu Pro Glu Ser Leu Glu Thr Leu Met
    2135                2140                2145

Leu Val Ala Leu Leu Gly Ala Met Thr Ala Gly Ile Phe Leu Phe
    2150                2155                2160

Phe Met Gln Gly Lys Gly Ile Gly Lys Leu Ser Met Gly Leu Ile
    2165                2170                2175

Ala Ile Ala Val Ala Ser Gly Leu Leu Trp Val Ala Glu Ile Gln
    2180                2185                2190

Pro Gln Trp Ile Ala Ala Ser Ile Ile Leu Glu Phe Phe Leu Met
    2195                2200                2205

Val Leu Leu Ile Pro Glu Pro Glu Lys Gln Arg Thr Pro Gln Asp
    2210                2215                2220

Asn Gln Leu Ile Tyr Val Ile Leu Thr Ile Leu Thr Ile Ile Gly
    2225                2230                2235

Leu Ile Ala Ala Asn Glu Met Gly Leu Ile Glu Lys Thr Lys Thr
    2240                2245                2250

Asp Phe Gly Phe Tyr Gln Val Lys Thr Glu Thr Thr Ile Leu Asp
    2255                2260                2265

Val Asp Leu Arg Pro Ala Ser Ala Trp Thr Leu Tyr Ala Val Ala
    2270                2275                2280

Thr Thr Ile Leu Thr Pro Met Leu Arg His Thr Ile Glu Asn Thr
    2285                2290                2295

Ser Ala Asn Leu Ser Leu Ala Ala Ile Ala Asn Gln Ala Ala Val
    2300                2305                2310

Leu Met Gly Leu Gly Lys Gly Trp Pro Leu His Arg Met Asp Leu
    2315                2320                2325

Gly Val Pro Leu Leu Ala Met Gly Cys Tyr Ser Gln Val Asn Pro
    2330                2335                2340

Thr Thr Leu Ile Ala Ser Leu Val Met Leu Leu Val His Tyr Ala
    2345                2350                2355

Ile Ile Gly Pro Gly Leu Gln Ala Lys Ala Thr Arg Glu Ala Gln
    2360                2365                2370

Lys Arg Thr Ala Ala Gly Ile Met Lys Asn Pro Thr Val Asp Gly
    2375                2380                2385

Ile Thr Val Ile Asp Leu Glu Pro Ile Ser Tyr Asp Pro Lys Phe
    2390                2395                2400

Glu Lys Gln Leu Gly Gln Val Met Leu Leu Val Leu Cys Ala Gly
    2405                2410                2415

Gln Leu Leu Leu Met Arg Thr Thr Trp Ala Phe Cys Glu Val Leu
    2420                2425                2430

Thr Leu Ala Thr Gly Pro Val Leu Thr Leu Trp Glu Gly Asn Pro
    2435                2440                2445

Gly Arg Phe Trp Asn Thr Thr Ile Ala Val Ser Thr Ala Asn Ile
    2450                2455                2460

Phe Arg Gly Ser Tyr Leu Ala Gly Ala Gly Leu Ala Phe Ser Leu
    2465                2470                2475

Ile Lys Asn Ala Gln Thr Pro Arg Arg Gly Thr Gly Thr Thr Gly
    2480                2485                2490

Glu Thr Leu Gly Glu Lys Trp Lys Arg Gln Leu Asn Ser Leu Asp
    2495                2500                2505

Arg Lys Glu Phe Glu Glu Tyr Lys Arg Ser Gly Ile Leu Glu Val
    2510                2515                2520

Asp Arg Thr Glu Ala Lys Ser Ala Leu Lys Asp Gly Ser Lys Ile
    2525                2530                2535

Lys His Ala Val Ser Arg Gly Ser Ser Lys Ile Arg Trp Ile Val
    2540                2545                2550

Glu Arg Gly Met Val Lys Pro Lys Gly Lys Val Val Asp Leu Gly
    2555                2560                2565

Cys Gly Arg Gly Gly Trp Ser Tyr Tyr Met Ala Thr Leu Lys Asn
    2570                2575                2580

Val Thr Glu Val Lys Gly Tyr Thr Lys Gly Gly Pro Gly His Glu
    2585                2590                2595

Glu Pro Ile Pro Met Ala Thr Tyr Gly Trp Asn Leu Val Lys Leu
    2600                2605                2610

His Ser Gly Val Asp Val Phe Tyr Lys Pro Thr Glu Gln Val Asp
    2615                2620                2625

Thr Leu Leu Cys Asp Ile Gly Glu Ser Ser Ser Asn Pro Thr Ile
    2630                2635                2640

Glu Glu Gly Arg Thr Leu Arg Val Leu Lys Met Val Glu Pro Trp
    2645                2650                2655

Leu Ser Ser Lys Pro Glu Phe Cys Ile Lys Val Leu Asn Pro Tyr
    2660                2665                2670

Met Pro Thr Val Ile Glu Glu Leu Glu Lys Leu Gln Arg Lys His
    2675                2680                2685

Gly Gly Ser Leu Val Arg Cys Pro Leu Ser Arg Asn Ser Thr His
    2690                2695                2700

Glu Met Tyr Trp Val Ser Gly Val Ser Gly Asn Ile Val Ser Ser

2705                2710                2715

Val Asn Thr Thr Ser Lys Met Leu Leu Asn Arg Phe Thr Thr Arg
    2720                2725                2730

His Arg Lys Pro Thr Tyr Glu Lys Asp Val Asp Leu Gly Ala Gly
    2735                2740                2745

Thr Arg Ser Val Ser Thr Glu Thr Glu Lys Pro Asp Met Thr Ile
    2750                2755                2760

Ile Gly Arg Arg Leu Gln Arg Leu Gln Glu Glu His Lys Glu Thr
    2765                2770                2775

Trp His Tyr Asp Gln Glu Asn Pro Tyr Arg Thr Trp Ala Tyr His
    2780                2785                2790

Gly Ser Tyr Glu Ala Pro Ser Thr Gly Ser Ala Ser Ser Met Val
    2795                2800                2805

Asn Gly Val Val Lys Leu Leu Thr Lys Pro Trp Asp Val Ile Pro
    2810                2815                2820

Met Val Thr Gln Leu Ala Met Thr Asp Thr Thr Pro Phe Gly Gln
    2825                2830                2835

Gln Arg Val Phe Lys Glu Lys Val Asp Thr Arg Thr Pro Gln Pro
    2840                2845                2850

Lys Pro Gly Thr Arg Met Val Met Thr Thr Thr Ala Asn Trp Leu
    2855                2860                2865

Trp Ala Leu Leu Gly Lys Lys Lys Asn Pro Arg Leu Cys Thr Arg
    2870                2875                2880

Glu Glu Phe Ile Ser Lys Val Arg Ser Asn Ala Ala Ile Gly Ala
    2885                2890                2895

Val Phe Gln Glu Glu Gln Gly Trp Thr Ser Ala Ser Glu Ala Val
    2900                2905                2910

Asn Asp Ser Arg Phe Trp Glu Leu Val Asp Lys Glu Arg Ala Leu
    2915                2920                2925

His Gln Glu Gly Lys Cys Glu Ser Cys Val Tyr Asn Met Met Gly
    2930                2935                2940

Lys Arg Glu Lys Lys Leu Gly Glu Phe Gly Arg Ala Lys Gly Ser
    2945                2950                2955

Arg Ala Ile Trp Tyr Met Trp Leu Gly Ala Arg Phe Leu Glu Phe
    2960                2965                2970

Glu Ala Leu Gly Phe Leu Asn Glu Asp His Trp Phe Gly Arg Glu
    2975                2980                2985

Asn Ser Trp Ser Gly Val Glu Gly Glu Gly Leu His Arg Leu Gly
    2990                2995                3000

Tyr Ile Leu Glu Asp Ile Asp Lys Lys Asp Gly Asp Leu Ile Tyr
    3005                3010                3015

Ala Asp Asp Thr Ala Gly Trp Asp Thr Arg Ile Thr Glu Asp Asp
    3020                3025                3030

Leu Leu Asn Glu Glu Leu Ile Thr Glu Gln Met Ala Pro His His
    3035                3040                3045

Lys Ile Leu Ala Lys Ala Ile Phe Lys Leu Thr Tyr Gln Asn Lys
    3050                3055                3060

Val Val Lys Val Leu Arg Pro Thr Pro Lys Gly Ala Val Met Asp
    3065                3070                3075

Ile Ile Ser Arg Lys Asp Gln Arg Gly Ser Gly Gln Val Gly Thr
    3080                3085                3090

Tyr Gly Leu Asn Thr Phe Thr Asn Met Glu Val Gln Leu Ile Arg
    3095                3100                3105

Gln Met Glu Ala Glu Gly Val Ile Thr Gln Asp Asp Met His Asn
    3110                3115                3120

Pro Lys Gly Leu Lys Glu Arg Val Glu Lys Trp Leu Lys Glu Cys
    3125                3130                3135

Gly Val Asp Arg Leu Lys Arg Met Ala Ile Ser Gly Asp Asp Cys
    3140                3145                3150

Val Val Lys Pro Leu Asp Glu Arg Phe Ser Thr Ser Leu Leu Phe
    3155                3160                3165

Leu Asn Asp Met Gly Lys Val Arg Lys Asp Ile Pro Gln Trp Glu
    3170                3175                3180

Pro Ser Lys Gly Trp Lys Asn Trp Gln Glu Val Pro Phe Cys Ser
    3185                3190                3195

His His Phe His Lys Ile Phe Met Lys Asp Gly Arg Ser Leu Val
    3200                3205                3210

Val Pro Cys Arg Asn Gln Asp Glu Leu Ile Gly Arg Ala Arg Ile
    3215                3220                3225

Ser Gln Gly Ala Gly Trp Ser Leu Arg Glu Thr Ala Cys Leu Gly
    3230                3235                3240

Lys Ala Tyr Ala Gln Met Trp Ser Leu Met Tyr Phe His Arg Arg
    3245                3250                3255

Asp Leu Arg Leu Ala Ser Met Ala Ile Cys Ser Ala Val Pro Thr
    3260                3265                3270

Glu Trp Phe Pro Thr Ser Arg Thr Thr Trp Ser Ile His Ala His
    3275                3280                3285

His Gln Trp Met Thr Thr Glu Asp Met Leu Lys Val Trp Asn Arg
    3290                3295                3300

Val Trp Ile Glu Asp Asn Pro Asn Met Thr Asp Lys Thr Pro Val
    3305                3310                3315

His Ser Trp Glu Asp Ile Pro Tyr Leu Gly Lys Arg Glu Asp Leu
    3320                3325                3330

Trp Cys Gly Ser Leu Ile Gly Leu Ser Ser Arg Ala Thr Trp Ala
    3335                3340                3345

Lys Asn Ile His Thr Ala Ile Thr Gln Val Arg Asn Leu Ile Gly
    3350                3355                3360

Lys Glu Glu Tyr Val Asp Tyr Met Pro Val Met Lys Arg Tyr Ser
    3365                3370                3375

Ala Pro Phe Glu Ser Glu Gly Val Leu
    3380                3385

<210>  28
<211>  3430
<212>  PRT
<213>  West Nile virus

<400>  28

Met Ser Lys Lys Pro Gly Gly Pro Gly Lys Asn Arg Ala Val Asn Met
1               5                   10                  15

Leu Lys Arg Gly Met Pro Arg Gly Leu Ser Leu Ile Gly Leu Lys Arg
            20                  25                  30

Ala Met Leu Ser Leu Ile Asp Gly Lys Gly Pro Ile Arg Phe Val Leu
        35                  40                  45

Ala Leu Leu Ala Phe Phe Arg Phe Thr Ala Ile Ala Pro Thr Arg Ala
    50                  55                  60

Val Leu Asp Arg Trp Arg Gly Val Asn Lys Gln Thr Ala Met Lys His
65                  70                  75                  80

Leu Leu Ser Phe Lys Lys Glu Leu Gly Thr Leu Thr Ser Ala Ile Asn
                85                  90                  95

Arg Arg Ser Thr Lys Gln Lys Lys Arg Gly Gly Thr Ala Gly Phe Thr
            100                 105                 110

Ile Leu Leu Gly Leu Ile Ala Cys Ala Gly Ala Val Thr Leu Ser Asn
        115                 120                 125

Phe Gln Gly Lys Val Met Met Thr Val Asn Ala Thr Asp Val Thr Asp
    130                 135                 140

Val Ile Thr Ile Pro Thr Ala Ala Gly Lys Asn Leu Cys Ile Val Arg
145                 150                 155                 160

Ala Met Asp Val Gly Tyr Leu Cys Glu Asp Thr Ile Thr Tyr Glu Cys
                165                 170                 175

Pro Val Leu Ala Ala Gly Asn Asp Pro Glu Asp Ile Asp Cys Trp Cys
            180                 185                 190

Thr Lys Ser Ser Val Tyr Val Arg Tyr Gly Arg Cys Thr Lys Thr Arg
        195                 200                 205

His Ser Arg Arg Ser Arg Arg Ser Leu Thr Val Gln Thr His Gly Glu
    210                 215                 220

Ser Thr Leu Ala Asn Lys Lys Gly Ala Trp Leu Asp Ser Thr Lys Ala
225                 230                 235                 240

Thr Arg Tyr Leu Val Lys Thr Glu Ser Trp Ile Leu Arg Asn Pro Gly
                245                 250                 255

Tyr Ala Leu Val Ala Ala Val Ile Gly Trp Met Leu Gly Ser Asn Thr
        260                 265                 270

Met Gln Arg Val Val Phe Ala Ile Leu Leu Leu Leu Val Ala Pro Ala
    275                 280                 285

Tyr Ser Phe Asn Cys Leu Gly Met Ser Asn Arg Asp Phe Leu Glu Gly
    290                 295                 300

Val Ser Gly Ala Thr Trp Val Asp Leu Val Leu Glu Gly Asp Ser Cys
305                 310                 315                 320

Val Thr Ile Met Ser Lys Asp Lys Pro Thr Ile Asp Val Lys Met Met
                325                 330                 335

Asn Met Glu Ala Ala Asn Leu Ala Asp Val Arg Ser Tyr Cys Tyr Leu
        340                 345                 350

Ala Ser Val Ser Asp Leu Ser Thr Arg Ala Ala Cys Pro Thr Met Gly
    355                 360                 365

Glu Ala His Asn Glu Lys Arg Ala Asp Pro Ala Phe Val Cys Lys Gln
    370                 375                 380

Gly Val Val Asp Arg Gly Trp Gly Asn Gly Cys Gly Leu Phe Gly Lys
385                 390                 395                 400

Gly Ser Ile Asp Thr Cys Ala Lys Phe Ala Cys Thr Thr Lys Ala Thr
                405                 410                 415

Gly Trp Ile Ile Gln Lys Glu Asn Ile Lys Tyr Glu Val Ala Ile Phe
        420                 425                 430

Val His Gly Pro Thr Thr Val Glu Ser His Gly Lys Ile Gly Ala Thr
    435                 440                 445

Gln Ala Gly Arg Phe Ser Ile Thr Pro Ser Ala Pro Ser Tyr Thr Leu
    450                 455                 460

Lys Leu Gly Glu Tyr Gly Glu Val Thr Val Asp Cys Glu Pro Arg Ser
465                 470                 475                 480

Gly Ile Asp Thr Ser Ala Tyr Tyr Val Met Ser Val Gly Glu Lys Ser
                485                 490                 495

Phe Leu Val His Arg Glu Trp Phe Met Asp Leu Asn Leu Pro Trp Ser

            500                 505                 510

Ser Ala Gly Ser Thr Thr Trp Arg Asn Arg Glu Thr Leu Met Glu Phe
        515                 520                 525

Glu Glu Pro His Ala Thr Lys Gln Ser Val Val Ala Leu Gly Ser Gln
    530                 535                 540

Glu Gly Ala Leu His Gln Ala Leu Ala Gly Ala Ile Pro Val Glu Phe
545                 550                 555                 560

Ser Ser Asn Thr Val Lys Leu Thr Ser Gly His Leu Lys Cys Arg Val
                565                 570                 575

Lys Met Glu Lys Leu Gln Leu Lys Gly Thr Thr Tyr Gly Val Cys Ser
            580                 585                 590

Lys Ala Phe Lys Phe Ala Arg Thr Pro Ala Asp Thr Gly His Gly Thr
        595                 600                 605

Val Val Leu Glu Leu Gln Tyr Thr Gly Thr Asp Gly Pro Cys Lys Val
    610                 615                 620

Pro Ile Ser Ser Val Ala Ser Leu Asn Asp Leu Thr Pro Val Gly Arg
625                 630                 635                 640

Leu Val Thr Val Asn Pro Phe Val Ser Val Ala Thr Ala Asn Ser Lys
                645                 650                 655

Val Leu Ile Glu Leu Glu Pro Pro Phe Gly Asp Ser Tyr Ile Val Val
            660                 665                 670

Gly Arg Gly Glu Gln Gln Ile Asn His His Trp His Lys Ser Gly Ser
        675                 680                 685

Ser Ile Gly Lys Ala Phe Thr Thr Thr Leu Arg Gly Ala Gln Arg Leu
    690                 695                 700

Ala Ala Leu Gly Asp Thr Ala Trp Asp Phe Gly Ser Val Gly Gly Val
705                 710                 715                 720

Phe Thr Ser Val Gly Lys Ala Ile His Gln Val Phe Gly Gly Ala Phe
                725                 730                 735

Arg Ser Leu Phe Gly Gly Met Ser Trp Ile Thr Gln Gly Leu Leu Gly
            740                 745                 750

Ala Leu Leu Leu Trp Met Gly Ile Asn Ala Arg Asp Arg Ser Ile Ala
        755                 760                 765

Met Thr Phe Leu Ala Val Gly Gly Val Leu Leu Phe Leu Ser Val Asn
    770                 775                 780

Val His Ala Asp Thr Gly Cys Ala Ile Asp Ile Gly Arg Gln Glu Leu
785                 790                 795                 800

Arg Cys Gly Ser Gly Val Phe Ile His Asn Asp Val Glu Ala Trp Met
                805                 810                 815

Asp Arg Tyr Lys Phe Tyr Pro Glu Thr Pro Gln Gly Leu Ala Lys Ile
            820                 825                 830

Ile Gln Lys Ala His Ala Glu Gly Val Cys Gly Leu Arg Ser Val Ser
        835                 840                 845

Arg Leu Glu His Gln Met Trp Glu Ala Ile Lys Asp Glu Leu Asn Thr
    850                 855                 860

Leu Leu Lys Glu Asn Gly Val Asp Leu Ser Val Val Val Glu Lys Gln
865                 870                 875                 880

Asn Gly Met Tyr Lys Ala Ala Pro Lys Arg Leu Ala Ala Thr Thr Glu
                885                 890                 895

Lys Leu Glu Met Gly Trp Lys Ala Trp Gly Lys Ser Ile Ile Phe Ala
            900                 905                 910

Pro Glu Leu Ala Asn Asn Thr Phe Val Ile Asp Gly Pro Glu Thr Glu
        915                 920                 925

Glu Cys Pro Thr Ala Asn Arg Ala Trp Asn Ser Met Glu Val Glu Asp
    930                 935                 940

Phe Gly Phe Gly Leu Thr Ser Thr Arg Met Phe Leu Arg Ile Arg Glu
945                 950                 955                 960

Thr Asn Thr Thr Glu Cys Asp Ser Lys Ile Ile Gly Thr Ala Val Lys
                965                 970                 975

Asn Asn Met Ala Val His Ser Asp Leu Ser Tyr Trp Ile Glu Ser Gly
            980                 985                 990

Leu Asn Asp Thr Trp Lys Leu Glu  Arg Ala Val Leu Gly  Glu Val Lys
        995                 1000                1005

Ser Cys Thr Trp Pro Glu Thr His Thr Leu Trp Gly Asp Gly Val
    1010                1015                1020

Leu Glu Ser Asp Leu Ile Ile Pro Ile Thr Leu Ala Gly Pro Arg
    1025                1030                1035

Ser Asn His Asn Arg Arg Pro Gly Tyr Lys Thr Gln Asn Gln Gly
    1040                1045                1050

Pro Trp Asp Glu Gly Arg Val Glu Ile Asp Phe Asp Tyr Cys Pro
    1055                1060                1065

Gly Thr Thr Val Thr Ile Ser Asp Ser Cys Glu His Arg Gly Pro
    1070                1075                1080

Ala Ala Arg Thr Thr Thr Glu Ser Gly Lys Leu Ile Thr Asp Trp
    1085                1090                1095

Cys Cys Arg Ser Cys Thr Leu Pro Pro Leu Arg Phe Gln Thr Glu
    1100                1105                1110

Asn Gly Cys Trp Tyr Gly Met Glu Ile Arg Pro Thr Arg His Asp
    1115                1120                1125

Glu Lys Thr Leu Val Gln Ser Arg Val Asn Ala Tyr Asn Ala Asp
    1130                1135                1140

Met Ile Asp Pro Phe Gln Leu Gly Leu Met Val Val Phe Leu Ala
    1145                1150                1155

Thr Gln Glu Val Leu Arg Lys Arg Trp Thr Ala Lys Ile Ser Ile
    1160                1165                1170

Pro Ala Ile Met Leu Ala Leu Leu Val Leu Val Phe Gly Gly Ile
    1175                1180                1185

Thr Tyr Thr Asp Val Leu Arg Tyr Val Ile Leu Val Gly Ala Ala
    1190                1195                1200

Phe Ala Glu Ala Asn Ser Gly Gly Asp Val Val His Leu Ala Leu
    1205                1210                1215

Met Ala Thr Phe Lys Ile Gln Pro Val Phe Leu Val Ala Ser Phe
    1220                1225                1230

Leu Lys Ala Arg Trp Thr Asn Gln Glu Ser Ile Leu Leu Met Leu
    1235                1240                1245

Ala Ala Ala Phe Phe Gln Met Ala Tyr Tyr Asp Ala Lys Asn Val
    1250                1255                1260

Leu Ser Trp Glu Val Pro Asp Val Leu Asn Ser Leu Ser Val Ala
    1265                1270                1275

Trp Met Ile Leu Arg Ala Ile Ser Phe Thr Asn Thr Ser Asn Val
    1280                1285                1290

Val Val Pro Leu Leu Ala Leu Leu Thr Pro Gly Leu Lys Cys Leu
    1295                1300                1305

Asn Leu Asp Val Tyr Arg Ile Leu Leu Leu Met Val Gly Val Gly
    1310                1315                1320

Ser Leu Ile Lys Glu Lys Arg Ser Ser Ala Ala Lys Lys Lys Gly
    1325                1330                1335

Ala Cys Leu Ile Cys Leu Ala Leu Ala Ser Thr Gly Val Phe Asn
    1340                1345                1350

Pro Met Ile Leu Ala Ala Gly Leu Met Ala Cys Asp Pro Asn Arg
    1355                1360                1365

Lys Arg Gly Trp Pro Ala Thr Glu Val Met Thr Ala Val Gly Leu
    1370                1375                1380

Met Phe Ala Ile Val Gly Gly Leu Ala Glu Leu Asp Ile Asp Ser
    1385                1390                1395

Met Ala Ile Pro Met Thr Ile Ala Gly Leu Met Phe Ala Ala Phe
    1400                1405                1410

Val Ile Ser Gly Lys Ser Thr Asp Met Trp Ile Glu Arg Thr Ala
    1415                1420                1425

Asp Ile Thr Trp Glu Ser Asp Ala Glu Ile Thr Gly Ser Ser Glu
    1430                1435                1440

Arg Val Asp Val Arg Leu Asp Asp Asp Gly Asn Phe Gln Leu Met
    1445                1450                1455

Asn Asp Pro Gly Ala Pro Trp Lys Ile Trp Met Leu Arg Met Ala
    1460                1465                1470

Cys Leu Ala Ile Ser Ala Tyr Thr Pro Trp Ala Ile Leu Pro Ser

1475                1480                1485

Val Ile Gly Phe Trp Ile Thr Leu Gln Tyr Thr Lys Arg Gly Gly
    1490                1495                1500

Val Leu Trp Asp Thr Pro Ser Pro Lys Glu Tyr Lys Lys Gly Asp
    1505                1510                1515

Thr Thr Thr Gly Val Tyr Arg Ile Met Thr Arg Gly Leu Leu Gly
    1520                1525                1530

Ser Tyr Gln Ala Gly Ala Gly Val Met Val Glu Gly Val Phe His
    1535                1540                1545

Thr Leu Trp His Thr Thr Lys Gly Ala Ala Leu Met Ser Gly Glu
    1550                1555                1560

Gly Arg Leu Asp Pro Tyr Trp Gly Ser Val Lys Glu Asp Arg Leu
    1565                1570                1575

Cys Tyr Gly Gly Pro Trp Lys Leu Gln His Lys Trp Asn Gly His
    1580                1585                1590

Asp Glu Val Gln Met Ile Val Val Glu Pro Gly Lys Asn Val Lys
    1595                1600                1605

Asn Val Gln Thr Lys Pro Gly Val Phe Lys Thr Pro Glu Gly Glu
    1610                1615                1620

Ile Gly Ala Val Thr Leu Asp Tyr Pro Thr Gly Thr Ser Gly Ser
    1625                1630                1635

Pro Ile Val Asp Lys Asn Gly Asp Val Ile Gly Leu Tyr Gly Asn
    1640                1645                1650

Gly Val Ile Met Pro Asn Gly Ser Tyr Ile Ser Ala Ile Val Gln
    1655                1660                1665

Gly Glu Arg Met Glu Glu Pro Ala Pro Ala Gly Phe Glu Pro Glu
    1670                1675                1680

Met Leu Arg Lys Lys Gln Ile Thr Val Leu Asp Leu His Pro Gly
    1685                1690                1695

Ala Gly Lys Thr Arg Lys Ile Leu Pro Gln Ile Ile Lys Glu Ala
    1700                1705                1710

Ile Asn Lys Arg Leu Arg Thr Ala Val Leu Ala Pro Thr Arg Val
    1715                1720                1725

Val Ala Ala Glu Met Ser Glu Ala Leu Arg Gly Leu Pro Ile Arg
    1730                1735                1740

Tyr Gln Thr Ser Ala Val His Arg Glu His Ser Gly Asn Glu Ile
    1745                1750                1755

Val Asp Val Met Cys His Ala Thr Leu Thr His Arg Leu Met Ser
    1760                1765                1770

Pro His Arg Val Pro Asn Tyr Asn Leu Phe Ile Met Asp Glu Ala
    1775                1780                1785

His Phe Thr Asp Pro Ala Ser Ile Ala Ala Arg Gly Tyr Ile Ala
    1790                1795                1800

Thr Lys Val Glu Leu Gly Glu Ala Ala Ala Ile Phe Met Thr Ala
    1805                1810                1815

Thr Pro Pro Gly Thr Ser Asp Pro Phe Pro Glu Ser Asn Ala Pro
    1820                1825                1830

Ile Ser Asp Met Gln Thr Glu Ile Pro Asp Arg Ala Trp Asn Thr
    1835                1840                1845

Gly Tyr Glu Trp Ile Thr Glu Tyr Val Gly Lys Thr Val Trp Phe
    1850                1855                1860

Val Pro Ser Val Lys Met Gly Asn Glu Ile Ala Leu Cys Leu Gln
    1865                1870                1875

Arg Ala Gly Lys Lys Val Ile Gln Leu Asn Arg Lys Ser Tyr Glu
    1880                1885                1890

Thr Glu Tyr Pro Lys Cys Lys Asn Asp Asp Trp Asp Phe Val Ile
    1895                1900                1905

Thr Thr Asp Ile Ser Glu Met Gly Ala Asn Phe Lys Ala Ser Arg
    1910                1915                1920

Val Ile Asp Ser Arg Lys Ser Val Lys Pro Thr Ile Ile Glu Glu
    1925                1930                1935

Gly Asp Gly Arg Val Ile Leu Gly Glu Pro Ser Ala Ile Thr Ala
    1940                1945                1950

Ala Ser Ala Ala Gln Arg Arg Gly Arg Ile Gly Arg Asn Pro Ser
    1955                1960                1965

Gln Val Gly Asp Glu Tyr Cys Tyr Gly Gly His Thr Asn Glu Asp
    1970                1975                1980

Asp Ser Asn Phe Ala His Trp Thr Glu Ala Arg Ile Met Leu Asp
    1985                1990                1995

Asn Ile Asn Met Pro Asn Gly Leu Val Ala Gln Leu Tyr Gln Pro
    2000                2005                2010

Glu Arg Glu Lys Val Tyr Thr Met Asp Gly Glu Tyr Arg Leu Arg
    2015                2020                2025

Gly Glu Glu Arg Lys Asn Phe Leu Glu Phe Leu Arg Thr Ala Asp
    2030                2035                2040

Leu Pro Val Trp Leu Ala Tyr Lys Val Ala Ala Ala Gly Ile Ser
    2045                2050                2055

Tyr His Asp Arg Lys Trp Cys Phe Asp Gly Pro Arg Thr Asn Thr
    2060                2065                2070

Ile Leu Glu Asp Asn Asn Glu Val Glu Val Ile Thr Lys Leu Gly
    2075                2080                2085

Glu Arg Lys Ile Leu Arg Pro Arg Trp Ala Asp Ala Arg Val Tyr
    2090                2095                2100

Ser Asp His Gln Ala Leu Lys Ser Phe Lys Asp Phe Ala Ser Gly
    2105                2110                2115

Lys Arg Ser Gln Ile Gly Leu Val Glu Val Leu Gly Arg Met Pro
    2120                2125                2130

Glu His Phe Met Val Lys Thr Trp Glu Ala Leu Asp Thr Met Tyr
    2135                2140                2145

Val Val Ala Thr Ala Glu Lys Gly Gly Arg Ala His Arg Met Ala
    2150                2155                2160

Leu Glu Glu Leu Pro Asp Ala Leu Gln Thr Ile Val Leu Ile Ala
    2165                2170                2175

Leu Leu Ser Val Met Ser Leu Gly Val Phe Phe Leu Leu Met Gln
    2180                2185                2190

Arg Lys Gly Ile Gly Lys Ile Gly Leu Gly Gly Val Ile Leu Gly
    2195                2200                2205

Ala Ala Thr Phe Phe Cys Trp Met Ala Glu Val Pro Gly Thr Lys
    2210                2215                2220

Ile Ala Gly Met Leu Leu Leu Ser Leu Leu Leu Met Ile Val Leu
    2225                2230                2235

Ile Pro Glu Pro Glu Lys Gln Arg Ser Gln Thr Asp Asn Gln Leu
    2240                2245                2250

Ala Val Phe Leu Ile Cys Val Leu Thr Leu Val Gly Ala Val Ala
    2255                2260                2265

Ala Asn Glu Met Gly Trp Leu Asp Lys Thr Lys Asn Asp Ile Gly
    2270                2275                2280

Ser Leu Leu Gly His Arg Pro Glu Ala Arg Glu Thr Thr Leu Gly
    2285                2290                2295

Val Glu Ser Phe Leu Leu Asp Leu Arg Pro Ala Thr Ala Trp Ser
    2300                2305                2310

Leu Tyr Ala Val Thr Thr Ala Val Leu Thr Pro Leu Leu Lys His
    2315                2320                2325

Leu Ile Thr Ser Asp Tyr Ile Asn Thr Ser Leu Thr Ser Ile Asn
    2330                2335                2340

Val Gln Ala Ser Ala Leu Phe Thr Leu Ala Arg Gly Phe Pro Phe
    2345                2350                2355

Val Asp Val Gly Val Ser Ala Leu Leu Leu Ala Val Gly Cys Trp
    2360                2365                2370

Gly Gln Val Thr Leu Thr Val Thr Val Thr Ala Ala Ala Leu Leu
    2375                2380                2385

Phe Cys His Tyr Ala Tyr Met Val Pro Gly Trp Gln Ala Glu Ala
    2390                2395                2400

Met Arg Ser Ala Gln Arg Arg Thr Ala Ala Gly Ile Met Lys Asn
    2405                2410                2415

Val Val Val Asp Gly Ile Val Ala Thr Asp Val Pro Glu Leu Glu

2420                2425                2430

Arg Thr Thr Pro Val Met Gln Lys Lys Val Gly Gln Ile Ile Leu
    2435                2440                2445

Ile Leu Val Ser Met Ala Ala Val Val Val Asn Pro Ser Val Arg
    2450                2455                2460

Thr Val Arg Glu Ala Gly Ile Leu Thr Thr Ala Ala Ala Val Thr
    2465                2470                2475

Leu Trp Glu Asn Gly Ala Ser Ser Val Trp Asn Ala Thr Thr Ala
    2480                2485                2490

Ile Gly Leu Cys His Ile Met Arg Gly Gly Trp Leu Ser Cys Leu
    2495                2500                2505

Ser Ile Met Trp Thr Leu Ile Lys Asn Met Glu Lys Pro Gly Leu
    2510                2515                2520

Lys Arg Gly Gly Ala Lys Gly Arg Thr Leu Gly Glu Val Trp Lys
    2525                2530                2535

Glu Arg Leu Asn His Met Thr Lys Glu Glu Phe Thr Arg Tyr Arg
    2540                2545                2550

Lys Glu Ala Ile Thr Glu Val Asp Arg Ser Ala Ala Lys His Ala
    2555                2560                2565

Arg Arg Glu Gly Asn Ile Thr Gly Gly His Pro Val Ser Arg Gly
    2570                2575                2580

Thr Ala Lys Leu Arg Trp Leu Val Glu Arg Arg Phe Leu Glu Pro
    2585                2590                2595

Val Gly Lys Val Val Asp Leu Gly Cys Gly Arg Gly Gly Trp Cys
    2600                2605                2610

Tyr Tyr Met Ala Thr Gln Lys Arg Val Gln Glu Val Lys Gly Tyr
    2615                2620                2625

Thr Lys Gly Gly Pro Gly His Glu Glu Pro Gln Leu Val Gln Ser
    2630                2635                2640

Tyr Gly Trp Asn Ile Val Thr Met Lys Ser Gly Val Asp Val Phe
    2645                2650                2655

Tyr Arg Pro Ser Glu Ala Ser Asp Thr Leu Leu Cys Asp Ile Gly
    2660                2665                2670

Glu Ser Ser Ser Ser Ala Glu Val Glu Glu His Arg Thr Val Arg
    2675                2680                2685

Val Leu Glu Met Val Glu Asp Trp Leu His Arg Gly Pro Lys Glu
    2690                2695                2700

Phe Cys Ile Lys Val Leu Cys Pro Tyr Met Pro Lys Val Ile Glu
    2705                2710                2715

Lys Met Glu Thr Leu Gln Arg Arg Tyr Gly Gly Gly Leu Ile Arg
    2720                2725                2730

Asn Pro Leu Ser Arg Asn Ser Thr His Glu Met Tyr Trp Val Ser
    2735                2740                2745

His Ala Ser Gly Asn Ile Val His Ser Val Asn Met Thr Ser Gln
    2750                2755                2760

Val Leu Leu Gly Arg Met Glu Lys Lys Thr Trp Lys Gly Pro Gln
    2765                2770                2775

Phe Glu Glu Asp Val Asn Leu Gly Ser Gly Thr Arg Ala Val Gly
    2780                2785                2790

Lys Pro Leu Leu Asn Ser Asp Thr Ser Lys Ile Lys Asn Arg Ile
    2795                2800                2805

Glu Arg Leu Lys Lys Glu Tyr Ser Ser Thr Trp His Gln Asp Ala
    2810                2815                2820

Asn His Pro Tyr Arg Thr Trp Asn Tyr His Gly Ser Tyr Glu Val
    2825                2830                2835

Lys Pro Thr Gly Ser Ala Ser Ser Leu Val Asn Gly Val Val Arg
    2840                2845                2850

Leu Leu Ser Lys Pro Trp Asp Thr Ile Thr Asn Val Thr Thr Met
    2855                2860                2865

Ala Met Thr Asp Thr Thr Pro Phe Gly Gln Gln Arg Val Phe Lys
    2870                2875                2880

Glu Lys Val Asp Thr Lys Ala Pro Glu Pro Pro Glu Gly Val Lys
    2885                2890                2895

Tyr Val Leu Asn Glu Thr Thr Asn Trp Leu Trp Ala Phe Leu Ala
    2900                2905                2910

Arg Asp Lys Lys Pro Arg Met Cys Ser Arg Glu Glu Phe Ile Gly
    2915                2920                2925

Lys Val Asn Ser Asn Ala Ala Leu Gly Ala Met Phe Glu Glu Gln
    2930                2935                2940

Asn Gln Trp Lys Asn Ala Arg Glu Ala Val Glu Asp Pro Lys Phe
    2945                2950                2955

Trp Glu Met Val Asp Glu Glu Arg Glu Ala His Leu Arg Gly Glu
    2960                2965                2970

Cys Asn Thr Cys Ile Tyr Asn Met Met Gly Lys Arg Glu Lys Lys
    2975                2980                2985

Pro Gly Glu Phe Gly Lys Ala Lys Gly Ser Arg Ala Ile Trp Phe
    2990                2995                3000

Met Trp Leu Gly Ala Arg Phe Leu Glu Phe Glu Ala Leu Gly Phe
    3005                3010                3015

Leu Asn Glu Asp His Trp Leu Gly Arg Lys Asn Ser Gly Gly Gly
    3020                3025                3030

Val Glu Gly Leu Gly Leu Gln Lys Leu Gly Tyr Ile Leu Lys Glu
    3035                3040                3045

Val Gly Thr Lys Pro Gly Gly Lys Val Tyr Ala Asp Asp Thr Ala
    3050                3055                3060

Gly Trp Asp Thr Arg Ile Thr Lys Ala Asp Leu Glu Asn Glu Ala
    3065                3070                3075

Lys Val Leu Glu Leu Leu Asp Gly Glu His Arg Arg Leu Ala Arg
    3080                3085                3090

Ser Ile Ile Glu Leu Thr Tyr Arg His Lys Val Val Lys Val Met
    3095                3100                3105

Arg Pro Ala Ala Asp Gly Lys Thr Val Met Asp Val Ile Ser Arg
    3110                3115                3120

Glu Asp Gln Arg Gly Ser Gly Gln Val Val Thr Tyr Ala Leu Asn
    3125                3130                3135

Thr Phe Thr Asn Leu Ala Val Gln Leu Val Arg Met Met Glu Gly
    3140                3145                3150

Glu Gly Val Ile Gly Pro Asp Asp Val Glu Lys Leu Gly Lys Gly
    3155                3160                3165

Lys Gly Pro Lys Val Arg Thr Trp Leu Phe Glu Asn Gly Glu Glu
    3170                3175                3180

Arg Leu Ser Arg Met Ala Val Ser Gly Asp Asp Cys Val Val Lys
    3185                3190                3195

Pro Leu Asp Asp Arg Phe Ala Thr Ser Leu His Phe Leu Asn Ala
    3200                3205                3210

Met Ser Lys Val Arg Lys Asp Ile Gln Glu Trp Lys Pro Ser Thr
    3215                3220                3225

Gly Trp Tyr Asp Trp Gln Gln Val Pro Phe Cys Ser Asn His Phe
    3230                3235                3240

Thr Glu Leu Ile Met Lys Asp Gly Arg Thr Leu Val Val Pro Cys
    3245                3250                3255

Arg Gly Gln Asp Glu Leu Ile Gly Arg Ala Arg Ile Ser Pro Gly
    3260                3265                3270

Ala Gly Trp Asn Val Arg Asp Thr Ala Cys Leu Ala Lys Ser Tyr
    3275                3280                3285

Ala Gln Met Trp Leu Leu Leu Tyr Phe His Arg Arg Asp Leu Arg
    3290                3295                3300

Leu Met Ala Asn Ala Ile Cys Ser Ala Val Pro Ala Asn Trp Val
    3305                3310                3315

Pro Thr Gly Arg Thr Thr Trp Ser Ile His Ala Lys Gly Glu Trp
    3320                3325                3330

Met Thr Thr Glu Asp Met Leu Ala Val Trp Asn Arg Val Trp Ile
    3335                3340                3345

Glu Glu Asn Glu Trp Met Glu Asp Lys Thr Pro Val Glu Arg Trp
    3350                3355                3360

Ser Asp Val Pro Tyr Ser Gly Lys Arg Glu Asp Ile Trp Cys Gly

3365                3370                3375

Ser Leu  Ile Gly Thr Arg Thr  Arg Ala Thr Trp Ala  Glu Asn Ile
    3380                3385                3390

His Val  Ala Ile Asn Gln Val  Arg Ser Val Ile Gly  Glu Glu Lys
    3395                3400                3405

Tyr Val  Asp Tyr Met Ser Ser  Leu Arg Arg Tyr Glu  Asp Thr Ile
    3410                3415                3420

Val Val  Glu Asp Thr Val Leu
    3425                3430

<210>  29
<211>  3432
<212>  PRT
<213>  Japanese encephalitis virus

<400>  29

Met Thr Lys Lys Pro Gly Gly Pro Gly Lys Asn Arg Ala Ile Asn Met
1               5                   10                  15

Leu Lys Arg Gly Leu Pro Arg Val Phe Pro Leu Val Gly Val Lys Arg
            20                  25                  30

Val Val Met Ser Leu Leu Asp Gly Arg Gly Pro Val Arg Phe Val Leu
        35                  40                  45

Ala Leu Ile Thr Phe Phe Lys Phe Thr Ala Leu Ala Pro Thr Lys Ala
    50                  55                  60

Leu Leu Gly Arg Trp Lys Ala Val Glu Lys Ser Val Ala Met Lys His
65                  70                  75                  80

Leu Thr Ser Phe Lys Arg Glu Leu Gly Thr Leu Ile Asp Ala Val Asn
                85                  90                  95

Lys Arg Gly Arg Lys Gln Asn Lys Arg Gly Gly Asn Glu Gly Ser Ile
            100                 105                 110

Met Trp Leu Ala Ser Leu Ala Val Val Ile Ala Cys Ala Gly Ala Met
        115                 120                 125

Lys Leu Ser Asn Phe Gln Gly Lys Leu Leu Met Thr Ile Asn Asn Thr
    130                 135                 140

Asp Ile Ala Asp Val Ile Val Ile Pro Thr Ser Lys Gly Glu Asn Arg

145                 150                 155                 160

Cys Trp Val Arg Ala Ile Asp Val Gly Tyr Met Cys Glu Asp Thr Ile
                165                 170                 175

Thr Tyr Glu Cys Pro Lys Leu Thr Met Gly Asn Asp Pro Glu Asp Val
            180                 185                 190

Asp Cys Trp Cys Asp Asn Gln Glu Val Tyr Val Gln Tyr Gly Arg Cys
        195                 200                 205

Thr Arg Thr Arg His Ser Lys Arg Ser Arg Arg Ser Val Ser Val Gln
    210                 215                 220

Thr His Gly Glu Ser Ser Leu Val Asn Lys Lys Glu Ala Trp Leu Asp
225                 230                 235                 240

Ser Thr Lys Ala Thr Arg Tyr Leu Met Lys Thr Glu Asn Trp Ile Ile
                245                 250                 255

Arg Asn Pro Gly Tyr Ala Phe Leu Ala Ala Val Leu Gly Trp Met Leu
            260                 265                 270

Gly Ser Asn Asn Gly Gln Arg Val Val Phe Thr Ile Leu Leu Leu Leu
        275                 280                 285

Val Ala Pro Ala Tyr Ser Phe Asn Cys Leu Gly Met Gly Asn Arg Asp
    290                 295                 300

Phe Ile Glu Gly Ala Ser Gly Ala Thr Trp Val Asp Leu Val Leu Glu
305                 310                 315                 320

Gly Asp Ser Cys Leu Thr Ile Met Ala Asn Asp Lys Pro Thr Leu Asp
                325                 330                 335

Val Arg Met Ile Asn Ile Glu Ala Ser Gln Leu Ala Glu Val Arg Ser
            340                 345                 350

Tyr Cys Tyr His Ala Ser Val Thr Asp Ile Ser Thr Val Ala Arg Cys
        355                 360                 365

Pro Thr Thr Gly Glu Ala His Asn Glu Lys Arg Ala Asp Ser Ser Tyr
    370                 375                 380

Val Cys Lys Gln Gly Phe Thr Asp Arg Gly Trp Gly Asn Gly Cys Gly
385                 390                 395                 400

Leu Phe Gly Lys Gly Ser Ile Asp Thr Cys Ala Lys Phe Ser Cys Thr
                405                 410                 415

Ser Lys Ala Ile Gly Arg Thr Ile Gln Pro Glu Asn Ile Lys Tyr Glu
            420                 425                 430

Val Gly Ile Phe Val His Gly Thr Thr Thr Ser Glu Asn His Gly Asn
        435                 440                 445

Tyr Ser Ala Gln Val Gly Ala Ser Gln Ala Ala Lys Phe Thr Val Thr
    450                 455                 460

Pro Asn Ala Pro Ser Ile Thr Leu Lys Leu Gly Asp Tyr Gly Glu Val
465                 470                 475                 480

Thr Leu Asp Cys Glu Pro Arg Ser Gly Leu Asn Thr Glu Ala Phe Tyr
                485                 490                 495

Val Met Thr Val Gly Ser Lys Ser Phe Leu Val His Arg Glu Trp Phe
            500                 505                 510

His Asp Leu Ala Leu Pro Trp Thr Ser Pro Ser Ser Thr Ala Trp Arg
        515                 520                 525

Asn Arg Glu Leu Leu Met Glu Phe Glu Gly Ala His Ala Thr Lys Gln
    530                 535                 540

Ser Val Val Ala Leu Gly Ser Gln Glu Gly Gly Leu His Gln Ala Leu
545                 550                 555                 560

Ala Gly Ala Ile Val Val Glu Tyr Ser Ser Ser Val Lys Leu Thr Ser
                565                 570                 575

Gly His Leu Lys Cys Arg Leu Lys Met Asp Lys Leu Ala Leu Lys Gly
            580                 585                 590

Thr Thr Tyr Gly Met Cys Thr Glu Lys Phe Ser Phe Ala Lys Asn Pro
        595                 600                 605

Val Asp Thr Gly His Gly Thr Val Val Ile Glu Leu Ser Tyr Ser Gly
    610                 615                 620

Ser Asp Gly Pro Cys Lys Ile Pro Ile Val Ser Val Ala Ser Leu Asn
625                 630                 635                 640

Asp Met Thr Pro Val Gly Arg Leu Val Thr Val Asn Pro Phe Val Ala
                645                 650                 655

Thr Ser Ser Ala Asn Ser Lys Val Leu Val Glu Met Glu Pro Pro Phe
        660                 665                 670

Gly Asp Ser Tyr Ile Val Val Gly Arg Gly Asp Lys Gln Ile Asn His
    675                 680                 685

His Trp His Lys Ala Gly Ser Thr Leu Gly Lys Ala Phe Ser Thr Thr
    690                 695                 700

Leu Lys Gly Ala Gln Arg Leu Ala Ala Leu Gly Asp Thr Ala Trp Asp
705                 710                 715                 720

Phe Gly Ser Ile Gly Gly Val Phe Asn Ser Ile Gly Arg Ala Val His
                725                 730                 735

Gln Val Phe Gly Gly Ala Phe Arg Thr Leu Phe Gly Gly Met Ser Trp
        740                 745                 750

Ile Thr Gln Gly Leu Met Gly Ala Leu Leu Leu Trp Met Gly Val Asn
    755                 760                 765

Ala Arg Asp Arg Ser Ile Ala Leu Ala Phe Leu Ala Thr Gly Gly Val
    770                 775                 780

Leu Val Phe Leu Ala Thr Asn Val His Ala Asp Thr Gly Cys Ala Ile
785                 790                 795                 800

Asp Ile Thr Arg Lys Glu Met Arg Cys Gly Ser Gly Ile Phe Val His
                805                 810                 815

Asn Asp Val Glu Ala Trp Val Asp Arg Tyr Lys Tyr Leu Pro Glu Thr
        820                 825                 830

Pro Arg Ser Leu Ala Lys Ile Val His Lys Ala His Lys Glu Gly Val
    835                 840                 845

Cys Gly Val Arg Ser Val Thr Arg Leu Glu His Gln Met Trp Glu Ala
    850                 855                 860

Val Arg Asp Glu Leu Asn Val Leu Leu Lys Glu Asn Ala Val Asp Leu
865                 870                 875                 880

Ser Val Val Val Asn Lys Pro Val Gly Arg Tyr Arg Ser Ala Pro Lys
                885                 890                 895

Arg Leu Ser Met Thr Gln Glu Lys Phe Glu Met Gly Trp Lys Ala Trp
        900                 905                 910

Gly Lys Ser Ile Leu Phe Ala Pro Glu Leu Ala Asn Ser Thr Phe Val
        915                 920                 925

Val Asp Gly Pro Glu Thr Lys Glu Cys Pro Asp Glu His Arg Ala Trp
    930                 935                 940

Asn Ser Met Gln Ile Glu Asp Phe Gly Phe Gly Ile Thr Ser Thr Arg
945                 950                 955                 960

Val Trp Leu Lys Ile Arg Glu Glu Ser Thr Asp Glu Cys Asp Gly Ala
                965                 970                 975

Ile Ile Gly Thr Ala Val Lys Gly His Val Ala Val His Ser Asp Leu
            980                 985                 990

Ser Tyr Trp Ile Glu Ser Arg Tyr Asn Asp Thr Trp Lys Leu Glu Arg
        995                 1000                1005

Ala Val Phe Gly Glu Val Lys Ser Cys Thr Trp Pro Glu Thr His
    1010                1015                1020

Thr Leu Trp Gly Asp Asp Val Glu Glu Ser Glu Leu Ile Ile Pro
    1025                1030                1035

His Thr Ile Ala Gly Pro Lys Ser Lys His Asn Arg Arg Glu Gly
    1040                1045                1050

Tyr Lys Thr Gln Asn Gln Gly Pro Trp Asp Glu Asn Gly Ile Val
    1055                1060                1065

Leu Asp Phe Asp Tyr Cys Pro Gly Thr Lys Val Thr Ile Thr Glu
    1070                1075                1080

Asp Cys Ser Lys Arg Gly Pro Ser Val Arg Thr Thr Thr Asp Ser
    1085                1090                1095

Gly Lys Leu Ile Thr Asp Trp Cys Cys Arg Ser Cys Ser Leu Pro
    1100                1105                1110

Pro Leu Arg Phe Arg Thr Glu Asn Gly Cys Trp Tyr Gly Met Glu
    1115                1120                1125

Ile Arg Pro Val Met His Asp Glu Thr Thr Leu Val Arg Ser Gln
    1130                1135                1140

Val Asp Ala Phe Lys Gly Glu Met Val Asp Pro Phe Gln Leu Gly

1145                1150                1155

Leu Leu Val Met Phe Leu Ala Thr Gln Glu Val Leu Arg Lys Arg
    1160                1165                1170

Trp Thr Ala Arg Leu Thr Ile Pro Ala Val Leu Gly Val Leu Leu
    1175                1180                1185

Val Leu Met Leu Gly Gly Ile Thr Tyr Thr Asp Leu Ala Arg Tyr
    1190                1195                1200

Val Val Leu Val Ala Ala Ala Phe Ala Glu Ala Asn Ser Gly Gly
    1205                1210                1215

Asp Val Leu His Leu Ala Leu Ile Ala Val Phe Lys Ile Gln Pro
    1220                1225                1230

Ala Phe Leu Val Met Asn Met Leu Ser Thr Arg Trp Thr Asn Gln
    1235                1240                1245

Glu Asn Val Ile Leu Val Leu Gly Ala Ala Phe Phe Gln Leu Ala
    1250                1255                1260

Ser Val Asp Leu Gln Ile Gly Val His Gly Ile Leu Asn Ala Ala
    1265                1270                1275

Ala Ile Ala Trp Met Ile Val Arg Ala Ile Thr Phe Pro Thr Thr
    1280                1285                1290

Ser Ser Val Thr Met Pro Val Leu Ala Leu Leu Thr Pro Gly Met
    1295                1300                1305

Arg Ala Leu Tyr Leu Asp Thr Tyr Arg Ile Ile Leu Leu Val Ile
    1310                1315                1320

Gly Ile Cys Ser Leu Leu His Glu Arg Lys Lys Thr Met Ala Lys
    1325                1330                1335

Lys Lys Gly Ala Val Leu Leu Gly Leu Ala Leu Thr Ser Thr Gly
    1340                1345                1350

Trp Phe Ser Pro Thr Thr Ile Ala Ala Gly Leu Met Val Cys Asn
    1355                1360                1365

Pro Asn Lys Lys Arg Gly Trp Pro Ala Thr Glu Phe Leu Ser Ala
    1370                1375                1380

Val Gly Leu Met Phe Ala Ile Val Gly Gly Leu Ala Glu Leu Asp
    1385                1390                1395

Ile Glu Ser Met Ser Ile Pro Phe Met Leu Ala Gly Leu Met Ala
    1400                1405                1410

Val Ser Tyr Val Val Ser Gly Lys Ala Thr Asp Met Trp Leu Glu
    1415                1420                1425

Arg Ala Ala Asp Ile Ser Trp Glu Met Asp Ala Ala Ile Thr Gly
    1430                1435                1440

Ser Ser Arg Arg Leu Asp Val Lys Leu Asp Asp Asp Gly Asp Phe
    1445                1450                1455

His Leu Ile Asp Asp Pro Gly Val Pro Trp Lys Val Trp Val Leu
    1460                1465                1470

Arg Met Ser Cys Ile Gly Leu Ala Ala Leu Thr Pro Trp Ala Ile
    1475                1480                1485

Val Pro Ala Ala Phe Gly Tyr Trp Leu Thr Leu Lys Thr Thr Lys
    1490                1495                1500

Arg Gly Gly Val Phe Trp Asp Thr Pro Ser Pro Lys Pro Cys Ser
    1505                1510                1515

Lys Gly Asp Thr Thr Thr Gly Val Tyr Arg Ile Met Ala Arg Gly
    1520                1525                1530

Ile Leu Gly Thr Tyr Gln Ala Gly Val Gly Val Met Tyr Glu Asn
    1535                1540                1545

Val Phe His Thr Leu Trp His Thr Thr Arg Gly Ala Ala Ile Met
    1550                1555                1560

Ser Gly Glu Gly Lys Leu Thr Pro Tyr Trp Gly Ser Val Arg Glu
    1565                1570                1575

Asp Arg Ile Ala Tyr Gly Gly Pro Trp Arg Phe Asp Arg Lys Trp
    1580                1585                1590

Asn Gly Thr Asp Asp Val Gln Val Ile Val Val Glu Pro Gly Lys
    1595                1600                1605

Ala Ala Val Asn Ile Gln Thr Lys Pro Gly Val Phe Arg Thr Pro
    1610                1615                1620

Phe Gly Glu Val Gly Ala Val Ser Leu Asp Tyr Pro Arg Gly Thr
    1625                1630                1635

Ser Gly Ser Pro Ile Leu Asp Ser Asn Gly Asp Ile Ile Gly Leu
    1640                1645                1650

Tyr Gly Asn Gly Val Glu Leu Gly Asp Gly Ser Tyr Val Ser Ala
    1655                1660                1665

Ile Val Gln Gly Asp Arg Gln Glu Glu Pro Val Pro Glu Ala Tyr
    1670                1675                1680

Thr Pro Asn Met Leu Arg Lys Arg Gln Met Thr Val Leu Asp Leu
    1685                1690                1695

His Pro Gly Ser Gly Lys Thr Arg Lys Ile Leu Pro Gln Ile Ile
    1700                1705                1710

Lys Asp Ala Ile Gln Gln Arg Leu Arg Thr Ala Val Leu Ala Pro
    1715                1720                1725

Thr Arg Val Val Ala Ala Glu Met Ala Glu Ala Leu Arg Gly Leu
    1730                1735                1740

Pro Val Arg Tyr Gln Thr Ser Ala Val Gln Arg Glu His Gln Gly
    1745                1750                1755

Asn Glu Ile Val Asp Val Met Cys His Ala Thr Leu Thr His Arg
    1760                1765                1770

Leu Met Ser Pro Asn Arg Val Pro Asn Tyr Asn Leu Phe Val Met
    1775                1780                1785

Asp Glu Ala His Phe Thr Asp Pro Ala Ser Ile Ala Ala Arg Gly
    1790                1795                1800

Tyr Ile Ala Thr Lys Val Glu Leu Gly Glu Ala Ala Ala Ile Phe
    1805                1810                1815

Met Thr Ala Thr Pro Pro Gly Thr Thr Asp Pro Phe Pro Asp Ser
    1820                1825                1830

Asn Ala Pro Ile His Asp Leu Gln Asp Glu Ile Pro Asp Arg Ala
    1835                1840                1845

Trp Ser Ser Gly Tyr Glu Trp Ile Thr Glu Tyr Ala Gly Lys Thr
    1850                1855                1860

Val Trp Phe Val Ala Ser Val Lys Met Gly Asn Glu Ile Ala Met
    1865                1870                1875

Cys Leu Gln Arg Ala Gly Lys Lys Val Ile Gln Leu Asn Arg Lys
    1880                1885                1890

Ser Tyr Asp Thr Glu Tyr Pro Lys Cys Lys Asn Gly Asp Trp Asp
    1895                1900                1905

Phe Val Ile Thr Thr Asp Ile Ser Glu Met Gly Ala Asn Phe Gly
    1910                1915                1920

Ala Ser Arg Val Ile Asp Cys Arg Lys Ser Val Lys Pro Thr Ile
    1925                1930                1935

Leu Glu Glu Gly Glu Gly Arg Val Ile Leu Gly Asn Pro Ser Pro
    1940                1945                1950

Ile Thr Ser Ala Ser Ala Ala Gln Arg Arg Gly Arg Val Gly Arg
    1955                1960                1965

Asn Pro Asn Gln Val Gly Asp Glu Tyr His Tyr Gly Gly Ala Thr
    1970                1975                1980

Ser Glu Asp Asp Ser Asn Leu Ala His Trp Thr Glu Ala Lys Ile
    1985                1990                1995

Met Leu Asp Asn Ile His Met Pro Asn Gly Leu Val Ala Gln Leu
    2000                2005                2010

Tyr Gly Pro Glu Arg Glu Lys Ala Phe Thr Met Asp Gly Glu Tyr
    2015                2020                2025

Arg Leu Arg Gly Glu Glu Lys Lys Asn Phe Leu Glu Leu Leu Arg
    2030                2035                2040

Thr Ala Asp Leu Pro Val Trp Leu Ala Tyr Lys Val Ala Ser Asn
    2045                2050                2055

Gly Ile Gln Tyr Thr Asp Arg Lys Trp Cys Phe Asp Gly Pro Arg
    2060                2065                2070

Thr Asn Ala Ile Leu Glu Asp Asn Thr Glu Val Glu Ile Val Thr
    2075                2080                2085

Arg Met Gly Glu Arg Lys Ile Leu Lys Pro Arg Trp Leu Asp Ala

2090                2095                2100

Arg Val Tyr Ala Asp His Gln Ala Leu Lys Trp Phe Lys Asp Phe
    2105                2110                2115

Ala Ala Gly Lys Arg Ser Ala Val Ser Phe Ile Glu Val Leu Gly
    2120                2125                2130

Arg Met Pro Glu His Phe Met Gly Lys Thr Arg Glu Ala Leu Asp
    2135                2140                2145

Thr Met Tyr Leu Val Ala Thr Ala Glu Lys Gly Gly Lys Ala His
    2150                2155                2160

Arg Met Ala Leu Glu Glu Leu Pro Asp Ala Leu Glu Thr Ile Thr
    2165                2170                2175

Leu Ile Val Ala Ile Thr Val Met Thr Gly Gly Phe Phe Leu Leu
    2180                2185                2190

Met Met Gln Arg Lys Gly Ile Gly Lys Met Gly Leu Gly Ala Leu
    2195                2200                2205

Val Leu Thr Leu Ala Thr Phe Phe Leu Trp Ala Ala Glu Val Pro
    2210                2215                2220

Gly Thr Lys Ile Ala Gly Thr Leu Leu Ile Ala Leu Leu Leu Met
    2225                2230                2235

Val Val Leu Ile Pro Glu Pro Glu Lys Gln Arg Ser Gln Thr Asp
    2240                2245                2250

Asn Gln Leu Ala Val Phe Leu Ile Cys Val Leu Thr Val Val Gly
    2255                2260                2265

Val Val Ala Ala Asn Glu Tyr Gly Met Leu Glu Lys Thr Lys Ala
    2270                2275                2280

Asp Leu Lys Ser Met Phe Gly Gly Lys Thr Gln Ala Ser Gly Leu
    2285                2290                2295

Thr Gly Leu Pro Ser Met Ala Leu Asp Leu Arg Pro Ala Thr Ala
    2300                2305                2310

Trp Ala Leu Tyr Gly Gly Ser Thr Val Val Leu Thr Pro Leu Leu
    2315                2320                2325

Lys His Leu Ile Thr Ser Glu Tyr Val Thr Thr Ser Leu Ala Ser
    2330                2335                2340

Ile Asn Ser Gln Ala Gly Ser Leu Phe Val Leu Pro Arg Gly Val
    2345                2350                2355

Pro Phe Thr Asp Leu Asp Leu Thr Val Gly Leu Val Phe Leu Gly
    2360                2365                2370

Cys Trp Gly Gln Ile Thr Leu Thr Thr Phe Leu Thr Ala Met Val
    2375                2380                2385

Leu Ala Thr Leu His Tyr Gly Tyr Met Leu Pro Gly Trp Gln Ala
    2390                2395                2400

Glu Ala Leu Arg Ala Ala Gln Arg Arg Thr Ala Ala Gly Ile Met
    2405                2410                2415

Lys Asn Ala Val Val Asp Gly Met Val Ala Thr Asp Val Pro Glu
    2420                2425                2430

Leu Glu Arg Thr Thr Pro Leu Met Gln Lys Lys Val Gly Gln Val
    2435                2440                2445

Leu Leu Ile Gly Val Ser Val Ala Ala Phe Leu Val Asn Pro Asn
    2450                2455                2460

Val Thr Thr Val Arg Glu Ala Gly Val Leu Val Thr Ala Ala Thr
    2465                2470                2475

Leu Thr Leu Trp Asp Asn Gly Ala Ser Ala Val Trp Asn Ser Thr
    2480                2485                2490

Thr Ala Thr Gly Leu Cys His Val Met Arg Gly Ser Tyr Leu Ala
    2495                2500                2505

Gly Gly Ser Ile Ala Trp Thr Leu Ile Lys Asn Ala Asp Lys Pro
    2510                2515                2520

Ser Leu Lys Arg Gly Arg Pro Gly Gly Arg Thr Leu Gly Glu Gln
    2525                2530                2535

Trp Lys Glu Lys Leu Asn Ala Met Ser Arg Glu Glu Phe Phe Lys
    2540                2545                2550

Tyr Arg Arg Glu Ala Ile Ile Glu Val Asp Arg Thr Glu Ala Arg
    2555                2560                2565

Arg Ala Arg Arg Glu Asn Asn Ile Val Gly Gly His Pro Val Ser
    2570                2575                2580

Arg Gly Ser Ala Lys Leu Arg Trp Leu Val Glu Lys Gly Phe Val
    2585                2590                2595

Ser Pro Ile Gly Lys Val Ile Asp Leu Gly Cys Gly Arg Gly Gly
    2600                2605                2610

Trp Ser Tyr Tyr Ala Ala Thr Leu Lys Lys Val Gln Glu Val Arg
    2615                2620                2625

Gly Tyr Thr Lys Gly Gly Ala Gly His Glu Glu Pro Met Leu Met
    2630                2635                2640

Gln Ser Tyr Gly Trp Asn Leu Val Ser Leu Lys Ser Gly Val Asp
    2645                2650                2655

Val Phe Tyr Lys Pro Ser Glu Pro Ser Asp Thr Leu Phe Cys Asp
    2660                2665                2670

Ile Gly Glu Ser Ser Pro Ser Pro Glu Val Glu Glu Gln Arg Thr
    2675                2680                2685

Leu Arg Val Leu Glu Met Thr Ser Asp Trp Leu His Arg Gly Pro
    2690                2695                2700

Arg Glu Phe Cys Ile Lys Val Leu Cys Pro Tyr Met Pro Lys Val
    2705                2710                2715

Ile Glu Lys Met Glu Val Leu Gln Arg Arg Phe Gly Gly Gly Leu
    2720                2725                2730

Val Arg Leu Pro Leu Ser Arg Asn Ser Asn His Glu Met Tyr Trp
    2735                2740                2745

Val Ser Gly Ala Ala Gly Asn Val Val His Ala Val Asn Met Thr
    2750                2755                2760

Ser Gln Val Leu Leu Gly Arg Met Asp Arg Thr Val Trp Arg Gly
    2765                2770                2775

Pro Lys Tyr Glu Glu Asp Val Asn Leu Gly Ser Gly Thr Arg Ala
    2780                2785                2790

Val Gly Lys Gly Glu Val His Ser Asn Gln Glu Lys Ile Lys Lys
    2795                2800                2805

Arg Ile Gln Lys Leu Lys Glu Glu Phe Ala Thr Thr Trp His Lys
    2810                2815                2820

Asp Pro Glu His Pro Tyr Arg Thr Trp Thr Tyr His Gly Ser Tyr
    2825                2830                2835

Glu Val Lys Ala Thr Gly Ser Ala Ser Ser Leu Val Asn Gly Val
    2840                2845                2850

Val Glu Leu Met Ser Lys Pro Trp Asp Ala Ile Ala Asn Val Thr
    2855                2860                2865

Thr Met Ala Met Thr Asp Thr Thr Pro Phe Gly Gln Gln Arg Val
    2870                2875                2880

Phe Lys Glu Lys Val Asp Thr Lys Ala Pro Glu Pro Pro Ala Gly
    2885                2890                2895

Ala Lys Glu Val Leu Asn Glu Thr Thr Asn Trp Leu Trp Ala His
    2900                2905                2910

Leu Ser Arg Glu Lys Arg Pro Arg Leu Cys Thr Lys Glu Glu Phe
    2915                2920                2925

Ile Lys Lys Val Asn Ser Asn Ala Ala Leu Gly Ala Val Phe Ala
    2930                2935                2940

Glu Gln Asn Gln Trp Ser Thr Ala Arg Glu Ala Val Asp Asp Pro
    2945                2950                2955

Arg Phe Trp Glu Met Val Asp Glu Glu Arg Glu Asn His Leu Arg
    2960                2965                2970

Gly Glu Cys His Thr Cys Ile Tyr Asn Met Met Gly Lys Arg Glu
    2975                2980                2985

Lys Lys Pro Gly Glu Phe Gly Lys Ala Lys Gly Ser Arg Ala Ile
    2990                2995                3000

Trp Phe Met Trp Leu Gly Ala Arg Tyr Leu Glu Phe Glu Ala Leu
    3005                3010                3015

Gly Phe Leu Asn Glu Asp His Trp Leu Ser Arg Glu Asn Ser Gly
    3020                3025                3030

Gly Gly Val Glu Gly Ser Gly Val Gln Lys Leu Gly Tyr Ile Leu

3035                3040                3045

Arg Asp Ile Ala Gly Lys Gln Gly Gly Lys Met Tyr Ala Asp Asp
    3050                3055                3060

Thr Ala Gly Trp Asp Thr Arg Ile Thr Arg Thr Asp Leu Glu Asn
    3065                3070                3075

Glu Ala Lys Val Leu Glu Leu Leu Asp Gly Glu His Arg Met Leu
    3080                3085                3090

Ala Arg Ala Ile Ile Glu Leu Thr Tyr Arg His Lys Val Val Lys
    3095                3100                3105

Val Met Arg Pro Ala Ala Glu Gly Lys Thr Val Met Asp Val Ile
    3110                3115                3120

Ser Arg Glu Asp Gln Arg Gly Ser Gly Gln Val Val Thr Tyr Ala
    3125                3130                3135

Leu Asn Thr Phe Thr Asn Ile Ala Val Gln Leu Val Arg Leu Met
    3140                3145                3150

Glu Ala Glu Gly Val Ile Gly Pro Gln His Leu Glu Gln Leu Pro
    3155                3160                3165

Arg Lys Thr Lys Ile Ala Val Arg Thr Trp Leu Phe Glu Asn Gly
    3170                3175                3180

Glu Glu Arg Val Thr Arg Met Ala Ile Ser Gly Asp Asp Cys Val
    3185                3190                3195

Val Lys Pro Leu Asp Asp Arg Phe Ala Thr Ala Leu His Phe Leu
    3200                3205                3210

Asn Ala Met Ser Lys Val Arg Lys Asp Ile Gln Glu Trp Lys Pro
    3215                3220                3225

Ser His Gly Trp His Asp Trp Gln Gln Val Pro Phe Cys Ser Asn
    3230                3235                3240

His Phe Gln Glu Ile Val Met Lys Asp Gly Arg Ser Ile Val Val
    3245                3250                3255

Pro Cys Arg Gly Gln Asp Glu Leu Ile Gly Arg Ala Arg Ile Ser
    3260                3265                3270

Pro Gly Ala Gly Trp Asn Val Lys Asp Thr Ala Cys Leu Ala Lys
    3275                3280                3285

Ala Tyr Ala Gln Met Trp Leu Leu Leu Tyr Phe His Arg Arg Asp
    3290                3295                3300

Leu Arg Leu Met Ala Asn Ala Ile Cys Ser Ala Val Pro Val Asp
    3305                3310                3315

Trp Val Pro Thr Gly Arg Thr Ser Trp Ser Ile His Ser Lys Gly
    3320                3325                3330

Glu Trp Met Thr Thr Glu Asp Met Leu Gln Val Trp Asn Arg Val
    3335                3340                3345

Trp Ile Glu Glu Asn Glu Trp Met Met Asp Lys Thr Pro Ile Thr
    3350                3355                3360

Ser Trp Thr Asp Val Pro Tyr Val Gly Lys Arg Glu Asp Ile Trp
    3365                3370                3375

Cys Gly Ser Leu Ile Gly Thr Arg Ser Arg Ala Thr Trp Ala Glu
    3380                3385                3390

Asn Ile Tyr Ala Ala Ile Asn Gln Val Arg Ala Val Ile Gly Lys
    3395                3400                3405

Glu Asn Tyr Val Asp Tyr Met Thr Ser Leu Arg Arg Tyr Glu Asp
    3410                3415                3420

Val Leu Ile Gln Glu Asp Arg Val Ile
    3425                3430

<210>  30
<211>  3411
<212>  PRT
<213>  Yellow fever virus

<400>  30

Met Ser Gly Arg Lys Ala Gln Gly Lys Thr Leu Gly Val Asn Met Val
1               5                   10                  15

Arg Arg Gly Val Arg Ser Leu Ser Asn Lys Ile Lys Gln Lys Thr Lys
            20                  25                  30

Gln Ile Gly Asn Arg Pro Gly Pro Ser Arg Gly Val Gln Gly Phe Ile
        35                  40                  45

Phe Phe Phe Leu Phe Asn Ile Leu Thr Gly Lys Lys Ile Thr Ala His
    50                  55                  60

Leu Lys Arg Leu Trp Lys Met Leu Asp Pro Arg Gln Gly Leu Ala Val
65                  70                  75                  80

Leu Arg Lys Val Lys Arg Val Val Ala Ser Leu Met Arg Gly Leu Ser
                85                  90                  95

Ser Arg Lys Arg Arg Ser His Asp Val Leu Thr Val Gln Phe Leu Ile
            100                 105                 110

Leu Gly Met Leu Leu Met Thr Gly Gly Val Thr Leu Val Arg Lys Asn
        115                 120                 125

Arg Trp Leu Leu Leu Asn Val Thr Ser Glu Asp Leu Gly Lys Thr Phe
    130                 135                 140

Ser Val Gly Thr Gly Asn Cys Thr Thr Asn Ile Leu Glu Ala Lys Tyr
145                 150                 155                 160

Trp Cys Pro Asp Ser Met Glu Tyr Asn Cys Pro Asn Leu Ser Pro Arg
                165                 170                 175

Glu Glu Pro Asp Asp Ile Asp Cys Trp Cys Tyr Gly Val Glu Asn Val
            180                 185                 190

Arg Val Ala Tyr Gly Lys Cys Asp Ser Ala Gly Arg Ser Arg Arg Ser
        195                 200                 205

Arg Arg Ala Ile Asp Leu Pro Thr His Glu Asn His Gly Leu Lys Thr
    210                 215                 220

Arg Gln Glu Lys Trp Met Thr Gly Arg Met Gly Glu Arg Gln Leu Gln
225                 230                 235                 240

Lys Ile Glu Arg Trp Phe Val Arg Asn Pro Phe Phe Ala Val Thr Ala
                245                 250                 255

Leu Thr Ile Ala Tyr Leu Val Gly Ser Asn Met Thr Gln Arg Val Val
            260                 265                 270

Ile Ala Leu Leu Val Leu Ala Val Gly Pro Ala Tyr Ser Ala His Cys
        275                 280                 285

Ile Gly Ile Thr Asp Arg Asp Phe Ile Glu Gly Val His Gly Gly Thr
    290                 295                 300

Trp Val Ser Ala Thr Leu Glu Gln Asp Lys Cys Val Thr Val Met Ala
305                 310                 315                 320

Pro Asp Lys Pro Ser Leu Asp Ile Ser Leu Glu Thr Val Ala Ile Asp
                325                 330                 335

Arg Pro Ala Glu Val Arg Lys Val Cys Tyr Asn Ala Val Leu Thr His
            340                 345                 350

Val Lys Ile Asn Asp Lys Cys Pro Ser Thr Gly Glu Ala His Leu Ala
        355                 360                 365

Glu Glu Asn Glu Gly Asp Asn Ala Cys Lys Arg Thr Tyr Ser Asp Arg
    370                 375                 380

Gly Trp Gly Asn Gly Cys Gly Leu Phe Gly Lys Gly Ser Ile Val Ala
385                 390                 395                 400

Cys Ala Lys Phe Thr Cys Ala Lys Ser Met Ser Leu Phe Glu Val Asp
                405                 410                 415

Gln Thr Lys Ile Gln Tyr Val Ile Arg Ala Gln Leu His Val Gly Ala
            420                 425                 430

Lys Gln Glu Asn Trp Thr Thr Asp Ile Lys Thr Leu Lys Phe Asp Ala
        435                 440                 445

Leu Ser Gly Ser Gln Glu Val Glu Phe Ile Gly Tyr Gly Lys Ala Thr
    450                 455                 460

Leu Glu Cys Gln Val Gln Thr Ala Val Asp Phe Gly Asn Ser Tyr Ile
465                 470                 475                 480

Ala Glu Met Glu Thr Glu Ser Trp Ile Val Asp Arg Gln Trp Ala Gln
                485                 490                 495

Asp Leu Thr Leu Pro Trp Gln Ser Gly Ser Gly Gly Val Trp Arg Glu
            500                 505                 510

Met His His Leu Val Glu Phe Glu Pro Pro His Ala Ala Thr Ile Arg
        515                 520                 525

Val Leu Ala Leu Gly Asn Gln Glu Gly Ser Leu Lys Thr Ala Leu Thr
    530                 535                 540

Gly Ala Met Arg Val Thr Lys Asp Thr Asn Asp Asn Asn Leu Tyr Lys
545                 550                 555                 560

Leu His Gly Gly His Val Ser Cys Arg Val Lys Leu Ser Ala Leu Thr
                565                 570                 575

Leu Lys Gly Thr Ser Tyr Lys Ile Cys Thr Asp Lys Met Phe Phe Val
            580                 585                 590

Lys Asn Pro Thr Asp Thr Gly His Gly Thr Val Val Met Gln Val Lys
        595                 600                 605

Val Ser Lys Gly Ala Pro Cys Arg Ile Pro Val Ile Val Ala Asp Asp
    610                 615                 620

Leu Thr Ala Ala Ile Asn Lys Gly Ile Leu Val Thr Val Asn Pro Ile
625                 630                 635                 640

Ala Ser Thr Asn Asp Asp Glu Val Leu Ile Glu Val Asn Pro Pro Phe
                645                 650                 655

Gly Asp Ser Tyr Ile Ile Val Gly Arg Gly Asp Ser Arg Leu Thr Tyr
            660                 665                 670

Gln Trp His Lys Glu Gly Ser Ser Ile Gly Lys Leu Phe Thr Gln Thr
        675                 680                 685

Met Lys Gly Val Glu Arg Leu Ala Val Met Gly Asp Thr Ala Trp Asp
    690                 695                 700

Phe Ser Ser Ala Gly Gly Phe Phe Thr Ser Val Gly Lys Gly Ile His
705                 710                 715                 720

Thr Val Phe Gly Ser Ala Phe Gln Gly Leu Phe Gly Gly Leu Asn Trp
                725                 730                 735

Ile Thr Lys Val Ile Met Gly Ala Val Leu Ile Trp Val Gly Ile Asn
            740                 745                 750

Thr Arg Asn Met Thr Met Ser Met Ser Met Ile Leu Val Gly Val Ile
        755                 760                 765

Met Met Phe Leu Ser Leu Gly Val Gly Ala Asp Gln Gly Cys Ala Ile
    770                 775                 780

Asn Phe Gly Lys Arg Glu Leu Lys Cys Gly Asp Gly Ile Phe Ile Phe
785                 790                 795                 800

Arg Asp Ser Asp Asp Trp Leu Asn Lys Tyr Ser Tyr Tyr Pro Glu Asp

                805                 810                 815

Pro Val Lys Leu Ala Ser Ile Val Lys Ala Ser Phe Glu Glu Gly Lys
            820                 825                 830

Cys Gly Leu Asn Ser Val Asp Ser Leu Glu His Glu Met Trp Arg Ser
        835                 840                 845

Arg Ala Asp Glu Ile Asn Ala Ile Phe Glu Glu Asn Glu Val Asp Ile
    850                 855                 860

Ser Val Val Val Gln Asp Pro Lys Asn Val Tyr Gln Arg Gly Thr His
865                 870                 875                 880

Pro Phe Ser Arg Ile Arg Asp Gly Leu Gln Tyr Gly Trp Lys Thr Trp
                885                 890                 895

Gly Lys Asn Leu Val Phe Ser Pro Gly Arg Lys Asn Gly Ser Phe Ile
            900                 905                 910

Ile Asp Gly Lys Ser Arg Lys Glu Cys Pro Phe Ser Asn Arg Val Trp
        915                 920                 925

Asn Ser Phe Gln Ile Glu Glu Phe Gly Thr Gly Val Phe Thr Thr Arg
    930                 935                 940

Val Tyr Met Asp Ala Val Phe Glu Tyr Thr Ile Asp Cys Asp Gly Ser
945                 950                 955                 960

Ile Leu Gly Ala Ala Val Asn Gly Lys Lys Ser Ala His Gly Ser Pro
                965                 970                 975

Thr Phe Trp Met Gly Ser His Glu Val Asn Gly Thr Trp Met Ile His
            980                 985                 990

Thr Leu Glu Ala Leu Asp Tyr Lys Glu Cys Glu Trp Pro Leu Thr His
        995                 1000                1005

Thr Ile Gly Thr Ser Val Glu Glu Ser Glu Met Phe Met Pro Arg
    1010                1015                1020

Ser Ile Gly Gly Pro Val Ser Ser His Asn His Ile Pro Gly Tyr
    1025                1030                1035

Lys Val Gln Thr Asn Gly Pro Trp Met Gln Val Pro Leu Glu Val
    1040                1045                1050

Lys Arg Glu Ala Cys Pro Gly Thr Ser Val Ile Ile Asp Gly Asn
    1055                1060                1065

Cys Asp Gly Arg Gly Lys Ser Thr Arg Ser Thr Thr Asp Ser Gly
    1070                1075                1080

Lys Val Ile Pro Glu Trp Cys Cys Arg Ser Cys Thr Met Pro Pro
    1085                1090                1095

Val Ser Phe His Gly Ser Asp Gly Cys Trp Tyr Pro Met Glu Ile
    1100                1105                1110

Arg Pro Arg Lys Thr His Glu Ser His Leu Val Arg Ser Trp Val
    1115                1120                1125

Thr Ala Gly Glu Ile His Ala Val Pro Phe Gly Leu Val Ser Met
    1130                1135                1140

Met Ile Ala Met Glu Val Val Leu Arg Lys Arg Gln Gly Pro Lys
    1145                1150                1155

Gln Met Leu Val Gly Gly Val Val Leu Leu Gly Ala Met Leu Val
    1160                1165                1170

Gly Gln Val Thr Leu Leu Asp Leu Leu Lys Leu Thr Val Ala Val
    1175                1180                1185

Gly Leu His Phe His Glu Met Asn Asn Gly Gly Asp Ala Met Tyr
    1190                1195                1200

Met Ala Leu Ile Ala Ala Phe Ser Ile Arg Pro Gly Leu Leu Ile
    1205                1210                1215

Gly Phe Gly Leu Arg Thr Leu Trp Ser Pro Arg Glu Arg Leu Val
    1220                1225                1230

Leu Thr Leu Gly Ala Ala Met Val Glu Ile Ala Leu Gly Gly Val
    1235                1240                1245

Met Gly Gly Leu Trp Lys Tyr Leu Asn Ala Val Ser Leu Cys Ile
    1250                1255                1260

Leu Thr Ile Asn Ala Val Ala Ser Arg Lys Ala Ser Asn Thr Ile
    1265                1270                1275

Leu Pro Leu Met Ala Leu Leu Thr Pro Val Thr Met Ala Glu Val
    1280                1285                1290

Arg Leu Ala Ala Met Phe Phe Cys Ala Val Val Ile Ile Gly Val
    1295                1300                1305

Leu His Gln Asn Phe Lys Asp Thr Ser Met Gln Lys Thr Ile Pro
    1310                1315                1320

Leu Val Ala Leu Thr Leu Thr Ser Tyr Leu Gly Leu Thr Gln Pro
    1325                1330                1335

Phe Leu Gly Leu Cys Ala Phe Leu Ala Thr Arg Ile Phe Gly Arg
    1340                1345                1350

Arg Ser Ile Pro Val Asn Glu Ala Leu Ala Ala Ala Gly Leu Val
    1355                1360                1365

Gly Val Leu Ala Gly Leu Ala Phe Gln Glu Met Glu Asn Phe Leu
    1370                1375                1380

Gly Pro Ile Ala Val Gly Gly Leu Leu Met Met Leu Val Ser Val
    1385                1390                1395

Ala Gly Arg Val Asp Gly Leu Glu Leu Lys Lys Leu Gly Glu Val
    1400                1405                1410

Ser Trp Glu Glu Glu Ala Glu Ile Ser Gly Ser Ser Ala Arg Tyr
    1415                1420                1425

Asp Val Ala Leu Ser Glu Gln Gly Glu Phe Lys Leu Leu Ser Glu
    1430                1435                1440

Glu Lys Val Pro Trp Asp Gln Val Val Met Thr Ser Leu Ala Leu
    1445                1450                1455

Val Gly Ala Ala Leu His Pro Phe Ala Leu Leu Leu Val Leu Ala
    1460                1465                1470

Gly Trp Leu Phe His Val Arg Gly Ala Arg Arg Ser Gly Asp Val
    1475                1480                1485

Leu Trp Asp Ile Pro Thr Pro Lys Ile Ile Glu Glu Cys Glu His
    1490                1495                1500

Leu Glu Asp Gly Ile Tyr Gly Ile Phe Gln Ser Thr Phe Leu Gly
    1505                1510                1515

Ala Ser Gln Arg Gly Val Gly Val Ala Gln Gly Gly Val Phe His
    1520                1525                1530

Thr Met Trp His Val Thr Arg Gly Ala Phe Leu Val Arg Asn Gly
    1535                1540                1545

Lys Lys Leu Ile Pro Ser Trp Ala Ser Val Lys Glu Asp Leu Val
    1550                1555                1560

Ala Tyr Gly Gly Ser Trp Lys Leu Glu Gly Arg Trp Asp Gly Glu
    1565                1570                1575

Glu Glu Val Gln Leu Ile Ala Ala Val Pro Gly Lys Asn Val Val
    1580                1585                1590

Asn Val Gln Thr Lys Pro Ser Leu Phe Lys Val Arg Asn Gly Gly
    1595                1600                1605

Glu Ile Gly Ala Val Ala Leu Asp Tyr Pro Ser Gly Thr Ser Gly
    1610                1615                1620

Ser Pro Ile Val Asn Arg Asn Gly Glu Val Ile Gly Leu Tyr Gly
    1625                1630                1635

Asn Gly Ile Leu Val Gly Asp Asn Ser Phe Val Ser Ala Ile Ser
    1640                1645                1650

Gln Thr Glu Val Lys Glu Glu Gly Lys Glu Glu Leu Gln Glu Ile
    1655                1660                1665

Pro Thr Met Leu Lys Lys Gly Met Thr Thr Val Leu Asp Phe His
    1670                1675                1680

Pro Gly Ala Gly Lys Thr Arg Arg Phe Leu Pro Gln Ile Leu Ala
    1685                1690                1695

Glu Cys Ala Arg Arg Arg Leu Arg Thr Leu Val Leu Ala Pro Thr
    1700                1705                1710

Arg Val Val Leu Ser Glu Met Lys Glu Ala Phe His Gly Leu Asp
    1715                1720                1725

Val Lys Phe His Thr Gln Ala Phe Ser Ala His Gly Ser Gly Arg
    1730                1735                1740

Glu Val Ile Asp Ala Met Cys His Ala Thr Leu Thr Tyr Arg Met
    1745                1750                1755

Leu Glu Pro Thr Arg Val Val Asn Trp Glu Val Ile Ile Met Asp

1760                1765                1770

Glu Ala His Phe Leu Asp Pro Ala Ser Ile Ala Ala Arg Gly Trp
    1775                1780                1785

Ala Ala His Arg Ala Arg Ala Asn Glu Ser Ala Thr Ile Leu Met
    1790                1795                1800

Thr Ala Thr Pro Pro Gly Thr Ser Asp Glu Phe Pro His Ser Asn
    1805                1810                1815

Gly Glu Ile Glu Asp Val Gln Thr Asp Ile Pro Ser Glu Pro Trp
    1820                1825                1830

Asn Thr Gly His Asp Trp Ile Leu Ala Asp Lys Arg Pro Thr Ala
    1835                1840                1845

Trp Phe Leu Pro Ser Ile Arg Ala Ala Asn Val Met Ala Ala Ser
    1850                1855                1860

Leu Arg Lys Ala Gly Lys Ser Val Val Val Leu Asn Arg Lys Thr
    1865                1870                1875

Phe Glu Arg Glu Tyr Pro Thr Ile Lys Gln Lys Lys Pro Asp Phe
    1880                1885                1890

Ile Leu Ala Thr Asp Ile Ala Glu Met Gly Ala Asn Leu Cys Val
    1895                1900                1905

Glu Arg Val Leu Asp Cys Arg Thr Ala Phe Lys Pro Val Leu Val
    1910                1915                1920

Asp Glu Gly Arg Lys Val Ala Ile Lys Gly Pro Leu Arg Ile Ser
    1925                1930                1935

Ala Ser Ser Ala Ala Gln Arg Arg Gly Arg Ile Gly Arg Asn Pro
    1940                1945                1950

Asn Arg Asp Gly Asp Ser Tyr Tyr Tyr Ser Glu Pro Thr Ser Glu
    1955                1960                1965

Asn Asn Ala His His Val Cys Trp Leu Glu Ala Ser Met Leu Leu
    1970                1975                1980

Asp Asn Met Glu Val Arg Gly Gly Met Val Ala Pro Leu Tyr Gly
    1985                1990                1995

Val Glu Gly Thr Lys Thr Pro Val Ser Pro Gly Glu Met Arg Leu
    2000                2005                2010

Arg Asp Asp Gln Arg Lys Val Phe Arg Glu Leu Val Arg Asn Cys
    2015                2020                2025

Asp Leu Pro Val Trp Leu Ser Trp Gln Val Ala Lys Ala Gly Leu
    2030                2035                2040

Lys Thr Asn Asp Arg Lys Trp Cys Phe Glu Gly Pro Glu Glu His
    2045                2050                2055

Glu Ile Leu Asn Asp Ser Gly Glu Thr Val Lys Cys Arg Ala Pro
    2060                2065                2070

Gly Gly Ala Lys Lys Pro Leu Arg Pro Arg Trp Cys Asp Glu Arg
    2075                2080                2085

Val Ser Ser Asp Gln Ser Ala Leu Ser Glu Phe Ile Lys Phe Ala
    2090                2095                2100

Glu Gly Arg Arg Gly Ala Ala Glu Val Leu Val Val Leu Ser Glu
    2105                2110                2115

Leu Pro Asp Phe Leu Ala Lys Lys Gly Gly Glu Ala Met Asp Thr
    2120                2125                2130

Ile Ser Val Phe Leu His Ser Glu Glu Gly Ser Arg Ala Tyr Arg
    2135                2140                2145

Asn Ala Leu Ser Met Met Pro Glu Ala Met Thr Ile Val Met Leu
    2150                2155                2160

Phe Ile Leu Ala Gly Leu Leu Thr Ser Gly Met Val Ile Phe Phe
    2165                2170                2175

Met Ser Pro Lys Gly Ile Ser Arg Met Ser Met Ala Met Gly Thr
    2180                2185                2190

Met Ala Gly Cys Gly Tyr Leu Met Phe Leu Gly Gly Val Lys Pro
    2195                2200                2205

Thr His Ile Ser Tyr Ile Met Leu Ile Phe Phe Val Leu Met Val
    2210                2215                2220

Val Val Ile Pro Glu Pro Gly Gln Gln Arg Ser Ile Gln Asp Asn
    2225                2230                2235

Gln Val Ala Tyr Leu Ile Ile Gly Ile Leu Thr Leu Val Ser Ala
    2240                2245                2250

Val Ala Ala Asn Glu Leu Gly Met Leu Glu Lys Thr Lys Glu Asp
    2255                2260                2265

Leu Phe Gly Lys Lys Asn Leu Ile Pro Ser Ser Ala Ser Pro Trp
    2270                2275                2280

Ser Trp Pro Asp Leu Asp Leu Lys Pro Gly Ala Ala Trp Thr Val
    2285                2290                2295

Tyr Val Gly Ile Val Thr Met Leu Ser Pro Met Leu His His Trp
    2300                2305                2310

Ile Lys Val Glu Tyr Gly Asn Leu Ser Leu Ser Gly Ile Ala Gln
    2315                2320                2325

Ser Ala Ser Val Leu Ser Phe Met Asp Lys Gly Ile Pro Phe Met
    2330                2335                2340

Lys Met Asn Ile Ser Val Ile Met Leu Leu Val Ser Gly Trp Asn
    2345                2350                2355

Ser Ile Thr Val Met Pro Leu Leu Cys Gly Ile Gly Cys Ala Met
    2360                2365                2370

Leu His Trp Ser Leu Ile Leu Pro Gly Ile Lys Ala Gln Gln Ser
    2375                2380                2385

Lys Leu Ala Gln Arg Arg Val Phe His Gly Val Ala Lys Asn Pro
    2390                2395                2400

Val Val Asp Gly Asn Pro Thr Val Asp Ile Glu Glu Ala Pro Glu
    2405                2410                2415

Met Pro Ala Leu Tyr Glu Lys Lys Leu Ala Leu Tyr Leu Leu Leu
    2420                2425                2430

Ala Leu Ser Leu Ala Ser Val Ala Met Cys Arg Thr Pro Phe Ser
    2435                2440                2445

Leu Ala Glu Gly Ile Val Leu Ala Ser Ala Ala Leu Gly Pro Leu
    2450                2455                2460

Ile Glu Gly Asn Thr Ser Leu Leu Trp Asn Gly Pro Met Ala Val
    2465                2470                2475

Ser Met Thr Gly Val Met Arg Gly Asn His Tyr Ala Phe Val Gly
    2480                2485                2490

Val Met Tyr Asn Leu Trp Lys Met Lys Thr Gly Arg Arg Gly Ser
    2495                2500                2505

Ala Asn Gly Lys Thr Leu Gly Glu Val Trp Lys Arg Glu Leu Asn
    2510                2515                2520

Leu Leu Asp Lys Arg Gln Phe Glu Leu Tyr Lys Arg Thr Asp Ile
    2525                2530                2535

Val Glu Val Asp Arg Asp Thr Ala Arg Arg His Leu Ala Glu Gly
    2540                2545                2550

Lys Val Asp Thr Gly Val Ala Val Ser Arg Gly Thr Ala Lys Leu
    2555                2560                2565

Arg Trp Phe His Glu Arg Gly Tyr Val Lys Leu Glu Gly Arg Val
    2570                2575                2580

Ile Asp Leu Gly Cys Gly Arg Gly Gly Trp Cys Tyr Tyr Ala Ala
    2585                2590                2595

Ala Gln Lys Glu Val Ser Gly Val Lys Gly Phe Thr Leu Gly Arg
    2600                2605                2610

Asp Gly His Glu Lys Pro Met Asn Val Gln Ser Leu Gly Trp Asn
    2615                2620                2625

Ile Ile Thr Phe Lys Asp Lys Thr Asp Ile His Arg Leu Glu Pro
    2630                2635                2640

Val Lys Cys Asp Thr Leu Leu Cys Asp Ile Gly Glu Ser Ser Ser
    2645                2650                2655

Ser Ser Val Thr Glu Gly Glu Arg Thr Val Arg Val Leu Asp Thr
    2660                2665                2670

Val Glu Lys Trp Leu Ala Cys Gly Val Asp Asn Phe Cys Val Lys
    2675                2680                2685

Val Leu Ala Pro Tyr Met Pro Asp Val Leu Glu Lys Leu Glu Leu
    2690                2695                2700

Leu Gln Arg Arg Phe Gly Gly Thr Val Ile Arg Asn Pro Leu Ser

2705                2710                2715

Arg Asn Ser Thr His Glu Met Tyr Tyr Val Ser Gly Ala Arg Ser
    2720                2725                2730

Asn Val Thr Phe Thr Val Asn Gln Thr Ser Arg Leu Leu Met Arg
    2735                2740                2745

Arg Met Arg Arg Pro Thr Gly Lys Val Thr Leu Glu Ala Asp Val
    2750                2755                2760

Ile Leu Pro Ile Gly Thr Arg Ser Val Glu Thr Asp Lys Gly Pro
    2765                2770                2775

Leu Asp Lys Glu Ala Ile Glu Glu Arg Val Glu Arg Ile Lys Ser
    2780                2785                2790

Glu Tyr Met Thr Ser Trp Phe Tyr Asp Asn Asp Asn Pro Tyr Arg
    2795                2800                2805

Thr Trp His Tyr Cys Gly Ser Tyr Val Thr Lys Thr Ser Gly Ser
    2810                2815                2820

Ala Ala Ser Met Val Asn Gly Val Ile Lys Ile Leu Thr Tyr Pro
    2825                2830                2835

Trp Asp Arg Ile Glu Glu Val Thr Arg Met Ala Met Thr Asp Thr
    2840                2845                2850

Thr Pro Phe Gly Gln Gln Arg Val Phe Lys Glu Lys Val Asp Thr
    2855                2860                2865

Arg Ala Lys Asp Pro Pro Ala Gly Thr Arg Lys Ile Met Lys Val
    2870                2875                2880

Val Asn Arg Trp Leu Phe Arg His Leu Ala Arg Glu Lys Asn Pro
    2885                2890                2895

Arg Leu Cys Thr Lys Glu Glu Phe Ile Ala Lys Val Arg Ser His
    2900                2905                2910

Ala Ala Ile Gly Ala Tyr Leu Glu Glu Gln Glu Gln Trp Lys Thr
    2915                2920                2925

Ala Asn Glu Ala Val Gln Asp Pro Lys Phe Trp Glu Leu Val Asp
    2930                2935                2940

Glu Glu Arg Lys Leu His Gln Gln Gly Arg Cys Arg Thr Cys Val
    2945                2950                2955

Tyr Asn Met Met Gly Lys Arg Glu Lys Lys Leu Ser Glu Phe Gly
    2960                2965                2970

Lys Ala Lys Gly Ser Arg Ala Ile Trp Tyr Met Trp Leu Gly Ala
    2975                2980                2985

Arg Tyr Leu Glu Phe Glu Ala Leu Gly Phe Leu Asn Glu Asp His
    2990                2995                3000

Trp Ala Ser Arg Glu Asn Ser Gly Gly Gly Val Glu Gly Ile Gly
    3005                3010                3015

Leu Gln Tyr Leu Gly Tyr Val Ile Arg Asp Leu Ala Ala Met Asp
    3020                3025                3030

Gly Gly Gly Phe Tyr Ala Asp Asp Thr Ala Gly Trp Asp Thr Arg
    3035                3040                3045

Ile Thr Glu Ala Asp Leu Asp Asp Glu Gln Glu Ile Leu Asn Tyr
    3050                3055                3060

Met Ser Pro His His Lys Lys Leu Ala Gln Ala Val Met Glu Met
    3065                3070                3075

Thr Tyr Lys Asn Lys Val Val Lys Val Leu Arg Pro Ala Pro Gly
    3080                3085                3090

Gly Lys Ala Tyr Met Asp Val Ile Ser Arg Arg Asp Gln Arg Gly
    3095                3100                3105

Ser Gly Gln Val Val Thr Tyr Ala Leu Asn Thr Ile Thr Asn Leu
    3110                3115                3120

Lys Val Gln Leu Ile Arg Met Ala Glu Ala Glu Met Val Ile His
    3125                3130                3135

His Gln His Val Gln Asp Cys Asp Glu Ser Val Leu Thr Arg Leu
    3140                3145                3150

Glu Ala Trp Leu Thr Glu His Gly Cys Asn Arg Leu Lys Arg Met
    3155                3160                3165

Ala Val Ser Gly Asp Asp Cys Val Val Arg Pro Ile Asp Asp Arg
    3170                3175                3180

Phe Gly Leu Ala Leu Ser His Leu Asn Ala Met Ser Lys Val Arg
    3185                3190                3195

Lys Asp Ile Ser Glu Trp Gln Pro Ser Lys Gly Trp Asn Asp Trp
    3200                3205                3210

Glu Asn Val Pro Phe Cys Ser His His Phe His Glu Leu Gln Leu
    3215                3220                3225

Lys Asp Gly Arg Arg Ile Val Val Pro Cys Arg Glu Gln Asp Glu
    3230                3235                3240

Leu Ile Gly Arg Gly Arg Val Ser Pro Gly Asn Gly Trp Met Ile
    3245                3250                3255

Lys Glu Thr Ala Cys Leu Ser Lys Ala Tyr Ala Asn Met Trp Ser
    3260                3265                3270

Leu Met Tyr Phe His Lys Arg Asp Met Arg Leu Leu Ser Leu Ala
    3275                3280                3285

Val Ser Ser Ala Val Pro Thr Ser Trp Val Pro Gln Gly Arg Thr
    3290                3295                3300

Thr Trp Ser Ile His Gly Lys Gly Glu Trp Met Thr Thr Glu Asp
    3305                3310                3315

Met Leu Glu Val Trp Asn Arg Val Trp Ile Thr Asn Asn Pro His
    3320                3325                3330

Met Gln Asp Lys Thr Met Val Lys Lys Trp Arg Asp Val Pro Tyr
    3335                3340                3345

Leu Thr Lys Arg Gln Asp Lys Leu Cys Gly Ser Leu Ile Gly Met
    3350                3355                3360

Thr Asn Arg Ala Thr Trp Ala Ser His Ile His Leu Val Ile His
    3365                3370                3375

Arg Ile Arg Thr Leu Ile Gly Gln Glu Lys Tyr Thr Asp Tyr Leu
    3380                3385                3390

Thr Val Met Asp Arg Tyr Ser Val Asp Ala Asp Leu Gln Leu Gly
    3395                3400                3405

Glu Leu Ile
    3410

<210>  31
<211>  3414
<212>  PRT
<213>  Tick-borne encephalitis virus

<400>  31

Met Ala Gly Lys Ala Ile Leu Lys Gly Lys Gly Gly Gly Pro Pro Arg
1               5                   10                  15

Arg Val Ser Lys Glu Thr Ala Arg Lys Thr Arg Gln Ser Arg Val Gln
            20                  25                  30

Met Pro Asn Gly Leu Val Leu Met Arg Met Leu Gly Ile Leu Trp His
        35                  40                  45

Ala Val Ala Gly Thr Ala Arg Ser Pro Val Leu Lys Ser Phe Trp Asn
    50                  55                  60

Ser Val Pro Leu Lys Gln Ala Met Ala Ala Leu Arg Lys Ile Lys Lys
65                  70                  75                  80

Ala Val Ser Thr Leu Met Val Gly Leu Gln Arg Arg Gly Lys Arg Arg
                85                  90                  95

Ser Ala Ala Asp Trp Thr Ser Trp Leu Leu Val Leu Val Leu Val Gly
            100                 105                 110

Val Thr Leu Ala Ala Thr Val Arg Lys Glu Arg Asp Gly Thr Thr Val
        115                 120                 125

Ile Arg Ala Glu Gly Lys Asp Ala Ala Thr Gln Val Arg Val Glu Asn
    130                 135                 140

Gly Thr Cys Val Ile Leu Ala Thr Asp Met Gly Ser Trp Cys Asp Asp
145                 150                 155                 160

Ser Leu Thr Tyr Glu Cys Val Thr Ile Asp Gln Gly Glu Glu Pro Val
                165                 170                 175

Asp Val Asp Cys Phe Cys Arg Asn Val Asp Gly Val Tyr Leu Glu Tyr
            180                 185                 190

Gly Arg Cys Gly Lys Gln Glu Gly Ser Arg Thr Arg Arg Ser Val Leu
        195                 200                 205

Ile Pro Ser His Ala Gln Gly Asp Leu Thr Gly Arg Gly His Lys Trp
    210                 215                 220

Leu Glu Gly Asp Ser Leu Arg Thr His Leu Thr Arg Val Glu Gly Trp
225                 230                 235                 240

Val Trp Lys Asn Lys Val Leu Thr Leu Ala Val Ile Ala Ile Val Trp
                245                 250                 255

Leu Thr Val Glu Ser Val Val Thr Arg Val Ala Val Val Val Ala Leu
            260                 265                 270

Leu Cys Leu Ala Pro Val Tyr Ala Ser Arg Cys Thr His Leu Glu Asn
        275                 280                 285

Arg Asp Phe Ile Thr Gly Thr Gln Gly Thr Thr Arg Val Thr Leu Val
    290                 295                 300

Leu Glu Leu Gly Gly Cys Val Thr Ile Thr Ala Glu Gly Lys Pro Ser
305                 310                 315                 320

Met Asp Val Trp Leu Asp Ser Ile Tyr Gln Glu Asn Pro Ala Lys Thr
                325                 330                 335

Arg Glu Tyr Cys Leu His Ala Lys Leu Ser Asp Thr Lys Val Val Ala
            340                 345                 350

Arg Cys Pro Thr Met Gly Pro Ala Thr Leu Ala Glu Glu His Gln Ser
        355                 360                 365

Gly Thr Val Cys Lys Arg Asp Gln Ser Asp Arg Gly Trp Gly Asn His
    370                 375                 380

Cys Gly Leu Phe Gly Lys Gly Ser Ile Val Thr Cys Val Lys Ala Ser
385                 390                 395                 400

Cys Glu Ala Lys Lys Lys Ala Thr Gly His Val Tyr Asp Ala Asn Lys
                405                 410                 415

Ile Val Tyr Thr Val Lys Val Glu Pro His Thr Gly Asp Tyr Val Ala
            420                 425                 430

Ala Asn Glu Thr His Ser Gly Arg Lys Thr Ala Ser Phe Thr Val Ser
        435                 440                 445

Ser Glu Lys Thr Ile Leu Thr Met Gly Asp Tyr Gly Asp Val Ser Leu
    450                 455                 460

Leu Cys Arg Val Ala Ser Gly Val Asp Leu Ala Gln Thr Val Ile Leu

465                 470                 475                 480

Glu Leu Asp Lys Thr Ser Glu His Leu Pro Thr Ala Trp Gln Val His
                485                 490                 495

Arg Asp Trp Phe Asn Asp Leu Ala Leu Pro Trp Lys His Glu Gly Ala
            500                 505                 510

Gln Asn Trp Asn Asn Ala Glu Arg Leu Val Glu Phe Gly Ala Pro His
        515                 520                 525

Ala Val Lys Met Asp Val Tyr Asn Leu Gly Asp Gln Thr Gly Val Leu
    530                 535                 540

Leu Lys Ser Leu Ala Gly Val Pro Val Ala His Ile Asp Gly Thr Lys
545                 550                 555                 560

Tyr His Leu Lys Ser Gly His Val Thr Cys Glu Val Gly Leu Glu Lys
                565                 570                 575

Leu Lys Met Lys Gly Leu Thr Tyr Thr Met Cys Asp Lys Thr Lys Phe
            580                 585                 590

Thr Trp Lys Arg Ile Pro Thr Asp Ser Gly His Asp Thr Val Val Met
        595                 600                 605

Glu Val Ala Phe Ser Gly Thr Lys Pro Cys Arg Ile Pro Val Arg Ala
    610                 615                 620

Val Ala His Gly Ser Pro Asp Val Asn Val Ala Met Leu Ile Thr Pro
625                 630                 635                 640

Asn Pro Thr Ile Glu Thr Asn Gly Gly Gly Phe Ile Glu Met Gln Leu
                645                 650                 655

Pro Pro Gly Asp Asn Ile Ile Tyr Val Gly Glu Leu Ser His Gln Trp
            660                 665                 670

Phe Gln Lys Gly Ser Ser Ile Gly Arg Val Phe Gln Lys Thr Arg Lys
        675                 680                 685

Gly Ile Glu Arg Leu Thr Val Ile Gly Glu His Ala Trp Asp Phe Gly
    690                 695                 700

Ser Thr Gly Gly Phe Leu Thr Ser Val Gly Lys Ala Leu His Thr Val
705                 710                 715                 720

Leu Gly Gly Ala Phe Asn Ser Leu Phe Gly Gly Val Gly Phe Leu Pro
                725                 730                 735

Lys Ile Leu Val Gly Val Val Leu Ala Trp Leu Gly Leu Asn Met Arg
            740                 745                 750

Asn Pro Thr Met Ser Met Ser Phe Leu Leu Ala Gly Gly Leu Val Leu
        755                 760                 765

Ala Met Thr Leu Gly Val Gly Ala Asp Val Gly Cys Ala Val Asp Thr
    770                 775                 780

Glu Arg Met Glu Leu Arg Cys Gly Glu Gly Leu Val Val Trp Arg Glu
785                 790                 795                 800

Val Ser Glu Trp Tyr Asp Asn Tyr Ala Tyr Tyr Pro Glu Thr Pro Gly
                805                 810                 815

Ala Leu Ala Ser Ala Ile Lys Glu Thr Phe Glu Glu Gly Thr Cys Gly
            820                 825                 830

Ile Val Pro Gln Asn Arg Leu Glu Met Ala Met Trp Arg Ser Ser Ala
        835                 840                 845

Thr Glu Leu Asn Leu Ala Leu Ala Glu Gly Asp Ala Asn Leu Thr Val
    850                 855                 860

Val Val Asp Lys Leu Asp Pro Thr Asp Tyr Arg Gly Gly Ile Pro Gly
865                 870                 875                 880

Leu Leu Lys Lys Gly Lys Asp Ile Lys Val Ser Trp Arg Ser Trp Gly
                885                 890                 895

His Ser Met Ile Trp Ser Val Pro Glu Ala Pro Arg Arg Phe Met Val
            900                 905                 910

Gly Thr Glu Gly Ser Ser Glu Cys Pro Pro Glu Arg Arg Lys Thr Gly
        915                 920                 925

Val Phe Thr Val Ala Glu Phe Gly Val Gly Leu Arg Thr Lys Val Phe
    930                 935                 940

Leu Asp Phe Arg Gln Glu Ser Thr His Glu Cys Asp Thr Gly Val Met
945                 950                 955                 960

Gly Ala Ala Val Lys Asn Gly Met Ala Val His Thr Asp Gln Ser Leu
                965                 970                 975

Trp Met Lys Ser Val Arg Asn Asp Thr Gly Thr Tyr Ile Val Glu Leu
            980                 985                 990

Leu Val Thr Asp Leu Arg Asn Cys  Ser Trp Pro Ala Ser  His Thr Ile
        995                 1000                 1005

Asp Asn  Ala Glu Val Val Asp  Ser Glu Leu Phe Leu  Pro Ala Ser
    1010                 1015                 1020

Leu Ala  Gly Pro Arg Ser Trp  Tyr Asn Arg Ile Pro  Gly Tyr Ser
    1025                 1030                 1035

Glu Gln  Val Lys Gly Pro Trp  Lys Tyr Ser Pro Ile  Arg Val Thr
    1040                 1045                 1050

Arg Glu  Glu Cys Pro Gly Thr  Arg Val Thr Ile Asn  Ala Asp Cys
    1055                 1060                 1065

Asp Lys  Arg Gly Ala Ser Val  Arg Ser Thr Thr Glu  Ser Gly Lys
    1070                 1075                 1080

Val Ile  Pro Glu Trp Cys Cys  Arg Thr Cys Thr Leu  Pro Pro Val
    1085                 1090                 1095

Thr Ile  Arg Thr Gly Thr Asp  Cys Trp Tyr Ala Met  Glu Ile Arg
    1100                 1105                 1110

Pro Val  His Asp Gln Gly Gly  Leu Val Arg Ser Met  Val Val Ala
    1115                 1120                 1125

Asp Asn  Gly Glu Leu Leu Ser  Glu Gly Gly Val Pro  Gly Ile Val
    1130                 1135                 1140

Ala Leu  Phe Val Val Leu Glu  Tyr Val Ile Arg Arg  Arg Pro Ala
    1145                 1150                 1155

Thr Gly  Thr Thr Ala Met Trp  Gly Gly Ile Val Val  Leu Ala Leu
    1160                 1165                 1170

Leu Val  Thr Gly Leu Val Arg  Ile Glu Ser Leu Val  Arg Tyr Val
    1175                 1180                 1185

Val Ala  Val Gly Ile Thr Phe  His Leu Glu Leu Gly  Pro Glu Ile
    1190                 1195                 1200

Val Ala  Leu Thr Leu Leu Gln  Ala Val Phe Glu Leu  Arg Val Gly
    1205                 1210                 1215

Leu Leu Ser Ala Phe Ala Leu Arg Ser Asn Leu Thr Val Arg Glu
    1220                1225                1230

Met Val Thr Ile Tyr Phe Leu Leu Leu Val Leu Glu Leu Gly Leu
    1235                1240                1245

Pro Ser Glu Gly Leu Gly Ala Leu Trp Lys Trp Gly Asp Ala Leu
    1250                1255                1260

Ala Met Gly Ala Leu Ile Phe Arg Ala Cys Thr Ala Glu Glu Lys
    1265                1270                1275

Thr Gly Val Gly Leu Leu Leu Met Ala Leu Met Thr Gln Gln Asp
    1280                1285                1290

Leu Ala Ile Val His Tyr Gly Leu Met Leu Phe Leu Gly Val Ala
    1295                1300                1305

Ser Cys Cys Ser Ile Trp Lys Leu Ile Arg Gly His Arg Glu Gln
    1310                1315                1320

Lys Gly Leu Thr Trp Val Val Pro Leu Ala Gly Leu Leu Gly Gly
    1325                1330                1335

Glu Gly Ser Gly Val Arg Leu Leu Ala Phe Trp Glu Leu Ala Ile
    1340                1345                1350

His Gly Arg Arg Arg Ser Phe Ser Glu Pro Leu Thr Val Val Gly
    1355                1360                1365

Val Met Leu Thr Leu Ala Ser Gly Met Met Trp His Thr Ser Gln
    1370                1375                1380

Glu Ala Leu Cys Ala Leu Ala Val Ala Ser Phe Leu Leu Leu Met
    1385                1390                1395

Leu Val Leu Gly Thr Arg Lys Met Gln Leu Val Ala Glu Trp Ser
    1400                1405                1410

Gly Cys Val Glu Trp His Pro Glu Leu Met Asn Glu Gly Gly Glu
    1415                1420                1425

Val Ser Leu Arg Val Arg Gln Asp Ser Met Gly Asn Phe His Leu
    1430                1435                1440

Thr Glu Leu Glu Lys Glu Glu Arg Val Met Ala Phe Trp Leu Leu

1445                1450                1455

Ala Gly Leu Ala Ala Ser Ala Phe His Trp Ser Gly Ile Leu Gly
    1460                1465                1470

Val Met Gly Leu Trp Thr Leu Ser Glu Met Leu Arg Thr Ala Arg
    1475                1480                1485

Arg Ser Asp Leu Val Phe Ser Gly Gln Gly Gly Arg Glu Arg Gly
    1490                1495                1500

Asp Arg Pro Phe Glu Val Lys Asp Gly Val Tyr Arg Ile Phe Ser
    1505                1510                1515

Pro Gly Leu Leu Trp Gly Gln Arg Gln Val Gly Val Gly Tyr Gly
    1520                1525                1530

Ser Lys Gly Val Leu His Thr Met Trp His Val Thr Arg Gly Ala
    1535                1540                1545

Ala Leu Ser Ile Asp Asp Ala Val Ala Gly Pro Tyr Trp Ala Tyr
    1550                1555                1560

Val Lys Glu Asp Val Val Cys Tyr Gly Gly Ala Trp Ser Leu Glu
    1565                1570                1575

Glu Lys Trp Lys Gly Glu Thr Val Gln Val His Ala Phe Pro Pro
    1580                1585                1590

Gly Arg Ala His Glu Val His Gln Cys Gln Pro Gly Glu Leu Leu
    1595                1600                1605

Leu Asp Thr Gly Arg Arg Ile Gly Ala Val Pro Ile Asp Leu Ala
    1610                1615                1620

Lys Gly Thr Ser Gly Ser Pro Thr Leu Asn Ser Gln Gly Val Val
    1625                1630                1635

Val Gly Leu Tyr Gly Asn Gly Leu Lys Thr Asn Glu Thr Tyr Val
    1640                1645                1650

Ser Ser Ile Ala Gln Gly Glu Ala Glu Lys Ser Arg Pro Asn Leu
    1655                1660                1665

Pro Pro Ala Val Ile Gly Thr Gly Trp Thr Ala Lys Gly Gln Ile
    1670                1675                1680

Thr Val Leu Asp Met His Pro Gly Ser Gly Lys Thr His Arg Val
    1685                1690                1695

Leu Pro Glu Leu Ile Arg Gln Cys Ile Asp Arg Arg Leu Arg Thr
    1700                1705                1710

Leu Val Leu Ala Pro Thr Arg Val Val Leu Lys Glu Met Glu Arg
    1715                1720                1725

Ala Leu Asn Gly Lys Arg Val Arg Phe His Ser Pro Ala Val Gly
    1730                1735                1740

Asp Gln Gln Val Gly Gly Ala Ile Val Asp Val Met Cys His Ala
    1745                1750                1755

Thr Tyr Val Asn Arg Arg Leu Leu Pro Gln Gly Arg Gln Asn Trp
    1760                1765                1770

Glu Val Ala Ile Met Asp Glu Ala His Trp Thr Asp Pro His Ser
    1775                1780                1785

Ile Ala Ala Arg Gly His Leu Tyr Thr Leu Ala Lys Glu Asn Lys
    1790                1795                1800

Cys Ala Leu Val Leu Met Thr Ala Thr Pro Pro Gly Lys Ser Glu
    1805                1810                1815

Pro Phe Pro Glu Ser Asn Gly Ala Ile Thr Ser Glu Glu Lys Gln
    1820                1825                1830

Ile Pro Asp Gly Glu Trp Arg Asp Gly Phe Asp Trp Ile Thr Glu
    1835                1840                1845

Tyr Glu Gly Arg Thr Ala Trp Phe Val Pro Ser Ile Ala Lys Gly
    1850                1855                1860

Gly Ile Ile Ala Arg Thr Leu Arg Gln Lys Gly Lys Ser Val Ile
    1865                1870                1875

Cys Leu Asn Ser Lys Thr Phe Glu Lys Asp Tyr Ser Arg Val Arg
    1880                1885                1890

Asp Glu Lys Pro Asp Phe Val Val Thr Thr Asp Ile Ser Glu Met
    1895                1900                1905

Gly Ala Asn Leu Asp Val Ser Arg Val Ile Asp Gly Arg Thr Asn
    1910                1915                1920

Ile Lys Pro Glu Glu Val Asp Gly Arg Val Glu Leu Thr Gly Thr
    1925                1930                1935

Arg Arg Val Thr Thr Ala Ser Ala Ala Gln Arg Arg Gly Arg Val
    1940                1945                1950

Gly Arg Gln Glu Gly Arg Thr Asp Glu Tyr Ile Tyr Ser Gly Gln
    1955                1960                1965

Cys Asp Asp Asp Asp Ser Gly Leu Val Gln Trp Lys Glu Ala Gln
    1970                1975                1980

Ile Leu Leu Asp Asn Ile Thr Thr Leu Arg Gly Pro Val Ala Thr
    1985                1990                1995

Phe Tyr Gly Pro Glu Gln Asp Lys Met Pro Glu Val Ala Gly His
    2000                2005                2010

Phe Arg Leu Thr Glu Glu Lys Arg Lys His Phe Arg His Leu Leu
    2015                2020                2025

Thr His Cys Asp Phe Thr Pro Trp Leu Ala Trp His Val Ala Ala
    2030                2035                2040

Asn Val Ser Ser Val Thr Ser Arg Asn Trp Thr Trp Glu Gly Pro
    2045                2050                2055

Glu Glu Asn Thr Val Asp Glu Ala Asn Gly Asp Leu Val Thr Phe
    2060                2065                2070

Arg Ser Pro Asn Gly Ala Glu Arg Thr Leu Arg Pro Val Trp Arg
    2075                2080                2085

Asp Ala Arg Met Phe Arg Glu Gly Arg Asp Ile Arg Glu Phe Ile
    2090                2095                2100

Ala Tyr Ala Ser Gly Arg Arg Ser Phe Gly Asp Val Leu Ser Gly
    2105                2110                2115

Met Ser Gly Val Pro Glu Leu Leu Arg His Arg Cys Val Ser Ala
    2120                2125                2130

Met Asp Val Phe Tyr Thr Leu Met His Glu Glu Pro Gly Ser Arg
    2135                2140                2145

Ala Met Lys Met Ala Glu Arg Asp Ala Pro Glu Ala Phe Leu Thr
    2150                2155                2160

Val Val Glu Met Met Val Leu Gly Leu Ala Thr Leu Gly Val Val
    2165                2170                2175

Trp Cys Phe Val Val Arg Thr Ser Ile Ser Arg Met Met Leu Gly
    2180                2185                2190

Thr Leu Val Leu Leu Ala Ser Leu Ala Leu Leu Trp Ala Gly Gly
    2195                2200                2205

Val Ser Tyr Gly Asn Met Ala Gly Val Ala Leu Ile Phe Tyr Thr
    2210                2215                2220

Leu Leu Thr Val Leu Gln Pro Glu Ala Gly Lys Gln Arg Ser Ser
    2225                2230                2235

Asp Asp Asn Lys Leu Ala Tyr Phe Leu Leu Thr Leu Cys Ser Leu
    2240                2245                2250

Ala Gly Leu Val Ala Ala Asn Glu Met Gly Phe Leu Glu Lys Thr
    2255                2260                2265

Lys Ala Asp Leu Ser Thr Val Leu Trp Ser Glu His Glu Glu Leu
    2270                2275                2280

Arg Ser Trp Glu Glu Trp Thr Asn Ile Asp Ile Gln Pro Ala Arg
    2285                2290                2295

Ser Trp Gly Thr Tyr Val Leu Val Val Ser Leu Phe Thr Pro Tyr
    2300                2305                2310

Ile Ile His Gln Leu Gln Thr Lys Ile Gln Gln Leu Val Asn Ser
    2315                2320                2325

Ala Val Ala Thr Gly Ala Gln Ala Met Arg Asp Leu Gly Gly Gly
    2330                2335                2340

Ala Pro Phe Ile Gly Val Ala Gly His Val Met Ala Leu Gly Val
    2345                2350                2355

Val Ser Leu Val Gly Ala Thr Pro Thr Ser Leu Val Val Gly Val
    2360                2365                2370

Gly Leu Ala Ala Phe His Leu Ala Ile Val Val Ser Gly Leu Glu
    2375                2380                2385

Ala Glu Leu Thr Gln Arg Ala His Lys Val Phe Phe Ser Ala Met

2390                2395                2400

Val Arg Asn Pro Met Val Asp Gly Asp Val Ile Asn Pro Phe Gly
    2405                2410                2415

Glu Gly Glu Ala Lys Pro Ala Leu Tyr Glu Arg Lys Met Ser Leu
    2420                2425                2430

Val Leu Ala Ile Val Leu Cys Leu Met Ser Val Val Met Asn Arg
    2435                2440                2445

Thr Val Pro Ser Ile Thr Glu Ala Ser Ala Val Gly Leu Ala Ala
    2450                2455                2460

Ala Gly Gln Leu Leu Arg Pro Glu Ala Asp Thr Leu Trp Thr Met
    2465                2470                2475

Pro Val Ala Cys Gly Leu Ser Gly Val Val Arg Gly Ser Leu Trp
    2480                2485                2490

Gly Phe Leu Pro Leu Gly His Arg Leu Trp Leu Arg Ala Ser Gly
    2495                2500                2505

Ser Arg Arg Gly Gly Ser Glu Gly Asp Thr Leu Gly Asp Leu Trp
    2510                2515                2520

Lys Arg Lys Leu Asn Gly Cys Thr Lys Glu Glu Phe Phe Ala Tyr
    2525                2530                2535

Arg Arg Thr Gly Ile Leu Glu Thr Glu Arg Asp Lys Ala Arg Glu
    2540                2545                2550

Leu Leu Arg Arg Gly Glu Thr Asn Met Gly Leu Ala Val Ser Arg
    2555                2560                2565

Gly Thr Ala Lys Leu Ala Trp Leu Glu Glu Arg Gly Tyr Ala Thr
    2570                2575                2580

Leu Lys Gly Glu Val Val Asp Leu Gly Cys Gly Arg Gly Gly Trp
    2585                2590                2595

Ser Tyr Tyr Ala Ala Ser Arg Pro Ala Val Met Ser Val Lys Ala
    2600                2605                2610

Tyr Thr Ile Gly Gly Lys Gly His Glu Thr Pro Lys Met Val Thr
    2615                2620                2625

Ser Leu Gly Trp Asn Leu Ile Lys Phe Arg Ala Gly Met Asp Val
    2630                2635                2640

Phe Ser Met Gln Pro His Arg Ala Asp Thr Ile Met Cys Asp Ile
    2645                2650                2655

Gly Glu Ser Asn Pro Asp Ala Val Val Glu Gly Glu Lys Thr Arg
    2660                2665                2670

Lys Val Ile Leu Leu Met Glu Gln Trp Lys Asn Arg Asn Pro Thr
    2675                2680                2685

Ala Thr Cys Val Phe Lys Val Leu Ala Pro Tyr Arg Pro Glu Val
    2690                2695                2700

Ile Glu Ala Leu His Arg Phe Gln Leu Gln Trp Gly Gly Gly Leu
    2705                2710                2715

Val Arg Thr Pro Phe Ser Arg Asn Ser Thr His Glu Met Tyr Tyr
    2720                2725                2730

Ser Thr Ala Val Thr Gly Asn Ile Val Asn Ser Val Asn Ile Gln
    2735                2740                2745

Ser Arg Lys Leu Leu Ala Arg Phe Gly Asp Gln Arg Gly Pro Thr
    2750                2755                2760

Arg Val Pro Glu Leu Asp Leu Gly Val Gly Thr Arg Cys Val Val
    2765                2770                2775

Leu Ala Glu Asp Lys Val Lys Glu Lys Asp Val Gln Glu Arg Ile
    2780                2785                2790

Ser Ala Leu Arg Glu Gln Tyr Gly Glu Thr Trp His Met Asp Arg
    2795                2800                2805

Glu His Pro Tyr Arg Thr Trp Gln Tyr Trp Gly Ser Tyr Arg Thr
    2810                2815                2820

Ala Pro Thr Gly Ser Ala Ala Ser Leu Ile Asn Gly Val Val Lys
    2825                2830                2835

Leu Leu Ser Trp Pro Trp Asn Ala Arg Glu Asp Val Val Arg Met
    2840                2845                2850

Ala Met Thr Asp Thr Thr Ala Phe Gly Gln Gln Arg Val Phe Lys
    2855                2860                2865

Glu Lys Val Asp Thr Lys Ala Gln Glu Pro Gln Pro Gly Thr Lys
    2870                2875                2880

Val Ile Met Arg Ala Val Asn Asp Trp Ile Leu Glu Arg Leu Ala
    2885                2890                2895

Arg Lys Ser Lys Pro Arg Met Cys Ser Arg Glu Glu Phe Ile Ala
    2900                2905                2910

Lys Val Lys Ser Asn Ala Ala Leu Gly Ala Trp Ser Asp Glu Gln
    2915                2920                2925

Asn Arg Trp Ser Ser Ala Lys Glu Ala Val Glu Asp Pro Ala Phe
    2930                2935                2940

Trp Gln Leu Val Asp Glu Glu Arg Glu Arg His Leu Ala Gly Arg
    2945                2950                2955

Cys Ala His Cys Ala Tyr Asn Met Met Gly Lys Arg Glu Lys Lys
    2960                2965                2970

Leu Gly Glu Phe Gly Val Ala Lys Gly Ser Arg Ala Ile Trp Tyr
    2975                2980                2985

Met Trp Leu Gly Ser Arg Phe Leu Glu Phe Glu Ala Leu Gly Phe
    2990                2995                3000

Leu Asn Glu Asp His Trp Ala Ser Arg Gly Ser Ser Gly Ser Gly
    3005                3010                3015

Val Glu Gly Ile Ser Leu Asn Tyr Leu Gly Trp Tyr Leu Lys Gly
    3020                3025                3030

Leu Ser Thr Leu Glu Gly Gly Leu Phe Tyr Ala Asp Asp Thr Ala
    3035                3040                3045

Gly Trp Asp Thr Lys Val Thr Asn Ala Asp Leu Glu Asp Glu Glu
    3050                3055                3060

Gln Leu Leu Arg Tyr Met Glu Gly Glu His Lys Gln Leu Ala Ala
    3065                3070                3075

Thr Ile Met Gln Lys Ala Tyr His Ala Lys Val Val Lys Val Ala
    3080                3085                3090

Arg Pro Ser Arg Asp Gly Gly Cys Val Met Asp Val Ile Thr Arg
    3095                3100                3105

Arg Asp Gln Arg Gly Ser Gly Gln Val Val Thr Tyr Ala Leu Asn
    3110                3115                3120

Thr Leu Thr Asn Ile Lys Val Gln Leu Ile Arg Met Met Glu Gly
    3125                3130                3135

Glu Gly Val Ile Glu Ala Ser Asp Ala His Asn Pro Arg Leu Leu
    3140                3145                3150

Arg Val Glu Arg Trp Leu Arg Asn His Gly Glu Glu Arg Leu Gly
    3155                3160                3165

Arg Met Leu Val Ser Gly Asp Asp Cys Val Val Arg Pro Val Asp
    3170                3175                3180

Asp Arg Phe Ser Arg Ala Leu Tyr Phe Leu Asn Asp Met Ala Lys
    3185                3190                3195

Thr Arg Lys Asp Ile Gly Glu Trp Glu His Ser Val Gly Phe Ser
    3200                3205                3210

Asn Trp Glu Glu Val Pro Phe Cys Ser His His Phe His Glu Leu
    3215                3220                3225

Val Met Lys Asp Gly Arg Ala Leu Ile Val Pro Cys Arg Asp Gln
    3230                3235                3240

Asp Glu Leu Val Gly Arg Ala Arg Val Ser Pro Gly Cys Gly Trp
    3245                3250                3255

Ser Val Arg Glu Thr Ala Cys Leu Ser Lys Ala Tyr Gly Gln Met
    3260                3265                3270

Trp Leu Leu Ser Tyr Phe His Arg Arg Asp Leu Arg Thr Leu Gly
    3275                3280                3285

Leu Ala Ile Cys Ser Ala Val Pro Ile Asp Trp Val Pro Thr Gly
    3290                3295                3300

Arg Thr Thr Trp Ser Ile His Ala Ser Gly Ala Trp Met Thr Thr
    3305                3310                3315

Glu Asp Met Leu Asp Val Trp Asn Arg Val Trp Ile Leu Asp Asn
    3320                3325                3330

Pro Phe Met His Ser Lys Glu Lys Ile Val Glu Trp Arg Asp Val

3335                3340                3345

Pro Tyr Leu Pro Lys Ser His Asp Met Leu Cys Ser Ser Leu Val
    3350                3355                3360

Gly Arg Lys Glu Arg Ala Glu Trp Ala Lys Asn Ile Trp Gly Ala
    3365                3370                3375

Val Glu Lys Val Arg Lys Met Ile Gly Gln Glu Arg Phe Lys Asp
    3380                3385                3390

Tyr Leu Ser Cys Met Asp Arg His Asp Leu His Trp Glu Leu Lys
    3395                3400                3405

Leu Glu Ser Ser Ile Ile
    3410

## Description

### FIELD OF THE INVENTION

The present invention relates generally to the fields of virology, immunology and diagnostics. More particularly, it concerns compositions and methods for detecting flaviviridae infections, preferably dengue virus infections.

### BACKGROUND OF THE INVENTION

The Flaviviridae family of viruses comprises at least three distinct genera: *pestiviruses,* which cause disease in cattle and pigs; *hepaciviruses,* whose sole member is HCV; and *flaviviruses,* which are the primary cause of viral hemorrhagic diseases such as dengue fever, west nile fever and yellow fever. The flavivirus genus includes almost 70 members separated into 20 groups on the basis of serological relatedness (Calisher et al., J. Gen. Virol, 1993, 70, 37-43). Clinical symptoms vary and include fever, encephalitis and hemorrhagic fever *(*Fields Virology, Editors: Fields, B. N., Knipe, D. M., and Howley, P.M., Lippincott-Raven Publishers, Philadelphia, PA, 1996, Chapter 31, 931-959). Flaviviruses of global concern that are associated with human disease include the Dengue Hemorrhagic Fever viruses (DENVs), Yellow Fever virus (YFV), West Nile virus (WNV), Japanese Encephalitis virus (JEV) and Tick-Borne Encephalitis virus (TBEV) (Halstead, S. B., Rev. Infect. Dis., 1984, 6, 251-264; Halstead, S. B., Science, 239:476-481, 1988; Monath, T. P., New Eng. J. Med, 1988, 319, 641-643).

DENVs cause infections in humans that can be distinguished into dengue fever (DF), dengue hemorrhagic fever (DHF) and dengue shock syndrome (DSS) (WHO, 1996). A characteristic of DENVs unique in the Flaviviridae family is the existence of serotypes. Four serotypes have been extensively described, commonly designated as DENV-1 to DENV-4. These serotypes are different in their antigenic potential and show on amino acid level a minimum of 65% sequence identity. The DENV serotypes react differently with the human immune system. Especially the induction of neutralizing antibodies is specific for the respective serotype. Recently a fifth serotype was detected indicating a continuously development of dengue variation. A report presented October 2013 by Dr Nikolaos Vasilakis at the Bangkok 3rd International Conference on Dengue and Dengue Haemorrhagic Fever describes the discovery of a new, the fifth type of dengue virus (DENV-5) serotype. As stated by Dr Nikolaos Vasilakis DENV-5 was identified in samples from DENV patients collected in northern Malaysia's. Even though no DENV-5 sequence has yet been published, it is expected, that the new DENV will be also be within the range of 65% amino acid identity with the known DENV-1-4 viruses.

In principle, the primary infection with one specific DENV serotype leads to a lifelong neutralizing immune response preventing an infection by the same serotype. The profound genetic variation of DENV, however causes not only the emergence of different serotypes, it also leads to the development of variants within each serotype, designated dengue virus genotypes. Those genotypes show amino acid variations in the DENV envelope genes and can therefore escape from serum antibody neutralization.

Another complication caused by the emergence of different DENV sero- and genotypes is antibody-dependent enhancement (ADE). Namely, during a primary DENV infection, in addition to neutralizing antibodies, a non-neutralizing humoral response is induced by the respective DENV. The resulting non-neutralizing, cross-reactive antibodies in the serum of a patient enhance viral entry when an infection with a second DENV serotype occurs different from the virus that caused the primary infection (Dejnirattisai et al., 2010; Halstead et al., 1977; Smith et al., 2012), and have been hypothesized to constitute a major factor leading to severe courses of DENV infections.

The preferred tool for determining flaviviral infections is detection of virus RNA or viral antigens, for example the NS1 antigen. Flaviviruses can also be isolated using *in vitro* cell culture systems, for example of insect cells, or, in the case of e.g. DENV, by mosquito feeding (Gubler, D.J. (1989) Surveillance for dengue and dengue hemorrhagic fever. Bull. Pan. Am. Health Organ. 23,397-404). These methods are all aimed at detecting the virus itself or its constituents and, therefore, are referred to as direct detection methods.

There are also indirect detection methods making use of the fact that, in later stages of infection, the humoral immune response in form of IgM and IgG antibodies is well established. A flaviviral infection can therefore be diagnosed indirectly by detecting the antibodies targeting the infecting virus using e.g. ELISA or IFA techniques. A further indirect detection method, which can be used to monitor DENV serotype specific antibodies, is the DENV neutralization assay. Here, patient serum is tested in cell cultures for its efficiency to block viral entry.

The antigens used for indirect detection of flaviviral infection, e.g. by ELISA or IFA techniques, belong to the two virus-coded glycoproteins all Flaviviruses carry integrated into their membrane, which glycoproteins are responsible for cell attachment, binding of receptor molecules and the fusion of the viral membrane with the cell. For the flaviviruses these two glycoproteins are designated M and E. Both are the predominant targets for antibodies in human serum samples. In natural infection antibodies preferentially bind to the E protein that consist of the three beta-barrel domains ED1, ED2 and ED3 followed by the stem region and the membrane anchor region. Patient sera derived neutralizing antibodies to DENV are found to be directed against all three domains with the ED3 domain as the major target for the most potent type-specific neutralizing antibodies (Crill et al., 2009, Simmons et al., 1998; Wahala et al., 2009, 2012; Izquierdo et al., 2012; Williams et al., 2012, Hsieh et al., 2008; Klein et al., 2013; Lin et al., 2011; Schmidt et al., 2010; Zhang et al., 2012).

The conventional methods for flavivirus detection have their limitations. Direct detection of virus RNA by RT-PCR or detection of soluble NS1 antigen is only possible in the early acute phase of the disease. The same applies also to isolation of virus by mosquito feeding. Thus, the type of flavivirus can only be diagnosed with these methods, when patient blood or serum samples are available from early infection. With a realistic look on the dengue endemic regions, it will become clear, however, that not everyone infected by dengue is seeking professional medical advice during the acute phase of primary or secondary dengue infection. Therefore, a substantive number of patients in these regions cannot be diagnosed using direct detection.

The main problem associated with ELISA or IFA based detection of patient's antibodies against infecting flaviviruses lies in the fact that these indirect procedures cannot reliably distinguish between different kinds of flaviviruses or the different serotypes or genotypes of DENV. Since the antigens used in these assays, i.e. in particular the ED3 domain of the flaviviral E-protein, share a high degree of sequence identity, many antibodies in patients' sera, are crossreactive for DENV-1-4, WNV, JEV, YFV and TBEV (Dobler G et al, 1997; Allwinn R, 2002; Stiasny K et al., 2006).

Similar crossreactivity is also expected for antibodies against the new DENV-5 serotype identified in the Philippines. The DENV neutralization assay, on the other hand, has the disadvantage that, requiring testing of viral entry efficiency for each of the DENV serotypes in separate cell cultures, it is a time consuming and very expensive method that cannot be used for rapid standard diagnostics. Moreover, because the test requires extensive handling of infections DENV strains, a safety laboratory (BSL3) for the isolation and cultivation of DENV, the preparation of virus stocks and the neutralization assays itself is required for doing this kind of serotype-specific virus assays.

Another deficiency common to all known techniques is related to the fact that, as mentioned in the outset, the antibody specificity that is principally formed after primary infection controls only the original DENV serotype and does not provide protection against all other serotypes. The serotype specific response and lack of protection to all other serotypes also complicates DENV diagnosis in a secondary infection especially in regions where different serotypes of DENVs are present and patients can have multiple DENV infections with different serotypes. Thus, patients will develop highly-specific immune responses not only to one DENV, in DENV endemic areas it is conceivable that serotype-specific antibodies to more than one DENV-type can be diagnosed. Using conventional techniques, however, even if the different DENV serotypes can be differentiated using the DENV neutralization assay, it cannot be determined, which of the serotypes found is the source of the secondary infection. Therefore, it is important to develop antigens which can detect serotype specific antibodies and that the results obtained using these antigens are clearly correlated with the diagnostic "golden standard" test: the serotyping by a DENV neutralization assay. Antibodytiter differences observed by DENV neutralization test should be ideally in correlation with the Antibody reactivities seen in the serotype specific antibody assays.

It follows from the above, that there is an urgent need for new diagnostic tools which can detect strain specific anti-flavivirus antibodies, especially serotype-specific anti-dengue antibodies, at any stage of the disease, especially in the stages were virus or virus antigen concentrations are below detection level for conventional diagnostic techniques. Serological diagnostic tools that would enable the identification of the DENV serotype are also of great importance for better epidemiological surveillance, outbreak investigations and vaccine efficacy trials (Peeling et al., 2010).

It is therefore an object of the present invention to provide compounds and methods for detecting flavivirus infections, in particular dengue virus infections, for improving existing methods, and in particular for improving sensitivity of existing detection methods to identify DENV-antibodies in human and animal samples and to determine the serotype-specificity of these antibodies.

The present invention disclosed herein addresses these objects and needs and other associated problems.

### SUMMARY OF THE INVENTION

The present invention relates to compounds and methods for detecting flavivirus infections, in particular dengue virus infections, which allow early and serotypespecifc detection of flavivirus infections by detection of antibodies in animal and human samples.

Specifically, the invention relates to flavivirus envelope protein (E protein), flavivirus envelope protein domain III (ED3) and domain III-stem (ED3S) comprising polypeptides that are denatured. Further embodiments of the invention relate to compositions where these denatured polypeptides are attached to solid substrates. The invention provides methods for detecting flavivirus infections in samples from subjects suspected to have a flavivirus infection as well as kits for performing said methods.

Earlier studies on serotype-specific antibodies have focused on ED3. The interest on ED3 was inspired by the observation that ED3 is a potent candidate for the induction of neutralizing antibodies after immunization and is therefore discussed as a promising vaccine candidate that has reached phase 2 in human vaccine trials (Sabchareon et al., 2012). The activity of neutralizing antibodies in human serum samples is not significantly absorbed by recombinant ED3 expressed in E. coli. Thus, the DENV ED3 antibody responses induced by vaccination seems to be different to the ED3 response induced by DENV during natural infection (Bernardo et al., 2008).

One reason for the different immunogenicity seems to be the complex orientation of the E protein on the DENV surface. The E protein undergoes conformational transitions when for example the temperature is changed from room temperature to 37°C (Fibriansah G et al., 2013; Zhang X et al., 2013). At room temperature, the envelope proteins assemble on the viral surface building the so-called smooth surface. In this stage the virus diameter is about 500 Å, as measured by electron microscopy. At higher temperatures the envelope proteins form the so-called bumpy surface structure. This temperature dependent transition of the E protein leads to a lager virus particle of about 550 Å diameter. In total, three different virus surface appearances, smooth, fusogenic and bumpy, are described in the literature. All these native envelope conformations on the viral surface are immunogenic and it is not clear if one and especially the genetically engineered E proteins can induce the same antibody specificities as all these native forms. It is proposed that the bumpy form of the virus might be the predominant structure that is presented to the human immune system. Altogether, the differences of envelope conformations seem to be one of the difficulties to find an optimal vaccine candidate. Such a candidate should activate the human immune system in a way that antibodies are induced that are preferentially directed against envelope epitopes exposed on the smooth, fusogenic and bumpy form of the virus.

On the other hand, the sequence variation seen in the group of flaviviruses should lead also to type-specific humoral responses directed to parts of the E protein. The invention uncovers, or exposes such a serotype specific epitope that is hidden in the ED3 polypeptide and becomes apparent when the protein is denatured by heat or denaturing reagents.

The observation that native envelope conformations induce other antibody specificities than envelope based vaccines is further complicated by the identification of a discontinuous epitope overlapping ED3 and parts of the fusion loop on the second molecule of the E heterodimer. This discontinuous epitope was shown to be one of the major target for highly potent neutralizing antibody in WNV infection (de Alwis et al., 2012).

The E protein ED3 domain was expressed in *E. coli* as a potential diagnostic tool. It was fused to trpE (Fonseca et al., 1991; Simmons et al., 1998), glutathione-S-transferase (Sugrue et al., 1997; Lazaro-Olán et al., 2008), His₆ (Ludolfs et al., 2002; Reddy et al., 2012) or maltose-binding-protein (Staropoli et al., 1996). All these fusion proteins were made to generate ED3 in the most natural conformation possible and intensive antibody binding studies have identified a correctly folded ED3 when fused to the maltose-binding-protein (MBP - Wahala et al., 2009). However, it has been demonstrated that the native ED3 domains have a high cross-reactivity and, in particular have low serotype-specificity (Beltramello M et al., 2010).

The present inventors have found that, cross-reactivity of ED3 domain based antigens can be overcome by providing antigens that, in addition to the ED3 domain of the E protein, comprise also the stem region connecting, in its natural conformation, the E-protein to its membrane anchor. The resulting polypeptides and antigens hereinafter are designated as ED3S.

Further, the inventors have found that antigen specificity can be further enhanced by providing the ED3 based antigens, including the full length E protein, in denatured form. A serotype-specific antibody binding was seen when the antigens were denatured by SDS, HCl and heat. Thus the destruction of parts or the whole 3D structure of the E-protein, the ED3 or ED3S proteins (also when e.g. fused to maltose binding protein; MBP) leads to the exposure of a serotype-specific epitope or structure in the ED3 domain. This structure might be hidden in the native conformation of the antigen or might be resistant to denaturation. Denatured antigens hereinafter are designated by a star following the antigen name, e.g. ED3*.

The denatured forms of ED3, including or without the stem region, are tools to monitor serotype-specific antibodies in human serum samples i.e. in dengue infection but also in other flavivirus (WNV, YFV, JEV, TBEV) induced diseases. The detection of flavivirus type- or serotype specific antibody is correlated to the presence of antibody that neutralizes the reactive type or serotype. The assay would therefore not only give important information on the flavivirus type that had infected the patient results of the assay can also be an indicator for the presence of serotype specific neutralizing antibody. The detection of such serotype-specific antibody can be conducted not only in the acute phase of the disease when patients are seeking medical advice, the test can also be helpful to diagnose non-apparent flavivirus infections in its serotype specific manner. This is important for epidemiological surveillance studies, studies on flavivirus outbreaks and studies following vaccine trials.

### DESCRIPTION OF THE FIGURES

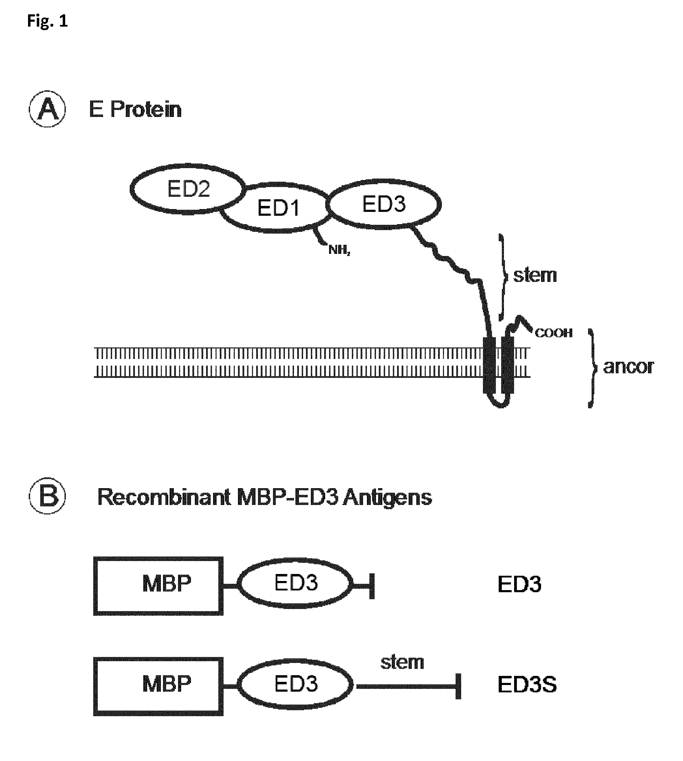

**Fig. 1****: Schematic structure of the Flavivirus E protein and the MBP-fusion proteins.**
   **A**, Schematic drawing illustrating the domain-structure of the E-protein: The E protein of flaviviruses is organized into three major domains ED1 (1), ED2 (2) and ED3 (3) which are linked to the membrane anchor by the stem (S) region.
   **B,** Schematic drawing illustrating the domain-structure of the ED3 and ED3S constructs generated and used in examples 1, 2 and 3. ED3 and the ED3S of Dengue-1-4, West Nile, Yellow Fever and TBE virus was expressed as a fusion to the maltose binding protein (MBP) in E. coli.

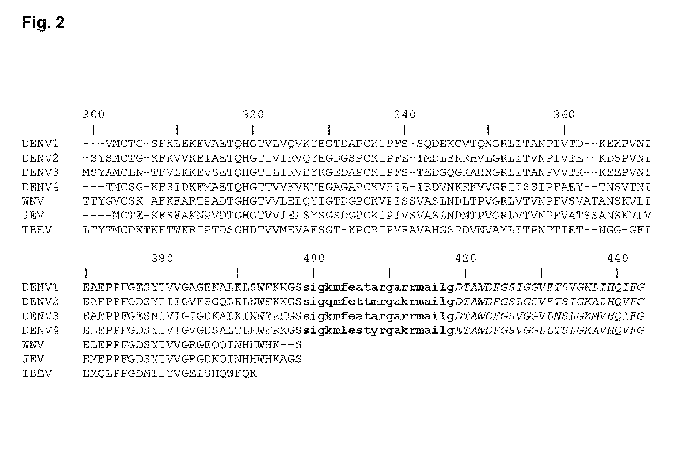

**Fig. 2****: Sequences of ED3 and ED3S**
   Sequence alignment of the c-termini of the E-proteins from DENV-1, -2, -3, -4, WN, JE and TBE viruses. The complete third domain of the E protein designated ED3 is shown large, regular print, ED3S includes part of the stem region, shown in small, regular, bold print. In addition, the C-Terminus of the E-protein is shown in large, italic print.

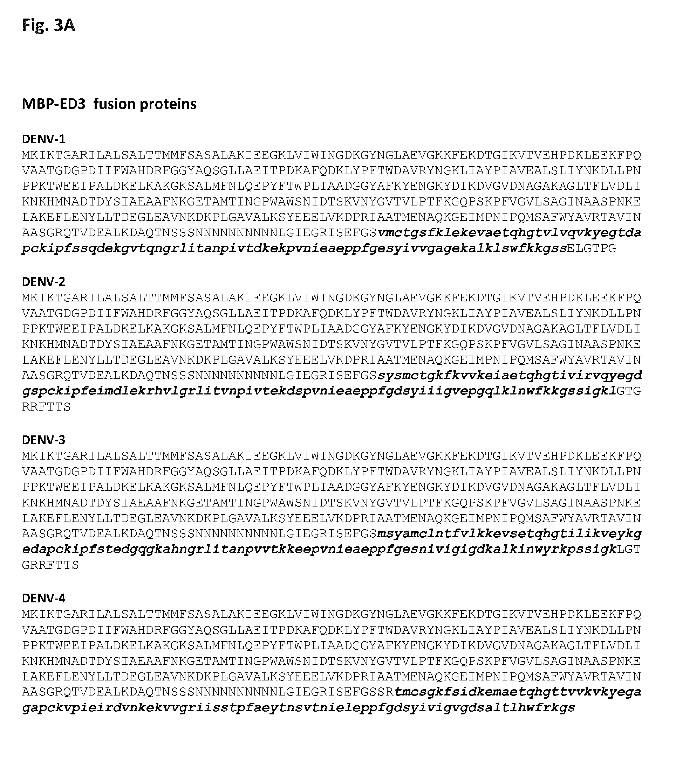

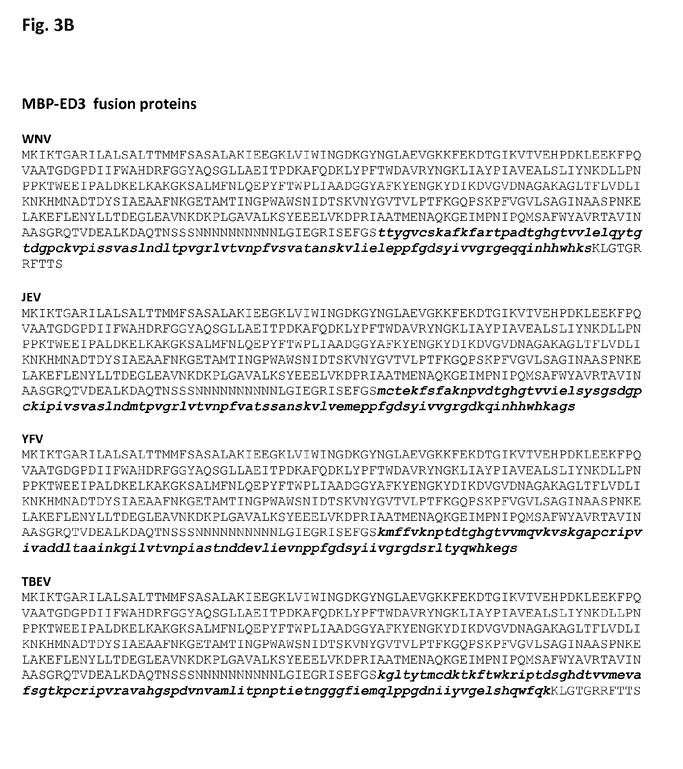

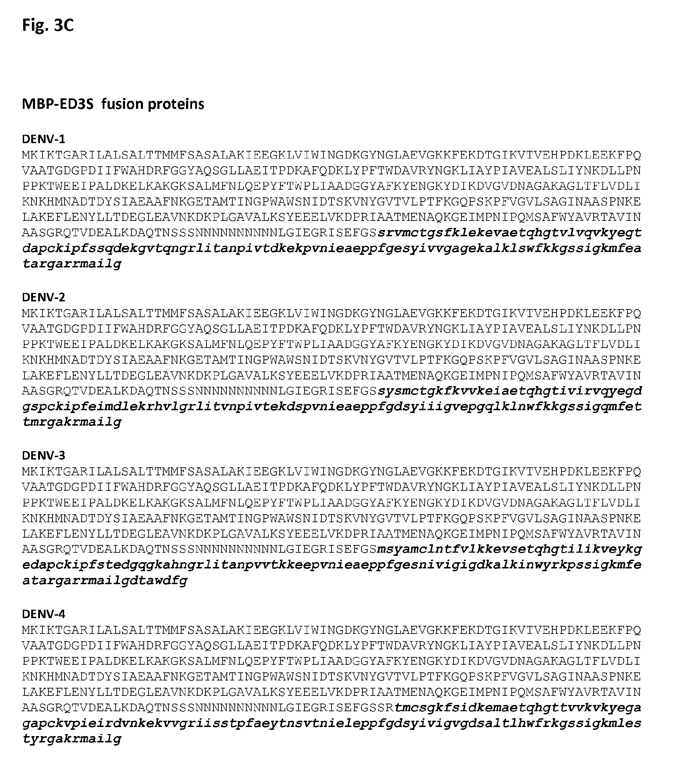

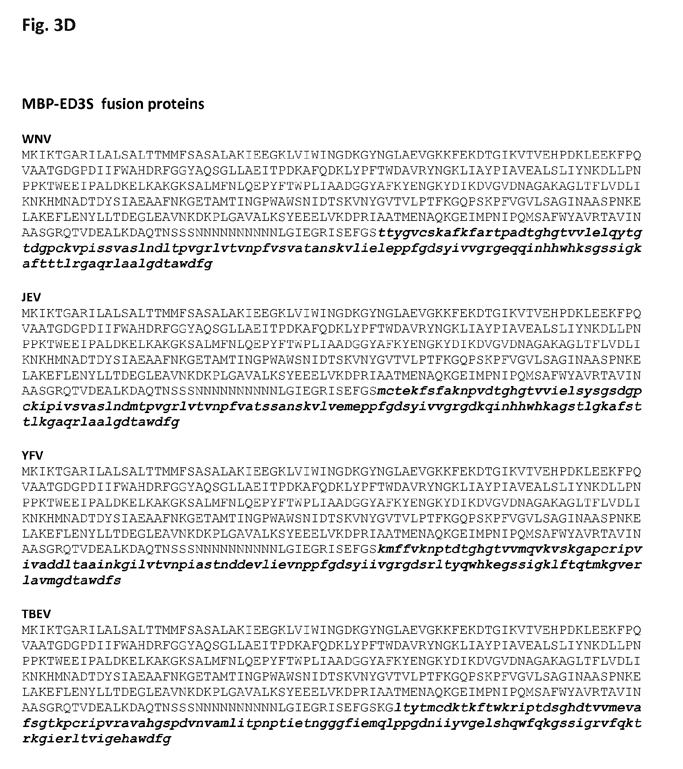

**Fig. 3****: Sequences of ED3- and ED3S-MBP fusion proteins.**
   Figure 3A shows the MBP-ED3 fusion proteins of DENV-1-4. Figure 3B shows the MBP-ED3 fusion proteins of WNV, JEV, YEV and TBEV. Figure 3C shows the MBP-ED3S fusion proteins of DENV-1-4. Figure 3D shows the MBP-ED3S fusion proteins of WNV, JEV, YEV and TBEV. In the sequences, the MBP-sequence is shown in large regular print. The ED3- and ED3S-domains fused to the MBP-sequence are shown in small italic print. The ED3 and ED3S sequences are respectively derived from the sequences retrievable under following accession numbers: U88535 (DENV-1), GQ868591 (DENV-2) M93130 (DENV-3), GQ868594 (DEN-4), M12294 (WNV), M55506 (JEV), JQ650523 (TBEV).
**Fig.: 4** **ED3 consensus sequences.**
   Shown are consensus sequences of flaviviruses DENV-1-4, JEV, TBEV, WNV and YFV, which have been generated by sequence comparison of multiple strains either available in electronic databases (e.g. Nucleotide database available at the National Center for Biotechnology Information under http://www.ncbi.nlm.nih.gov/) or available for sequencing at the inventor's laboratories.

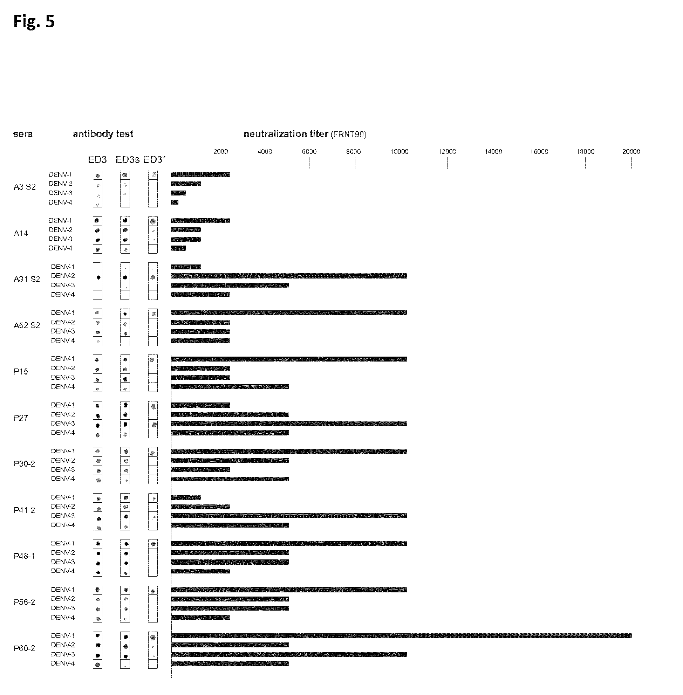

**Fig. 5****: Correlation between serotype-specific antibody reactivity to ED3* antigen and neutralization of DENVs of serotype 1-4.**
   Sera were tested for reactivity with ED3, ED3S and ED3* MBP fusion proteins for DENV-specific antibodies by a dot assay. The sera were also tested for virus neutralization to DENV-1-4. The 90% neutralization titers are indicated by the black bars. Proteins were purified from *E. coli,* dotted on nitrocellulose strips and incubated with patient serum diluted 1:100. Bound antibody was detected with a secondary anti-human HRP-conjugated antibody followed by 4-chloro-1-naphtol staining.

### DETAILED DESCRIPTION OF THE INVENTION

In one embodiment, the invention relates to a polypeptide comprising flavivirus envelope protein domain III (ED3), i.e. a polypeptide comprising an amino acid sequence that is at least 65% identical to a sequence selected from the group of SEQ ID NO: 1, 5, 9 and 13 (*E-proteins partial sequence; N-terminally truncated*), from the group of SEQ ID NO: 2, 6, 10 and 14 (*ED3S; ED3 domain and stem region*), from the group of SEQ ID NO: 3, 4, 7, 8, 11, 12 and 15 to 23 (*ED3; ED3 domain specific clone and consensus sequence*) or from the group of SEQ ID NO: 24 to 31 (*E-protein full sequence),* wherein said polypeptide is denatured.

In the context of the present invention "denatured" means that the polypeptide antigen does not or does no longer exhibit its natural secondary, tertiary and/or quarternary structure. This means that part or all of the protein's natural three dimensional structure is disrupted, ie. the polypeptide is partly or fully denatured. In other words, depending on the denaturing method, denaturing may affect the entire three dimensional structure of the protein or may affect only parts of the protein, while leaving intact or essentially intact the three dimensional structure of other parts of the protein, e.g. parts that bear a higher stability against the employed denaturing agents or conditions. Such denaturing can be accomplished by for example, but not limited to, applying heat e.g. at temperatures ranging from 50°C to 121°C and/or by treatment with denaturing reagents (i.e. SDS, guanidine, Urea, NaOH, HCl, and other inorganic and organic acids (e.g. H₂SO₄ or acetic acid) (Green and Sambrook, 2012; Lottspeich F. and Engels J.W., 2012; Westermeier R., 1990)

For example, the polypeptides of the invention may be denatured using the following conditions: 100°C for 5 min in 0,01% SDS, or 100°C for 5 min in 0,1% SDS, or 100°C for 5 min in 1% SDS. or 60°C for 5 min in 0,01% SDS, or 60°C for 5 min in 0,1% SDS, or 60°C for 5 min in 1% SDS. In addition, depending on the conditions of expression, proteins expressed in host cell (e.g. bacteria) may be expressed in a non-native, denatured form or may occur in denatured form when isolated from bacteria, eukaryotic cells or from virus samples.

While the known, native ED3 based antigens present epitopes that cross-react between DENVs and other flaviviruses, the inventors of the present invention have found that, surprisingly, ED3 based antigens create an epitope for serotype specific antibodies that is exposed only when the proteins are denatured. In addition, the cross reactive epitopes are sensitive to denaturation. Thus, the denaturing of the ED3 based antigens reduces the cross reactivity of these antigens and simultaneously enhances their serotype specificity.

The invention also encompasses sequences exhibiting deviations from the sequences represented by SEQ ID NOs 1 to 31 recited above. Deviations from the sequences represented by SEQ ID NOs 1 to 31 recited above may be amino acids replacements e.g. conservative amino acid replacements (e.g. glutamate to aspartate E to D, glutamine to asparagine Q to N, phenylalanine to tyrosine F to Y, leucine to isoleucine L to I). Deviations from the sequences represented by SEQ ID NOs 1 to 31 recited above may also be deletions or additions of one or more amino acids. Such deletions or additions may either occur at the N- or C-terminus of the sequences or may occur internally in the sequences. A condition for the antigenic properties of the polypeptides according to the invention is the preservation of the disulfide bridge that is formed, e.g. between cystein residues at amino acid positions 3 and 34 of DENV-1 ED3 (SEQ ID NO: 3). This disulfide bridge is found also in e.g. all other DENV serotypes and the flaviviruses WNV, JEV, YFV and TBEV referred to herein.

In this connection, it is preferred if the ED3 sequence, for example from DENV-1, starts with the amino acid preceding the c-terminal cystein residue involved in the formation of the disulfide bridge (SEQ ID NO. 3, position 2-3) and ends with the amino acid motif GS (SEQ ID NO. 3, position 96-97).

In addition ED3 antigen sequence can be extended into the stem region. For example, a preferred DENV-1 ED3S sequence will start with the amino acid preceding the c-terminal cystein residue involved in the formation of the disulfide bridge (SEQ ID NO. 2, position 2-3) and end at the ILG amino acid motif (SEQ ID NO. 2, position 115-117).

In one embodiment, the amino acid sequence is at least 75%, or at least 85%, or at least 90%, or at least 95%, or at least 98% identical to the sequence selected from the group consisting of SEQ ID NO: 1, 5, 9 or 13, from the group consisting of SEQ ID NO: 2, 6, 10 and 14, from the group consisting of SEQ ID NO: 3, 4, 7, 8, 11, 12 or 15 to 23 or from the group consisting of SEQ ID NO: 24 to 31.

In another embodiment, the polypeptide comprises an amino acid sequence selected from the group consisting of the sequences indicated by their accession numbers in table 1:

**Table 1**

| **Species** | **Genbank Accession** | **Strain** |
|---|---|---|
| DENV-1 | U88535 | Nauru Island, Western Pacific |
| DENV-2 | GQ868591 | DENV-2/TH/BID-V3357/1964 |
| DENV-3 | M93130 | H87 |
| DENV-4 | GQ868594 | DENV-4/PH/BID-V3361/1956 |
| WNV | M12294 | Wengler |
| JEV | M55506 | SA-14 |
| YFV | JN628279 | 17D |
| TBEV | JQ650523 | Western Subtype |

Even though the polypeptides of the invention can be obtained or isolated from virus samples, preparation using genetic engineering techniques are preferred. For example, in certain embodiments of the invention, the nucleic acid sequences encoding the flavivirus envelope ED3 and ED3S comprising polypeptide may be expressed in host cells, such as E. coli, yeast, plant, animal or human cell lines or algae. The polypeptides are either expressed as such, i.e. as isolated polypeptides, or as fusion proteins, such as fusion proteins with maltose-binding-protein (MBP). The ED3 and ED3S comprising polypeptides can also be fused to other protein carriers like GST (glutathione-S-transferase) or for example ß-galactosidase from E. coli or yeast. Using ED3 and ED3S comprising polypeptides as i.e. MBP fusion proteins, or bearing certain tags that facilitate isolation and purification, such as His-tagged, flag-tagged, myc-tagged, strep-tagged or any other antibody or substrate specific amino acid sequences attached to the ED3 or ED3S comprising antigens can be used. It needs to be considered, however, that GST and His₆₋tagged proteins show crossreactivities. The dengue endemic regions overlap with the occurrence of the malaria pathogen *P. falciparum* and *Schistosoma* infection also occur frequently in these areas. Therefore, glutathione-S-transferase from *Schistosoma japonicum* and His₆, part of the histidine-rich *P. falciparum* antigen (Wellems & Howard, 1986) are not recommended as fusion carriers to generate recombinant antigens to test human serum samples collected in DENV endemic regions.

According to another embodiment, the polypeptides according to the invention, alternatively or in addition to the above, can be linked to a reporter-molecule. A reporter molecule, within the scope of the present invention, is understood to be a molecule that allows direct or indirect detection of either the absence or presence of the polypeptide it is linked to, or the absence or presence of an antibody bound thereto. Many kinds of reporter molecules are known in the art, including for example radioactive labels, fluorescent dyes or proteins (e.g. fluorescine, tetramethylrodamine, green fluorescent protein (GFP)), haptenes (e.g. biotin) or enzymes (e.g. alpha-galactosidase A, luciferase, alkaline phosphatase or horseradish peroxidase, suitable for detection using enzyme convertible dyes). Such reporter molecules may be added to the target-protein either during protein synthesis (inclusion of radioactively labeled amino acids, generation of fusion proteins) or after protein synthesis by chemical coupling.

The invention also relates to various uses and methods for detecting a flavivirus infection in a subject.

Consequently, the invention also relates to the polypeptide of the invention for use in a method of diagnosis of a flavivirus infection in a subject. The flavivirus infection comprises an infection with any virus belonging to the flavivirus genus. In the context of the invention described herein, the flavivirus includes but is not limited to the following flavivirus species:

Tick-borne viruses, including but not limited to mammalian tick-borne virus group: including but not limited to *Alkhurma virus* (ALKV), *Deer tick virus* (DT), *Gadgets Gully virus* (GGYV), *Kadam virus* (KADV), *Karshi virus, Kyasanur Forest disease virus* (KFDV), *Langat virus* (LGTV), *Louping ill virus* (LIV), *Omsk Hemorrhagic fever virus* (OHFV), *Powassan virus* (POWV), *Royal Farm virus* (RFV), *Tick-borne encephalitis virus* (TBEV), *Turkish sheep encephalitis virus* (TSE), Seabird tick-borne virus group, *Meaban virus* (MEAV), *Saumarez Reef virus* (SREV), *Tyuleniy virus* (TYUV);
Mosquito-borne viruses, including but not limited to *Aedes flavivirus, Barkedji virus, Calbertado virus, Cell fusing agent virus, Chaoyang virus, Culex flavivirus, Culex theileri flavivirus, Donggang virus, Kamiti River virus, Lammi virus, Nakiwogo virus, Nounane virus, Quang Binh virus;* Aroa virus group: including but not limited to *Aroa virus* (AROAV); Dengue virus group: including but not limited to *Dengue virus* (DENV), *Kedougou virus* (KEDV), Japanese encephalitis virus group, *Bussuquara virus, Cacipacore virus* (CPCV), *Koutango virus* (KOUV), *Ilheus virus* (ILHV), *Japanese encephalitis virus* (JEV), *Murray Valley encephalitis virus* (MVEV), *Rocio virus* (ROCV), *St. Louis encephalitis virus* (SLEV), *Usutu virus* (USUV), *West Nile virus* (WNV), *Yaounde virus* (YAOV); Kokobera virus group: including but not limited to *Kokobera virus* (KOKV); Ntaya virus group: including but not limited to *Bagaza virus* (BAGV), *Duck egg drop syndrome virus* (BYDV), *Ilheus virus* (ILHV), *Jiangsu virus* (JSV), *Israel turkey meningoencephalomyelitis virus* (ITV), *Ntaya virus* (NTAV), *Tembusu virus* (TMUV); Spondweni virus group: including but not limited to *Zika virus* (ZIKV); Yellow fever virus group: including but not limited to *Banzi virus* (BANV), *Bouboui virus* (BOUV), *Edge Hill virus* (EHV), *Jugra virus* (JUGV), *Saboya virus* (SABV), *Sepik virus* (SEPV), *Uganda S virus* (UGSV), *Wesselsbron virus* (WESSV), *Yellow fever virus* (YFV);
Viruses with no known arthropod vector, including but not limited to Entebbe virus group: including but not limited to *Entebbe bat virus* (ENTV), *Yokose virus* (YOKV); Modoc virus group: including but not limited to *Apoi virus* (APOIV), *Cowbone Ridge virus* (CRV), *Jutiapa virus* (JUTV), *Modoc virus* (MODV), *Sal Vieja virus* (SVV), *San Perlita virus* (SPV); Rio Bravo virus group: including but not limited to *Bukalasa bat virus* (BBV), *Carey Island virus* (CIV), *Dakar bat virus* (DBV), *Montana myotis leukoencephalitis virus* (MMLV), *Phnom Penh bat virus* (PPBV), *Rio Bravo virus* (RBV).

In a preferred embodiment, the flavivirus is Japanese encephalitis virus, West Nile virus, dengue virus, tick-borne encephalitis virus, or yellow fever virus, most preferably dengue virus. An important task is the differentiation of dengue virus infection and other flavivirus infections that coexist in endemic areas. For example DENV-1-4 are present in Cambodia together with Japanese encephalitis virus. Thus, DENV infections must be differentiated preferentially from infections by JEV, WNV, YFV and TBEV.

"Flavivirus envelope protein domain III" as used herein relates to domain III (domain 3, or "ED3") of the virus-coded glycoprotein designated E that is integrated into the flavivirus membrane, which - together with the glycoprotein designated M - is involved in facilitating cell attachment, receptor binding and virus-cell membrane fusion. The details of DENV M and E protein for viral entry are well studied and described for example in the standard virology textbook: Fields Virology 6th Edition (Lippincott Williams & Wilkins, 2013). A review is published by Heinz and Stiasny (Flaviviruses and their antigenic structure, J. Clin Virol. 2012 55:289-95). Both of these references are incorporated herein by reference.

In a preferred embodiment, the polypeptide of the invention is for use in detecting a flavivirus infection in a subject, wherein the flavivirus is West Nile virus, Japanese encephalitis virus, dengue virus, tick-borne encephalitis virus, or yellow fever virus. A dengue virus infection includes infection with dengue virus serotypes 1, 2, 3 and/or 4 (DENV-1, DENV-2, DENV-3 and/or DENV-4) as well as the new proposed serotype DENV-5. Accordingly, according to another preferred embodiment, the polypeptide of the invention is for use in a method of diagnosis of a dengue virus infection, wherein the use comprises specific detection of the dengue virus serotype. According to a further embodiment of the invention, the dengue virus serotype to be detected by the use is one or more, preferably all of serotype 1, serotype 2, serotype 3 and serotype 4. According to a further embodiment, the dengue virus serotype to be detected by the use also includes the newly proposed serotype 5.

The invention also relates to a method for determining or detecting a flavivirus infection in a subject, wherein the method comprises contacting a sample from the subject with a polypeptide of the invention, and detecting binding of an antibody from the sample to the polypeptide.

In one embodiment, the sample is contacted with antigens from a variety of flaviviruses in parallel. In particular, the sample may be contacted with antigens, i.e. the polypeptides of the invention, representing DENV, JEV, WNV, YFV and TBEV. With regard to DENV, the sample may be contacted with antigens, i.e. the polypeptides of the invention, representing more than one, preferably all DENV serotypes 1 to 4 as well as the new proposed serotype DENV-5.

In one embodiment of the method, the sample is contacted with at least two, at least three, at least four, at least five or more polypeptide antigens, respectively, per flaviviral strain or, in case of DENV, per serotype. These polypeptides, for example, may correspond to the sequences according to SEQ ID NOs 1 to 31, they may, however, also represent sequence variants of the ED3 and ED3S amino acid sequences provided herein, e.g. different genotypes of the species or serotypes referred to herein above. In this case, it is preferred if the sequence variants have at least 65%, at least 75%, or at least 85%, or at least 90%, or at least 95%, or at least 98% sequence identity with the sequences of SEQ ID NOs 1 to 31 described herein.

Alternatively, or in addition, the sample is contacted with sets of the respective polypeptide antigens of ED3* and/or ED3S* wherein each member of a set is denatured by a different denaturing protocol described before (heat, SDS and/or acids etc.).

According to a further embodiment, the binding of an antibody to the polypeptide is detected by an immunofluorescence test, a protein microarray, an enzyme linked immunosorbent assay (ELISA), a luminescence test, a radioimmune test, such as a radioimmunoassay (RIA), immunoradiometric assay, fluoroimmunoassay, chemiluminescent assay, bioluminescent assay, a blot, such as a Western blot or a dot blot. The antigen can be coupled to surfaces of membranes, biosensors, or bead surfaces for the detection of antibodies by, for example, surface resonance spectroscopy or bead-based multiplexing. Since the assay is based on the testing of different flavivirus ED3 and/or ED3S polypeptides, multiplex methods based on beads, for example color coded beads for Luminex applications, or surface-based test-strip or chip applications are preferred methods for antibody detection. These methods and assays are well known to those of ordinary skill. The steps of various useful immunodetection methods have been described in the scientific literature, such as, *e.g.,* Doolittle *et al.,* 1999; Gulbis *et al.,* 1993; DeJager *et al.,* 1993; and Nakamura *et al.,* 1987, each incorporated herein by reference.

In some embodiments of the ELISA assay, a flavivirus ED3 comprising polypeptide of the invention or at least two flavivirus ED3 comprising polypeptides of the invention are immobilized onto a selected surface, preferably a surface exhibiting a protein affinity such as the wells of a polystyrene microtiter plate. After washing to remove incompletely adsorbed material, one will desire to bind or coat a nonspecific protein such as bovine serum albumin (BSA), casein, solutions of milk powder, gelatin, PVP, superblock, or horse albumin onto the well that is known to be antigenically neutral with regard to the test antisera. This allows for blocking of nonspecific adsorption sites on the immobilizing surface and thus reduces the background caused by nonspecific binding of antisera onto the surface. Following an appropriate coating period (for example, 3 hours), the coated wells will be blocked with a suitable protein, such as bovine serum albumin (BSA), casein, solutions of milk powder, gelatin, PVP, superblock, or horse albumin, and rinsed several times (e.g., 4 or 5 times) with a suitable buffer, such as PES. The wells of the plates may then be allowed to dry, or may instead be used while they are still wet.

After binding of antigenic material to the well, coating with a non-reactive material to reduce background, and washing to remove unbound material, the immobilizing surface is contacted with the antisera or clinical or biological extract to be tested in a manner conducive to immune complex (antigen/antibody) formation. Such conditions preferably include diluting the antisera with diluents such as BSA, bovine gamma globulin (BGG) and phosphate buffered saline (PBS)/Tween. These added agents also tend to assist in the reduction of nonspecific background. The layered antisera are then allowed to incubate for from 1 to 4 hours, at temperatures preferably on the order of 20° to 25°C. Following incubation, the antisera-contacted surface is washed so as to remove non-immunocomplexed material. A preferred washing procedure includes washing with a solution such as PBS/Tween, or borate buffer.

Following formation of specific immunocomplexes between the test sample and the bound antigen, and subsequent washing, the occurrence and even amount of immunocomplex formation may be determined by subjecting same to a second antibody having specificity for the first. Of course, in that the test sample will typically be of human origin, the second antibody will preferably be an antibody having specificity in general for human IgG, IgM or IgA. To provide a detecting means, the second antibody will preferably have an associated enzyme that will generate a color development upon incubating with an appropriate chromogenic substrate.

Thus, for example, one will desire to contact and incubate the antisera-bound surface with a urease, alkaline phosphatase, or peroxidase-conjugated anti-human IgG for a period of time and under conditions which favor the development of immunocomplex formation (e.g., incubation for about 2 hours at room temperature in a PBS-containing solution such as PBS-Tween).

After incubation with the second enzyme-tagged antibody, and subsequent to washing to remove unbound material, the amount of label is quantified by incubation with a chromogenic
substrate such as urea and bromocresol purple or 2,2'-azino-di-(3-ethylbenzthiazoline-6-sulfonic acid (ABTS) and H₂O₂, in the case of peroxidase as the enzyme label. Quantification is then achieved by measuring the degree of color generation, e.g., using a visible spectra spectrophotometer.

In an exemplary embodiment, in each of the microtiter wells will be placed about 10 µl of the test patient sample along with about 90 µl of reaction buffer (*e.g.,* PBS with about 1 % digitonin or other mild protein solubilizing agent). Control wells of the ELISA plate will include normal sera (human sera without flavivirus antibody), and anti-flavivirus antibody collected from subjects.

Irrespective of the format employed, ELISAs have certain features in common, such as coating, incubating and binding, washing to remove non-specifically bound species, and detecting the bound immune complexes. These are described below.

In coating a plate with either antigen or antibody, one will generally incubate the wells of the plate with a solution of the antigen or antibody, either overnight or for a specified period of hours. The wells of the plate will then be washed to remove incompletely adsorbed material. Any remaining available surfaces of the wells are then "coated" with a nonspecific protein that is antigenically neutral with regard to the test antisera. These include bovine serum albumin (BSA), casein or solutions of milk powder. The coating allows for blocking of nonspecific adsorption sites on the immobilizing surface and thus reduces the background caused by nonspecific binding of antisera onto the surface.

In ELISAs, it is probably more customary to use a secondary or tertiary detection means rather than a direct procedure. Thus, after binding of a protein or antibody to the well, coating with a non-reactive material to reduce background, and washing to remove unbound material, the immobilizing surface is contacted with the biological sample to be tested under conditions effective to allow immune complex (antigen/antibody) formation. Detection of the immune complex then requires a labeled secondary binding ligand or antibody, and a secondary binding ligand or antibody in conjunction with a labeled tertiary antibody or a third binding ligand.

"Under conditions effective to allow immune complex (antigen/antibody) formation" means that the conditions preferably include diluting the antigens and/or antibodies with solutions such as BSA, bovine gamma globulin (BGG) or phosphate buffered saline (PBS)/Tween. These added agents also tend to assist in the reduction of nonspecific background. The "suitable" conditions also mean that the incubation is at a temperature or for a period of time sufficient to allow effective binding. Incubation steps are typically from about 1 to about 2 to 4 hours or so, at temperatures preferably on the order of 25°C to 27°C or may be overnight at about 4°C or so.

Following all incubation steps in an ELISA, the contacted surface is washed so as to remove non-complexed material. An example of a washing procedure includes washing with a solution such as PBS/Tween, or borate buffer. Following the formation of specific immune complexes between the test sample and the originally bound material, and subsequent washing, the occurrence of even minute amounts of immune complexes may be determined.

To provide a detecting means, the second or third antibody will have an associated label to allow detection. This may be an enzyme that will generate color development upon incubating with an appropriate chromogenic substrate. Thus, for example, one will desire to contact or incubate the first and second immune complex with a urease, glucose oxidase, alkaline phosphatase or hydrogen peroxidase-conjugated antibody for a period of time and under conditions that favor the development of further immune complex formation (e.g., incubation for 2 hours at room temperature in a PBS-containing solution such as PBS-Tween).

After incubation with the labeled antibody, and subsequent to washing to remove unbound material, the amount of label is quantified, *e.g*., by incubation with a chromogenic substrate such as urea, or bromocresol purple, or 2,2'-azino-di-(3-ethyl-benzthiazoline-6-sulfonic acid (ABTS), or H₂O₂, in the case of peroxidase as the enzyme label. Quantification is then achieved by measuring the degree of color generated, *e.g*., using a visible spectra spectrophotometer.

In other embodiments, the method or assay may be performed using a blot technique. The flavivirus ED3* and ED3S* comprising polypeptide antigens are transferred to a binding surface such as nitrocellulose, nylon, glass-fiber or polyvinylidenfluorid (PVDF) membrane. After antigen binding, the membrane is incubated with a blocking solution, containing for example bovine serum albumin, milk powder or other blocking reagents that prevent further binding of proteins to the membrane. After blocking the membrane is incubated with a buffer such as phosphate buffered or Tris-HCl buffered saline containing reagents for example 0,05% Tween 20 Triton-X100 or NP40. The buffer can also contain the blocking reagent. The test sample, such as plasma or serum or any other antibody containing sample is diluted into this buffer at 1:10, 1:100, 1:1000 or higher dilutions and the test membrane is incubated with this test solution for at least 30 min, preferentially 1h at RT. After binding of the first antibody, the test membrane is washed at least once in buffer and incubated with the secondary antibody, an anti-antibody specifically directed against the first antibody. The second antibody is for example an antibody specific against human IgG but can also be an antibody against IgM or any other class of antibody. The secondary antibody is labeled with an enzyme, for example alkaline phosphatase or horseradish peroxidase. After binding of the secondary antibody, the test membrane is washed again in phosphate or Tris buffered saline or any other appropriate buffer and the bound antibody is detected by staining using the activity of the enzymes linked to the secondary antibody.

In other embodiments the method or assay may be performed using a bead based multiplex technique. Here, the antigens are covalently linked to the surface of microbeads by standard chemical procedures, for example the NHS/carbodiimide method. For the multiplex analysis beads are used that, for example are stained differentially thus in a single reaction up to 100 different flavivirus antigen preparations can be tested.

The bead-based multiplex technology, also known as Luminex method, uses existing technologies which is in principle a combination of flow cytometry, microspheres, lasers, digital signal processing and traditional chemistry (Cen H et al., 2013; Christopher-Hennings J et al., 2013)

Color-coded beads, called microspheres, can be stained to generate 100 distinct bead sets that can be separated each by standard flow cytometry because of their unique color, for example, 100 different shades of red. Each bead set that has one of the 100 unique colors can be coated with one of the ED3* or ED3S* comprising antigens or with the native forms and the denaturing of the ED3 and/or ED3S comprising antigens is performed after coupling to the beads. Thus, a single antigen is bound to a specific bead and can be separated because of its unique color. Thus all the antigen preparations for all the flaviviruses can be easily combined in such a bead-based multiplex assay.

After preparation of the antigen-specific bead sets the bead sets are mixed together to generate a bead mixture representing all the antigens in one mixture. This bead-antigen mixture is in principle handled according to the methodology of the ELISA technique. Unspecific binding to the beads is blocked by i.e. bovine serum albumin and the beads are incubated with the test sample, serum, plasma or any other antibody containing sample, to allow antibody binding to the various antigens. After antibody binding the beads are washed and bound antibody is marked by a secondary anti-antibody that is marked by a fluorescence label, providing a second fluorescent color (i.e. green) that allows the measurement of the amount of bound test antibody. After washing, the bead mixture is separated based on the color of the beads (i.e. 100 different red tones) and the amount of bound antibody is measured by the second fluorescent staining (i.e. green) that was generated by the for example FITC-conjugated secondary anti-IgG and/or IgM antibody.

Within the bead analyzer, lasers excite the different red tones of the 100 different bead sets that identify each microsphere particle, and also any reporter dye captured by the bound antibody during the assay. Using this approach, the bead-based multiplex technology allows multiplexing of up to 100 unique antigen assays within a single sample.

In other embodiments the method or assay may be performed using a method that is based on the capturing of immune complexes. Therefore the ED3* and ED3S* flavivirus antigens will be labeled with a specific marker that can be a fused tag identical to the tag which was used for antigen purification from E. coli such as the MBP protein. The purified antigens can also be labeled by other tags that are specific for antibodies like the flag-tag or by enzymes like alkaline phosphatase or by DNA molecules to allow detection of isolated immune complexes. Test samples can be used unmodified but are preferentially pre-heated to 58°C, 60°C, or 65°C to destroy immune complexes present in the test sample and also to destroy immune complex-building factors (for example C1q, IgM-RF or CD32) in the test sample. Immune complexes will be formed by the labeled antigen and the test antibody present in serum, plasma or any other antibody containing samples in standard buffer solutions like PBS or tris-buffered saline. ED3* and/or ED3S* antigen test antibody formed immune complexes are isolated specifically by proteins that bind preferentially to the FC part of antibodies that are bound to an antigen (ED3* and/or ED3S*). Here, the preferred protein to capture the formed immune complexes is C1q that specifically binds to the FC part of the test antibody that is part of the formed immune complex. Non-bound antibodies do not bind to C1q. C1q can be used on surfaces, for example plastic plates that are engineered for ELISA techniques, plastic beads, membranes like PVDF or nitrocellulose, glass or activated dextran surfaces. The test sample containing the immune complexes are given for example to the well of a C1q coated 96-well microplate. After incubation for at least 1h at RT the plates are washed and the isolated immune complex is detected by its fused label. The inventors have used an anti-MBP antibody to detect the MBP-ED3* antigen in the isolated immune complex. In principle C1q can be replaced by any other protein that specifically binds to antibodies which are part of an immune complex, for example the human reumatoid factor or CD32.

As described above, the method of the invention a method for determining or detecting a flavivirus infection. The flavivirus infection is an infection with a flavivirus, more particularly with a flavivirus species as described above. In a preferred embodiment, the flavivirus is West Nile virus, dengue virus, tick-borne encephalitis virus, or yellow fever virus. Specifically, the method relates to detecting dengue virus infection, wherein the dengue virus infection includes infection with dengue virus serotypes 1, 2, 3 and/or 4 and the proposed new DENV-5 serotype.

The invention also provides means for performing the method of the invention, such as compositions and kits that are specifically designed for performing said detection method.

In one embodiment, the invention relates to a composition comprising the polypeptide of the invention linked or attached to a solid phase, such as a substrate, preferably a microtiter plate, beads, a strip, such as a nitrocellulose or PVDF membrane, glass plates, activated dextran surfaces, or color-coded microspheres.

In a specific embodiment, the composition that is linked or attached to a solid phase comprises at least two polypeptides of the invention, wherein the polypeptides are sequence variants of the amino acid sequence of SEQ ID NO: 1, 5, 9 and 13 (*E-protein partial sequence; N-terminally truncated*), of SEQ ID NO: 2, 6, 10 and 14 (*ED3S; ED3 domain and stem region*), of SEQ ID NO: 3, 4, 7, 8, 11, 12 and 15 to 23 (*ED3; ED3 domain specific clone and consensus sequence*) or of SEQ ID NO: 24 to 31 (*E-protein full sequence*), wherein said polypeptide is denatured. More specifically, the polypeptides comprise an amino acid sequence that is at least 65%, at least 75%, or at least 85%, or at least 90%, or at least 95%, or at least 98% identical to the sequence selected from the group of SEQ ID NO: 1, 5, 9 and 13 (*E-protein partial sequence; N-terminally truncated*), from the group of SEQ ID NO: 2, 6, 10 and 14 (*ED3S; ED3 domain and stem region*), from the group of SEQ ID NO: 3, 4, 7, 8, 11, 12 and 15 to 23 (*ED3; ED3 domain specific clone and consensus sequence*) or from the group of SEQ ID NO: 24 to 31 (*E-protein full sequence*), wherein said polypeptide is denatured.

The invention also relates to a kit for use in a method of the invention, wherein the kit comprises the polypeptide of the invention or the composition of the invention, optionally with further reagents and means for performing said method.

In a specific embodiment, the kit comprises at least two polypeptides of the invention or at least two compositions of the invention, wherein the polypeptides are sequence variants of the amino acid sequence of SEQ ID NO: 1, 5, 9 and 13 (*E-protein partial sequence; N-terminally truncated*), of SEQ ID NO: 2, 6, 10 and 14 (*ED3S; ED3 domain and stem region*), of SEQ ID NO: 3, 4, 7, 8, 11, 12 and 15 to 23 (*ED3; ED3 domain specific clone and consensus sequence*), or of SEQ ID NO: 24 to 31 (*E-protein full sequence*), wherein said polypeptide is denatured. More specifically, the polypeptides comprise an amino acid sequence that is at least 65%, at least 75%, or at least 85%, or at least 90%, or at least 95%, or at least 98% identical to the sequence selected from the group of SEQ ID NO: 1, 5, 9 and 13 (*E-protein partial sequence; N-terminally truncated*), from the group of SEQ ID NO: 2, 6, 10 and 14 (*ED3S; ED3 domain and stem region*), from the group of SEQ ID NO: 3, 4, 7, 8, 11, 12 and 15 to 23 (*ED3; ED3 domain specific clone and consensus sequence*), or from the group of SEQ ID NO: 24 to 31 (*E-protein full sequence*), wherein said polypeptide is denatured.

In certain embodiments, the kit comprises an assay plate comprising a multiplicity of microtiter wells that comprise the polypeptide of the invention or the composition of the invention, or that comprise at least two polypeptides of the invention or at least two compositions of the invention; and a container means comprising a labeled secondary antibody having specific binding affinity for a flavivirus antibody in the sample.

For DENV serology, the most important domain is the ED3 domain. Most studies rely on the presentation of ED3 in its most perfect conformation to distinguish between DENV serotypes. That ED3 is presented in a perfect conformation was demonstrated by accessibility of the ED3 to mAb or sera from vaccinated animals. Other studies used classical approaches like the Western blot technique to study antibody responses to ED3 (Ludolfs et al., 2002). Altogether, the use of ED3-derived antigens to distinguish between the DENV-serotypes by an antibody test, its specificity and its applicability seems to be controversial (Zidane et al., 2013) and needs further clarification.

In the context of the present invention, the ED3 antigen was denatured in various ways to enhance the specificity of the ED3* antigen. One of the inventors' surprising observations was that the ED3 construct ED3-388 was negative in screening experiments indicating that the little C-terminal part (amino acid position 389-399, Fig. 2) of the ED3 domain is responsible for the building of the serotype specific epitope of ED3* and ED3S* denatured antigens. Based on this observation, further constructs were provided where the amino acid sequence was elongated from the ED3 into the stem region. The fusion of parts of the stem region onto the ED3 domain, lead to an antigen (ED3S) with a higher serotype-specific reactivity. Such a serotype specificity was not seen with the ED3 antigen (ED3-399, Fig. 2) itself.

The following ED3-stem (ED3S) constructs were prepared (the numbering corresponding to the numbering respectively following from Fig. 2):
DENV-1 amino acid 302-410, 302-420, 302-429, 302-435, 302-443
DENV-2 amino acid 300-410, 300-420, 300-429, 300-435, 300-443;
DENV-3 amino acid 299-410, 299-420, 299-429, 299-435, 299-443; and
DENV-4 amino acid 302-410. 302-420, 302-429, 302-435, 302-443

In a preferred embodiment, an ED3-stem construct is provided which starts at amino acid position 302 (DENV-1, DENV-4), 300 (DENV-2), 299 (DENV-3) according to the numbering of DENV sequences shown in figure 2 of ED3 and ends with the amino acid glycine at amino acid position 420 (Fig. 2). This polypeptide of the invention shows a very high serotype-specific response. Without to be bound by theory, it is believed that one reason for its higher specificity could be that the fused amino acids are in part responsible for stabilizing the ED3 serotype specific epitope that is more common to the natural conformation during infection (Klein et al., 2013) and therefore is more qualified as a diagnostic antigen. It is also conceivable that parts of ED3, representing for example a cross-reactive epitope, are covered by the stem amino acids preventing the binding of the cross-reactive antibodies.

On the other hand, one has to look on immune responses to other viral envelope proteins. Proteins in general are assembled by domains and such a domain can induce antibodies to a linear cross-reactive and at the same time to a for example highly specific discontinuous epitope. These antigenic epitopes can be both located on the same molecule, the same envelope domain. For example, in HIV-1 infection the antibody response is directed to the coreceptor binding region of gp120, the so-called V3 loop. Against the conserved linear V3 loop amino acid sequence GPGRAF antibodies can be detected. These antibodies are sequence specific and are used to determine the HIV-1 subtype B from other HIV-1 subtypes. Unfortunately, the antibodies directed to this conserved linear epitope have only weak or no HIV-1 neutralizing potential. In contrast, the antibody response that represents highly potent neutralizing antibodies is directed to an epitope that is only present when the V3 loop is in its original conformational structure, stabilized by the Cys-Cys bridge (Schreiber et al., 1997). It is therefore plausible and generally accepted that also in natural DENV infection a E protein domain, i.e. the ED3 domain, will induce antibodies to highly complex conformational and normal linear epitopes at the same time. The denaturing of the ED3 and ED3S comprising polypeptides, e.g. by heat and/or SDS and/or acid treatment, destroys antigenic epitopes and therefore cross-reactive antibodies do not bind as efficiently as they bind to the native polypeptide. The importance of this finding is that there seems to be a hidden and actually unknown epitope in the ED3 domain that is exposed and accessible in the denatured form of the ED3 or ED3S comprising antigen. It is furthermore plausible that this serotype specific epitope can be transferred into the genetically background of a non-DENV ED3 domain that is antigenic silent, for example the USUTU virus ED3 domain, given a even more specific and sensitive antigen for DENV serotyping. Thus, chimeras of DENV-ED3 and ED3 domains from other flaviviruses not cross-reactive or immunological silent can be used as a background to express the DENV serotype specific epitopes for a further enhancement of the DENV serotyping assay.

The initial screening experiments using DENV antibody positive sera from a study in Vietnam showed that about 50 % of the sera were not reactive with ED3*. This might be because the antigen used in the first studies was treated with 2% SDS at 100°C which is a very strong denaturing condition. DENV-2 and DENV-4 ED3 antigen was more reactive using denaturing conditions with 0.1% SDS and 70°C for 10 min. Under these conditions serotyping for DENV-2 and DENV-4 was more efficient, thus, for each of the DENV serotypes, the denaturation of the MBP-ED3 fusion protein has to be adjusted to achieve the most sensitive assay results. DENV-1 and DENV-3 ED3* was generated by high SDS concentrations and high temperatures (2% SDS, 100°C). The preferred denaturing conditions for DENV-2 and DENV-4 ED3 was 0,1% SDS and 70°C. Under these conditions, serotyping was possible in more than 90 % of sera tested.

Detection of serotype specific antibodies is dependent on their development during the infection. Generally this needs several days or even weeks after the primary infection. Thus, in serum samples from patients that are infected by DENV for the first time and the serum sample was taken in the early acute phase of the infection, the presence of serotype specific antibodies is questionable. Therefore, sera from primary infected patients can show negative results since the serotype response was not created in the early stage. Sera from secondary infections show at least the serotype specific responses that was originally induced by the primary infection or the humoral response that was created against the new DENV. Thus serotyping in the acute phase is not always correlated to the virus present but gives an actual status on the specificity on the immune response that is present at the time of sampling.

The assay will be of benefit for studies outside of the time period were virus or virus antigen can be detected, the later stages of the disease, and in surveillance studies and studies that will detect asymptomatic infections. In the acute phase of a primary DENV infection the antibody response to the E glycoprotein is highly cross-reactive at the beginning (Lai et al., 2008) and is rendered to serotype-specificity after at least 6-8 weeks. Therefore, it is difficult to detect such serotype-specific responses in all of the patients with primary infection during the early stage of dengue infection.

In one embodiment, to generate the most specific diagnostic tool, it will be necessary to use more than one, preferably the ED3* comprising antigens which actually circulate in the endemic regions to generate a highly specific and sensitive ED3 diagnostic antigen array.

In the initial testing ED3 antigens based on DENV sequences from strains in the inventor's laboratory were used. In the last years the technique of RT-PCR and the sampling methods for DENV has significantly improved and virus isolates and sequences thereof are available. Thus, the DENV isolates that circulate in the endemic areas are well known now. These data have changed the historical picture of DENV antigenic variation. Each serotype of DENV is now described as a set of distinct genotypes (Rico-Hesse, 2003). It is conceivable that the changes seen in DENV variation also affect any assay that will detect antibodies to a specific epitope in the for example M or E envelopes. Variation of E for example affects ED1, ED2 and the ED3 domain and amino acid exchanges in ED3 can lead to viral escape from neutralizing antibodies as shown by Wahala et al. (2010).
The relevance for the serotype assay is that its high specificity for an ED3 sequence might be the reason why not all sera from Vietnam reacted positively to ED3* or ED3S. Thus, non-reactivity to only one ED3* antigen for one of the four DENV serotypes can be the result of antigenic variation of the ED3 sequence in the DENV that originally had infected the patient. Therefore, in a specific embodiment, the assay will be extended in such a way that sequences of ED3 antigens from DENVs from endemic areas will be used to complete the set of antigens. A multi-antigen assay using a set of ED3 antigens that reflects the antigenic diversity of the local DNV population will be a more suited tool to detect serotype or genotype specific antibodies in patients. As demonstrated a highly anti-ED3 specific antibody response is developed after a period of 6-8 weeks after the initial infection (Oliphant et al., 2007) and will therefore be detectable in all dengue infected individuals. One additional feature of the assay is that serotype specific antibodies can also be detected in people not developing any severe sign of symptoms. These so-called asymptomatic carriers might play an important role, the so-called "silent" virus reservoir that allows DENV to survive and to initialize epidemics under certain conditions.

In a preferred embodiment of the invention, the method for detecting a flavivirus infection in a subject, preferably a DENV infection in a subject, is performed using the so-called bead based multiplex technology, which measures multiple antigens simultaneously in a single reaction vessel. Such a technology will be used to measure antibody responses to a larger antigen array of ED3S* and ED3* comprising peptides to obtain a more detailed and accurate picture of the serotype-specific antibody pattern present in patients, asymptomatic carriers and vaccinated individuals. In one specific embodiment, the set of DENV ED3* comprising antigens will be adapted to the continuously growing set of sequence data on the genetic diversity of DENVs found in humans and the mosquito host (Thaia et al., 2012).

According to the present invention, serotype-specific anti-ED3 responses can be detected in sera from DENV-infected patients by using different preparations of recombinant ED3 comprising antigens. The antigens can be produced in form of fusion proteins, for example as fusion with MBP or tagged by a poly-His tail or a sequence specific for a serum or monoclonal antibody as for example the flag-tag epitope. Another form of the ED3 comprising antigen can be as part of a chimera in the context of soluble E protein with ED1 and ED2 from a different, preferentially immunosilent, E protein variant. The ED3 comprising antigen can also be prepared in four of multimers. Multimers of one of the ED3 comprising antigens, a DENV-1 ED3 comprising multimer, or in form hetero-multimers, DENV-1-4 ED3 comprising multimers which then are denatured. The ED3 itself can be produced as a chimera with immunosilent region from other flavivirus ED3 sequences to minimize crossreactive antibody recognition and to maximize the DENV serotype reactivity of such an antigen.

In other specific embodiments of the invention, the polypeptide of the invention is an ED3 comprising construct which is a denaturized MBP fusion protein (designated as ED3*-MBP fusion protein), or a properly folded ED3S-MBP fusion protein or an ED3-MBP fusion protein. The ED3*-MBP fusion protein was more specific than the properly folded ED3S-MBP and the ED3-MBP fusion proteins.

Serotype-specific responses to ED3* were detected in 50 % of all Vietnam sera when using ED3*-MBP.

In the panel of the ED3* negative sera positive reactivity was observed for ED3 in about 20% and for ED3S in about 10% of the serum samples.

The invention will be further described in the following examples. These examples are included to demonstrate preferred embodiments of the invention. It should be appreciated by those of skill in the art that the techniques disclosed in the examples are not limiting the invention, and those of skill in the art should, in light of the present disclosure, appreciate that changes can be made in the specific embodiments which are disclosed and still obtain a like or similar result without departing from the spirit and scope of the invention.

### EXAMPLES

### EXAMPLE 1

### Serum samples

Sera were collected and characterized as described by Ludolfs et al., 2002. DENV negative sera were from the BNITM diagnostic department.

### EXAMPLE 2

### Dengue type-specific RT-PCR

RT-PCR was performed after viral RNA extraction from acute serum samples using QIAmp Viral RNA Mini Kit (Qiagen, Hilden, Germany). Either a conventional nested RT-PCR according to the protocol established by Lanciotti *et al.* (Lanciotti et al., 1992) and modified by Reynes *et al.* (Reynes et al., 2003) or a real-time multiplex RT-PCR based on the technique developed by Hue *et al.* (Hue et al., 2011) was performed.

### EXAMPLE 3

### IgM-capture ELISA (MAC-ELISA)

An in-house MAC-ELISA was used to detect anti-DENV and anti-Japanese Encephalitis virus (JEV) IgM as describe previously (Vong et al., 2010). A result was considered positive for dengue when the optical density (OD) was higher than 0.1 for the DENV IgM and when the OD of the anti-DENV ELISA was higher than the OD of the anti-JEV ELISA.

### EXAMPLE 4

### Hemagglutination-inhibition test (HAI)

HAI followed the method described by Clark and Casals (Clarke & Casals, 1958) adapted to 96-well microtiter plate. Primary or secondary acute dengue infection was determined by HAI titer according to criteria established by WHO (WHO/TRD, 1997). In brief, the patient was defined as having a primary infection when the convalescent serum had a HAI titer ≤ 2560 associated with a fourfold rise of the titer between the acute and convalescent sera (collected with a time interval of at least 7 days). When the convalescent serum had an HAI titer > 2560, the patient was defined as having a secondary dengue infection.

### EXAMPLE 5

### Antibody detection

Antibody to DENV was monitored by indirect immunofluorescence assay (IFA). In brief, virus was propagated in VeroB4 cells and infected cells were collected and washed with PBS. The cell suspension was added onto glass slides (teflon printed diagnostic slides, Immuno-Cell) and air dried. Completely dried cells layers were fixed to the slides by incubation in acetone at 4-10 °C for 20 min. After fixation the slides were dried at RT and were stored at -20 °C sealed in plastic foil. To detect antibody, in for example serum samples, the slides were washed in PBS and sera dilution (30 µl) was added to each area of the teflon printed diagnostic slides. Sera were incubated to the fixed cells for 1 h at 37 °C in a closed chamber of high humidity. After antibody binding, the glass slides were washed three times with PBS. To detect bound antibody, to each area on the teflon printed diagnostic slides a secondary antibody directed against the human antibody was added (30 µl, anti-human IgG FITC, 1:350 in PBS, Sifin). Glass slides were incubated with the secondary antibody for 20 min at 37 °C. After antibody binding the slides were washed three times with PBS and additionally stained with DAPI (50-200 µg/ml in methanol) for 15 min at 37 °C. Slides were washed again three times with PBS and were air dried prior to analysis by fluorescence microscopy.

### EXAMPLE 6

### Preparation of MBP-fusion proteins

Viral RNA was extracted from infected cell culture supernatant using QIAamp® Viral RNA Mini Kit (Qiagen). The extracted RNA was reverse-transcribed using Revert Aid™ H Minus M-MuLV Reverse Transcriptase (Fermentas) according to the manufacturer's instructions. The envelope protein of all four DENV serotypes, WNV, JEV and TBEV was amplified from cDNA with specific primers and Phusion® High-Fidelity DNA Polymerase (NEB). The forward primers were designed to introduce a BamHI restriction site at 5'-end and the reverse primers contained a Hind III restriction site at the 3'-end of the PCR product. After digestion with BamHI and HindIII the PCR DNA-fragment was cloned into pMALp4x vector (NEB). All cloned constructs were sequenced to verify the respective identity (LGC). All other constructs were generated from a previously cloned E protein envelope gene, kindly provided by Janne Hülsemann (BNI, Hamburg). *E. coli* Top10 cells (Invitrogen) were used for transformation. For expression of the MBP-fusion proteins, pMALp4x plasmids containing the insert of choice were transformed into *E. coli* BL21 DE3 (Promega) cells. A volume of 2.7 liter of LB-medium was inoculated 1:100 with an *E. coli* overnight culture and incubated at 37°C in a horizontal shaker (GFL, Germany) at 200 rpm. The MBP-expression was induced at 0.8 OD₆₀₀ by adding IPTG to a final concentration of 1 mM. The bacteria were harvested after four hours. Bacterial lysis was carried out by sonification at 0 °C and clear lysates were obtained after centrifugation (Eppendorf 5810R, FA-45-6-30, 4 °C, 15,000 g). The MBP-fusion proteins were purified from clear lysates using amylose resin affinity chromatography according to the manufacturer's recommendations (NEB). The protein was eluted with (20 mM Tris/HCl pH 7.4; 200 mM NaCl; 1 mM EDTA; 10 mM Maltose) and the resulting 1 ml fractions were analyzed on a 10 % SDS-PAGE. In general, the yield of purified DENV-MBP-fusion protein was between 7 and 20 mg per liter *E. coli* culture.

### EXAMPLE 7

### Immunoscreening

Patient's sera were diluted in blocking buffer (10 mM Tris/HCl pH 7.4; 150 mM NaCl; 0,1 % Tween20 containing 5 % low fat milk) and purified MBP was added to a final concentration of 75 µg/ml. To adsorb anti-MBP-antibodies in the patient serum samples, the test sera were incubated at 8-10 °C for 2h at RT or overnight at 8 °C. On the next day, purified MBP-fusion proteins solubilized in elution buffer (20 mM Tris/HCl pH 7.4; 200 mM NaCl; 1 mM EDTA; 10 mM Maltose) were transferred to a 394-well micro-plate and dotted on 2,7 mm wide and 115 mm long nitrocellulose strips by using a custom made 24-pin dot blotter with 20 mm long 1,2 mm round pins and 2,5 mm stainless steel ball endings. For each dot 0.5 µl of the antigen solution (1 mg/ml) was transferred to the nitrocellulose test strip. The test strips were transferred to a 30-well incubation tray (Viramed, Germany) and were incubated with blocking buffer for 1 hour at room temperature. The blocking buffer was discarded and 1 ml of the diluted serum was applied to each of the wells. The 30-well trays were incubated on a shaker for 2 hours at room temperature. Serum incubation was followed by three wash cycles, 5 min each. To detect bound human antibodies an anti-human IgG (Bio-Rad) antibody conjugated to horseradish peroxidase was used at a dilution of 1:1500 in blocking buffer. After 1 hour of incubation at RT the test strips were washed three times with TBST buffer (blocking buffer without low fat milk) followed by three wash cycles with TBS buffer (TBST without Tween 20) with a duration time of 5 min for each washing step. Bound antibody was visualized after 10-20 min using 4-chloro-1-naphtol (4CN) as a substrate after incubation with a freshly prepared mixture of a) 40 ml methanol/4CN 0.3 % and b) 100 µl H₂O₂ in 200 ml TBS.

### EXAMPLE 8

### Virus neutralization

Neutralization of virus was carried out in a 96-well format using BHK or VeroB4 cells. Cells were grown in DMEM including 10% fetal calf serum to 90% - 100% confluence. Virus was added to the cells, which produced about 100 foci forming units (single infection events) per wells together with the test serum. Test serum was diluted in 2-log intervals and each dilution was incubated with a virus solution representing 100 ffu in a total volume of 100 µl. The Serum-virus mix was incubated for 1h at 37 °C prior to infection. The virus-serum mix (100 µl) was left on the cell layer for 1h at 37 °C. After infection, the serum-virus mix was replaced against a low-viscosity overlay medium that was 200 µl DMEM medium including 0.8% methylcellulose. The cell cultures were incubated for 3 days in a 37 °C incubator with 5% CO₂ atmosphere. After 3 days, to each well 100 µl of a 3.7% formalin solution was added for 30 min at room temperature (RT). After inactivation, the low-viscosity overly was removed from the wells and the cell layer was washed three times with 1 x PBS. To stain cells for viral antigen, the cells were permeabilized using 50 µl PBS including 0.5% Triton X-100 for 20 min at RT. After Triton X-100 treatment, the cell layer was washed three times with 1 x PBS and 200 µl blocking solution (PBS including 10% FCS) was added to each well for 30 min at RT. The blocking solution was carefully removed and the primary detection mouse antibody (Dengue clone 1-11(3), AbD Serotec) directed against all four DENVs was added for 1h at RT. After primary antibody binding, the cell layers were washed three times with PBS and the secondary antibody, directed against the mouse antibody was added (Bio-Rad anti-mouse HRP conjugate 1:2000 in blocking buffer) and incubated for 1h at RT. After antibody binding the wells were washed three times with PBS and 30 µl of the staining substrate (recomBlot Immunoblot, Mikrogen) was added for 20 min at RT. Infected cells were counted and the neutralization efficiency of a test serum was determined as the reduction of ffu compared to the 100ffu in the control wells.

### RESULTS

### Recombinant antigens

In order to create an ED3-based assay to detect antibody in human serum samples a set of ED3 antigens fused to the maltose-binding-protein (MBP) was generated. In addition to DENV-1-4 also the ED3 domains from three other viruses were cloned, WNV, JEV and TBEV, to be used as controls. Since the shortened ED3-388 construct and the C-terminal deleted ED3 domain did not react with patient sera, five ED3S constructs were generated where the ED3 domain was linked to fractional units of the stem region (ED3-410, -420, 429, 435 and 443, the numbering indicates the C-terminal amino acid position). All five ED3-stem constructs were tested for reactivity with patient sera and the reactivity was compared to the reactivity observed with the ED3-399 construct. From that screening experiment it turned out that the ED3-stem construct ED3-420 demonstrated a more specific response compared to the other four ED3-stem constructs ED3-410, -429, -435 and -443.

The ED3S constructs reacted more specifically compared to ED3 indicating that the fused stem amino acids play a significant role for the detection of type-specific antibodies to the native conformation of the *E. coli* expressed ED3 domain.

From the results it can be concluded that a hidden epitope must be present in the ED3 domain. It seems to be possible that this epitope is only formed in the context of the complete ED3 polypeptide. Cross reactivity between the four DENV ED3 polypeptides is the consequence. The inventors have reduced cross reactivity by denaturing the polypeptide but it is also possible to bring the serotype-specific epitope into a flavivirus context that shows no cross reactivity. Humans will develop cross reactivity to other flaviviruses like for example USUTU virus. Therefore it is conceivable that the serotype-specific DENV epitope can be brought into the ED3 polypeptide from USUTU. Such a chimera will be another example to generate serotype-specific diagnostic antigens to differentiate DENV-1-4.

Denatured forms of ED3 can also be isolated from E. coli in the form of the full soluble E protein. When DENV E, containing the ED1, ED2 and ED3 domains is expressed and purified from bacterial extracts, the polypeptide can be in a denatured, non-active conformation. Such a conformation is also one example to reduce cross reactivity and therefore can be used to detect serotype-specific antibodies against DENV-1-4, WNV or other flaviviruses. A denatured, non-active conformation can also be generated by chimeras of ED1 and/or ED2 domains from other flaviviruses for example USUTU virus and the ED3 domain from DENV-1-4. Such chimeras are candidates for serotype specific assays, especially when the non-DENV E protein domains (ED1 and/or ED2) are from immunosilent flaviviruses where humoral immuneresponses in humans are low or absent.

### Screening for serotype-specific responses in serum samples obtained from DENV-infected patients

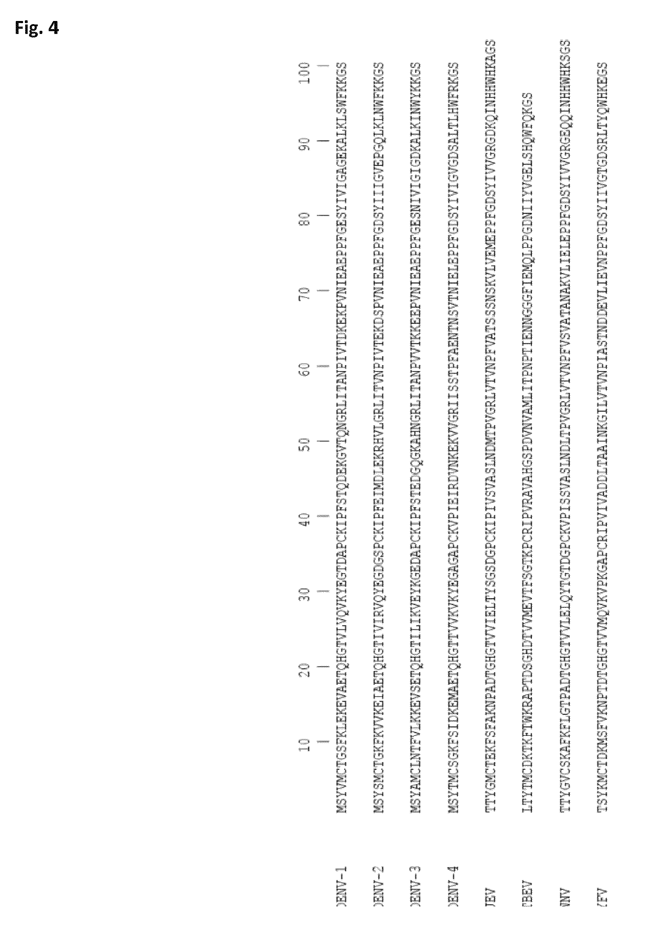

Next the reactivity of patient serum antibodies to a denatured form of the ED3 and ED3S constructs, designated ED3* and ED3S*, was investigated. All antigens for DENV, WNV, JEV and TBEV were dotted on nitrocellulose strips and tested for type-specific responses using a set of 225 DENV-positive sera. As a first result the inventors identified a predominant pattern of reactivity to the DENV-1 ED3* antigen. The serotype-specific responses are exemplarily shown in Fig.4. The highest signal-noise ratio was observed for the ED3* antigens in comparison to the non-denatured ED3S and ED3 MBP-fusions. Specific antibody binding to ED3* was seen in 50% of sera and most of the sera showed single reactions to one of the serotype-specific antigens, mainly ED3*-DENV-1 and ED3*-DENV-3. Using DENV-negative sera revealed that no reactivity was seen with the ED3*, ED3S* and ED3 S antigens.

### Correlation of serotype-specific responses and type-specific neutralization.

To further evaluate the ED3* test results 11 sera were chosen which showed serotype-specific responses in the ED3* dot assay. The serotype of a human serum is defined by its ability to neutralize one of the four DENVs better than the other three. Therefore, the sera were tested for neutralization of DENV-1, DENV-2, DENV-3 or DENV-4. As shows in Fig. 4 the highest neutralization titers were seen for the DENV strain that was tested positively in the ED3* dot assay. Serum A14 was clearly positive for ED3*-DENV-1 and no serotype-specific result could be obtained with ED3 or ED3S antigens. Altogether, most of the sera showed crossreactivity to ED3 and ED3S (A14, A52 S2, P15, P27, P30-2, P41-2, P48-1, P56-2, P60-2) but could be classified because of the ED3* test results. From the 11 sera, only 2 sera were positively tested for one DENV serotype (A3 S2, DENV-1); A31 S2, DENV-2) by all three antigen preparations but in the other 9 cases a serotype result was not obtained by the native ED3 conformations. Thus, the serological test using denatured ED3 (ED3*) identifies the serotype-specific antibody response to one of the DENVs in patient serum samples more efficiently. The test results were also in close correlation to the results of virus neutralization, Thus, the ED3* antigen test is a new diagnostic tool to determine the DENV serotype in human test samples.

### REFERENCES

Allwinn R, Doerr HW, Emmerich P, Schmitz H, Preiser Cross-reactivity in flavivirus serology: new implications of an old finding? Med Microbiol Immunol. 2002; 190:199-202.
Beltramello M, Williams KL, Simmons CP, Macagno A, Simonelli L, Quyen NT, Sukupolvi-Petty S, Navarro-Sanchez E, Young PR, de Silva AM, Rey FA, Varani L, Whitehead SS, Diamond MS, Harris E, Lanzavecchia A, Sallusto F. The human immune response to Dengue virus is dominated by highly cross-reactive antibodies endowed with neutralizing and enhancing activity. Cell Host Microbe. 2010 16:271-283.
Bernardo, L., Izquierdo, A., Alvarez, M., Rosario, D., Prado, I., López, C., ... Guzmán, M. G. (2008). Immunogenicity and protective efficacy of a recombinant fusion protein containing the domain III of the dengue 1 envelope protein in non-human primates. Antiviral research, 80(2), 194-9. doi:10.1016/j.antiviral.2008.06.005
Cen H, Windler SL, Rice LS, Zhang A, Zhou H., Multiplex epitope detection: A new method overcomes limitations of antibody arrays. Proteomics 2013, 41923:1696-1700.
Chastel, C. (2012). Eventual role of asymptomatic cases of dengue for the introduction and spread of dengue viruses in non-endemic regions. Frontiers in physiology, 3, 70. doi:10.3389/fphys.2012.00070
Christopher-Hennings J, Araujo KP, Souza CJ, Fang Y, Lawson S, Nelson EA, Clement T, Dunn M, Lunney JK., Opportunities for bead-based multiplex assays in veterinary diagnostic laboratories. Journal of Veterinary Diagnostic Investigation, 2013, 6:671-691.
Clarke, D. H., & Casals, J. (1958). Techniques for hemagglutination and hemagglutination-inhibition with arthropod-borne viruses. The American journal of tropical medicine and hygiene, 7(5), 561-73. Retrieved from http://www.ncbi.nlm.nih.gov/pubmed/13571577
Crill, W. D., Hughes, H. R., Delorey, M. J., & Chang, G.-J. J. (2009). Humoral immune responses of dengue fever patients using epitope-specific serotype-2 virus-like particle antigens. PloS one, 4(4), e4991. doi:10.1371/journal.pone.0004991
De Alwis, R., Smith, S. a, Olivarez, N. P., Messer, W. B., Huynh, J. P., Wahala, W. M. P. B., ... de Silva, A. M. (2012). Identification of human neutralizing antibodies that bind to complex epitopes on dengue virions. Proceedings of the National Academy of Sciences of the United States of America, 109(19), 7439-44. doi:10.1073/pnas.1200566109
Dejnirattisai, W., Jumnainsong, A., Onsirisakul, N., Fitton, P., Vasanawathana, S., Limpitikul, W., ... Screaton, G. (2010). Cross-reacting antibodies enhance dengue virus infection in humans. Science (New York, N.Y.), 328(5979), 745-8. doi:10.1126/science.1185181
Diamond, M. S., Pierson, T. C., & Fremont, D. H. (2008). The structural immunology of antibody protection against West Nile virus. Immunological reviews, 225, 212-25. doi:10.1111/j.1600-065X.2008.00676.x
Dobler G, Jelinek T, Frösner G, Nothdurft HD, Löscher T. Cross reactions of patients with acute dengue fever to tick-borne encephalitis. Wien Med Wochenschr. 1997; 147:463-464.
Duong, V., Ly, S., Lorn Try, P., Tuiskunen, A., Ong, S., Chroeung, N., ... Buchy, P. (2011). Clinical and virological factors influencing the performance of a NS1 antigen-capture assay and potential use as a marker of dengue disease severity. PLoS neglected tropical diseases, 5(7), e1244. doi:10.1371/journal.pntd.0001244
Endy, T. P., Nisalak, A., Chunsuttitwat, S., Vaughn, D. W., Green, S., Ennis, F. A., ... Libraty, D. H. (2004). Relationship of preexisting dengue virus (DV) neutralizing antibody levels to viremia and severity of disease in a prospective cohort study of DV infection in Thailand. The Journal of infectious diseases, 189(6), 990-1000. doi:10.1086/382280
Fibriansah G, Ng TS, Kostyuchenko VA, Lee J, Lee S, Wang J, Lok SM. Structural changes in dengue virus when exposed to a temperature of 37°C. J Virol. 2013 87:7585-7592.
Fry, S. R., Meyer, M., Semple, M. G., Simmons, C. P., Sekaran, S. D., Huang, J. X., ... Cooper, M. A. (2011). The diagnostic sensitivity of dengue rapid test assays is significantly enhanced by using a combined antigen and antibody testing approach. PLoS neglected tropical diseases, 5(6), e1199. doi:10.1371/journal.pntd.0001199
Green und Sambrook, Molecular Cloning: A Laboratory Manual, 4. Ausgabe, 2012, Cold Spring Harbor Laboratory. ISBN 978-1936113422.
Guzman, M. G., Halstead, S. B., Artsob, H., Buchy, P., Farrar, J., Gubler, D. J., ... Peeling, R. W. (2010). Dengue: a continuing global threat. Nature reviews. Microbiology, 8(12 Suppl), S7-16. doi:10.1038/nrmicro2460
Guzman, M. G., & Vazquez, S. (2010). The complexity of antibody-dependent enhancement of dengue virus infection. Viruses, 2(12), 2649-62. doi:10.3390/v2122649
Halstead, S. B., & O'Rourke, E. J. (1977). Dengue viruses and mononuclear phagocytes. I. Infection enhancement by non-neutralizing antibody. The Journal of experimental medicine, 146(1), 201-17. Retrieved from http://www.pubmedcentral.nih.gov/articlerender.fcgi?artid=2180729&tool=pmcentrez& rendertype=abstract
Halstead, S. B., Rojanasuphot, S., & Sangkawibha, N. (1983). Original antigenic sin in dengue. The American journal of tropical medicine and hygiene, 32(1), 154-6. Retrieved from http://www.ncbi.nlm.nih.gov/pubmed/6824120
Hsieh, S.-C., Liu, I.-J., King, C.-C., Chang, G.-J., & Wang, W.-K. (2008). A strong endoplasmic reticulum retention signal in the stem-anchor region of envelope glycoprotein of dengue virus type 2 affects the production of virus-like particles. Virology, 374(2), 338-50. doi:10.1016/j.virol.2007.12.041
Hue, K. D. T., Tuan, T. V., Thi, H. T. N., Bich, C. T. N., Anh, H. H. Le, Wills, B. A., & Simmons, C. P. (2011). Validation of an internally controlled one-step real-time multiplex RT-PCR assay for the detection and quantitation of dengue virus RNA in plasma. Journal of virological methods, 177(2), 168-73. doi:10.1016/j.jviromet.2011.08.002
Izquierdo, A., Valdés, I., Gil, L., Hermida, L., Gutiérrez, S., Garcia, A., ... Guzmán, M. G. (2012). Serotype specificity of recombinant fusion protein containing domain III and capsid protein of dengue virus 2. Antiviral research, 95(1), 1-8. doi:10.1016/j.antiviral.2012.04.006
Klein, D. E., Choi, J. L., & Harrison, S. C. (2013). Structure of a dengue virus envelope protein late-stage fusion intermediate. Journal of virology, 87(4), 2287-93. doi:10.1128/JVI.02957-12
Lai, C.-Y., Tsai, W.-Y., Lin, S.-R., Kao, C.-L., Hu, H.-P., King, C.-C., ... Wang, W.-K. (2008). Antibodies to envelope glycoprotein of dengue virus during the natural course of infection are predominantly cross-reactive and recognize epitopes containing highly conserved residues at the fusion loop of domain II. Journal of virology, 82(13), 6631-43. doi:10.1128/JVI.00316-08
Lanciotti, R. S., Calisher, C. H., Gubler, D. J., Chang, G. J., & Vorndam, A. V. (1992). Rapid detection and typing of dengue viruses from clinical samples by using reverse transcriptase-polymerase chain reaction. Journal of clinical microbiology, 30(3), 545-51. Retrieved from http://www.pubmedcentral.nih.gov/articlerender.fcgi?artid=265106&tool=pmcentrez&r endertype=abstract
Lazaro-Olán, L., Mellado-Sánchez, G., Garcia-Cordero, J., Escobar-Gutiérrez, A., Santos-Argumedo, L., Gutiérrez-Castañeda, B., & Cedillo-Barrón, L. (2008). Analysis of antibody response in human dengue patients from the Mexican coast using recombinant antigens. Vector borne and zoonotic diseases (Larchmont, N.Y.), 8(1), 69-79. doi:10.1089/vbz.2007.0117
Lin, S.-R., Zou, G., Hsieh, S.-C., Qing, M., Tsai, W.-Y., Shi, P.-Y., & Wang, W.-K. (2011). The helical domains of the stem region of dengue virus envelope protein are involved in both virus assembly and entry. Journal of virology, 85(10), 5159-71. doi:10.1128/JVI.02099-10
Lottspeich, F. and Engels J. W., Bioanalytik, 2012, 3. Edition, Spektrum Akademischer Verlag, ISBN 978-3-8274-2942-1
Ludolfs, D., Schilling, S., Altenschmidt, J., & Schmitz, H. (2002). Serological Differentiation of Infections with Dengue Virus Serotypes 1 to 4 by Using Recombinant Antigens, 40(11), 4317-4320. doi: 10.1128/JCM.40.11.4317
Oliphant, T., Nybakken, G. E., Austin, S. K., Xu, Q., Bramson, J., Loeb, M., ... Diamond, M. S. (2007). Induction of epitope-specific neutralizing antibodies against West Nile virus. Journal of virology, 81(21), 11828-39. doi:10.1128/JVI.00643-07
Peeling, R. W., Artsob, H., Pelegrino, J. L., Buchy, P., Cardosa, M. J., Devi, S., ... Yoksan, S. (2010). Evaluation of diagnostic tests: dengue. Nature reviews. Microbiology, 8(12 Suppl), S30-8. Retrieved from http://www.ncbi.nlm.nih.gov/pubmed/21548185
Reddy, P. B. J., Pattnaik, P., Tripathi, N. K., Srivastava, A., & Rao, P. V. L. (2012). Expression, purification and evaluation of diagnostic potential and immunogenicity of dengue virus type 3 domain III protein. Protein and peptide letter, 19(5), 509-19. Retrieved from http://www.ncbi.nlm.nih.gov/pubmed/22486646
Reynes, J.-M., Ong, S., Mey, C., Ngan, C., Hoyer, S., & Sall, A. A. (2003). Improved molecular detection of dengue virus serotype 1 variants. Journal of clinical microbiology, 41(8), 3864-7. Retrieved from http://www.pubmedcentral.nih.gov/articlerender.fcgi?artid=179838&tool=pmcentrez&r endertype=abstract
Rico-Hesse, R. (2003). Microevolution and virulence of dengue viruses. Advances in virus research, 59, 315-41. Retrieved from http://www.pubmedcentral.nih.gov/articlerender.fcgi?artid=3045824&tool=pmcentrez& rendertype=abstract
Sabchareon, A., Wallace, D., Sirivichayakul, C., Limkittikul, K., Chanthavanich, P., Suvannadabba, S., ... Lang, J. (2012). Protective efficacy of the recombinant, live-attenuated, CYD tetravalent dengue vaccine in Thai schoolchildren: a randomised, controlled phase 2b trial. Lancet, 380(9853), 1559-67. doi:10.1016/S0140-6736(12)61428-7
Schmidt, A. G., Yang, P. L., & Harrison, S. C. (2010). Peptide inhibitors of flavivirus entry derived from the E protein stem. Journal of virology, 84(24), 12549-54. doi:10.1128/JVI.01440-10
Simmons, M., Nelson, W. M., Wu, S. J., & Hayes, C. G. (1998). Evaluation of the protective efficacy of a recombinant dengue envelope B domain fusion protein against dengue 2 virus infection in mice. The American journal of tropical medicine and hygiene, 58(5), 655-62. Retrieved from http://www.ncbi.nlm.nih.gov/pubmed/9598457
Smith, S. A., Zhou, Y., Olivarez, N. P., Broadwater, A. H., de Silva, A. M., & Crowe, J. E. (2012). Persistence of circulating memory B cell clones with potential for dengue virus disease enhancement for decades following infection. Journal of virology, 86(5), 2665-75. doi:10.1128/JVI.06335-11
Staropoli, I., Clement, J. M., Frenkiel, M. P., Hofnung, M., & Deubel, V. (1996). Dengue virus envelope glycoprotein can be secreted from insect cells as a fusion with the maltose-binding protein. Journal of virological methods, 56(2), 179-89. Retrieved from http://www.ncbi.nlm.nih.gov/pubmed/8882648
Stiasny K, Kiermayr S, Holzmann H, Heinz FX. Cryptic properties of a cluster of dominant flavivirus cross-reactive antigenic sites. J Virol. 2006; 80:9557-9568.].
Sugrue, R. J., Cui, T., Xu, Q., Fu, J., & Chan, Y. C. (1997). The production of recombinant dengue virus E protein using Escherichia coli and Pichia pastoris. Journal of virological methods, 69(1-2), 159-69. Retrieved from http://www.ncbi.nlm.nih.gov/pubmed/9504761
Thaia et al., High-Resolution Analysis of Intrahost Genetic Diversity in Dengue Virus Serotype 1 Infection Identifies Mixed Infections. 2012, J. Virol. 86: 2 835-843.
Vong, S., Khieu, V., Glass, O., Ly, S., Duong, V., Huy, R., ... Buchy, P. (2010). Dengue incidence in urban and rural Cambodia: results from population-based active fever surveillance, 2006-2008. PLoS neglected tropical diseases, 4(11), e903. doi:10.1371/journal.pntd.0000903
Wahala, W M P B, Kraus, A. A., Haymore, L. B., Accavitti-Loper, M. A., & de Silva, A. M. (2009). Dengue virus neutralization by human immune sera: role of envelope protein domain III-reactive antibody. Virology, 392(1), 103-13. doi:10.1016/j.virol.2009.06.037
Wahala, Wahala M P B, Donaldson, E. F., de Alwis, R., Accavitti-Loper, M. A., Baric, R. S., & de Silva, A. M. (2010). Natural strain variation and antibody neutralization of dengue serotype 3 viruses. PLoS pathogens, 6(3), e1000821. doi:10.1371/journal.ppat.1000821
Wahala, Wahala M P B, Huang, C., Butrapet, S., White, L. J., & de Silva, A. M. (2012). Recombinant dengue type 2 viruses with altered e protein domain III epitopes are efficiently neutralized by human immune sera. Journal of virology, 86(7), 4019-23. doi:10.1128/JVI.06871-11
Wellems, T. E., & Howard, R. J. (1986). Homologous genes encode two distinct histidine-rich proteins in a cloned isolate of Plasmodium falciparum. Proceedings of the National Academy of Sciences of the United States of America, 83(16), 6065-9. Retrieved from http://www.pubmedcentral.nih.gov/articlerender.fcgi?artid=386439&tool=pmcentrez&r endertype=abstract
Westermeier, R.; Elektrophorese-Praktikum. VonVCH Verlagsgesellschaft, Weinheim 1990. ISBN 3-527-28172-X
WHO. (1996). Dengue and dengue haemorrhagic fever. Weekly epidemiological reword, 71(25), 195-196.
WHO/TRD. (1997). Dengue Hemorrhagic Fever: diagnosis, treatment prevention and control. 2nd edition.
WHO/TRD. (2009). Dengue: Guidelines for Diagnosis, Treatment, Prevention and C... Retrieved June 24, 2013, from http://www.ncbi.nlm.nih.gov/pubmed/23762963
Williams, K. L., Wahala, W. M. P. B., Orozco, S., de Silva, A. M., & Harris, E. (2012). Antibodies targeting dengue virus envelope domain III are not required for serotype-specific protection or prevention of enhancement in vivo. Virology, 429(1), 12-20. doi:10.1016/j.virol.2012.03.003
Zhang, Q., Hunke, C., Yau, Y.-H., Seow, V., Lee, S., Tanner, L. B., ... Lok, S.-M. (2012). The stem region of premembrane protein plays an important role in the virus surface protein rearrangement during dengue maturation. The Journal of biological chemistry, 287(48), 40525-40534. doi:10.1074/jbc.M112.384446
Zhang X, Sheng J, Plevka P, Kuhn RJ, Diamond MS, Rossmann MG. Dengue structure differs at the temperatures of its human and mosquito hosts. Proc Natl Acad Sci USA. 2013; 110:6795-6799.
Zidane, N., Dussart, P., Bremand, L., & Bedouelle, H. (2013). Cross-reactivities between human IgMs and the four serotypes of dengue virus as probed with artificial homodimers of domain-III from the envelope proteins. BMC infectious diseases, 13(1), 302. doi:10.1186/1471-2334-13-302

## Claims

1. Polypeptide comprising an amino acid sequence that is at least 65% identical to a sequence selected from the group of SEQ ID NO: 1, 5, 9 and 13 (*E-protein partial sequence; N-terminally truncated*), from the group of SEQ ID NO: 2, 6, 10 and 14 (*ED3S; ED3 domain and stem region*), from the group of SEQ ID NO: 3, 4, 7, 8, 11, 12 and 15 to 23 (*ED3; ED3 domain specific clone and consensus sequence),* or from the group of SEQ ID NO: 24 to 31 (*E-protein full sequence*), wherein said polypeptide is denatured.

2. Polypeptide according to claim 1, wherein the amino acid sequence is at least 75%, or at least 85%, or at least 90%, or at least 95%, or at least 98% identical to the sequence selected from the group of SEQ ID NO: 1, 5, 9 and 13, from the group of SEQ ID NO: 2, 6, 10 and 14 or from the group of SEQ ID NO: 3, 4, 7, 8, 11, 12 and 15 to 23.

3. Polypeptide according to claim 1 or 2 for use in a method of diagnosis of a flavivirus infection in a subject.

4. Polypeptide according to claim 1 or 2 or polypeptide for use according to claim 3, wherein the flavivirus is West Nile virus, Japanese encephalitis virus, dengue virus, tick-borne encephalitis virus, or yellow fever virus.

5. Polypeptide or polypeptide for use according to claim 4, wherein the dengue virus is dengue virus serotype 1, 2, 3 or 4.

6. Method for determining a flavivirus infection in a subject, comprising contacting a sample from the subject with a polypeptide according to claim 1 or 2, and detecting binding of an antibody from the sample to the polypeptide.

7. Method according to claim 6 or 7, wherein the sample is contacted with at least two polypeptides according to claim 1 or 2, wherein the polypeptides are sequence variants of the amino acid sequence selected from the group of SEQ ID NO: 1, 5, 9 and 13, from the group of SEQ ID NO: 2, 6, 10 and 14, from the group of SEQ ID NO: 3, 4, 7, 8, 11, 12 and 15 to 23 or from the group of SEQ ID NO: 24 to 31, wherein the variants have at least 85% sequence identity.

8. Method according to claim 6 or 7, wherein the binding of an antibody to the polypeptide is detected by an immunofluorescence-test, a protein microarray, an ELISA, a luminescence-test, a blot, a radioimmune test, a Western blot or a dot blot.

9. Method according to any one of claims 6 to 8, wherein the flavivirus is West Nile virus, dengue virus, tick-borne encephalitis virus, or yellow fever virus.

10. Method according to claim 9, wherein the dengue virus is dengue virus serotype 1, 2, 3 or 4.

11. Composition comprising the polypeptide according to claim 1 or 2 linked or attached to a solid phase.

12. Composition according to claim 11 comprising at least two polypeptides according to claim 1 to 2, wherein the polypeptides are sequence variants of the amino acid sequence selected from the group of SEQ ID NO: 1, 5, 9 and 13, from the group of SEQ ID NO: 2, 6, 10 and 14, from the group of SEQ ID NO: 3, 4, 7, 8, 11, 12 and 15 to 23 or from the group of SEQ ID NO: 24 to 31, wherein the variants have at least 85% sequence identity.

13. Composition according to claim 11 or 12, wherein the solid phase is a substrate, preferably a microtiter plate, a strip, or a bead.

14. Kit for use in a method according to any one of claims 6 to 10 comprising the polypeptide according to claim 1 or 2 or the composition according to any one of claims 12 to 13, optionally with further reagents and means for performing said method.

15. Kit according to claim 14, comprising at least two polypeptides according to claim 1 or 2 or at least two compositions according to any one of claims 11 to 13, wherein the polypeptides are sequence variants of the amino acid sequence selected from the group of SEQ ID NO: 1, 5, 9 and 13, from the group of SEQ ID NO: 2, 6, 10 and 14, from the group of SEQ ID NO: 3, 4, 7, 8, 11, 12 and 15 to 23 or from the group of SEQ ID NO: 24 to 31, wherein the variants have at least 85% sequence identity.

16. Kit according to claim 13 or claim 14, comprising an assay plate comprising a multiplicity of microtiter wells comprising the polypeptide according to claim 1 or 2 or the composition according to any one of claims 11 to 13, or comprising at least two polypeptides according to claim 1 or 2 or at least two compositions according to any one of claims 11 to 13; and a container means comprising a labeled secondary antibody having specific binding affinity for a flavivirus antibody in the sample.
